# EUROPEAN PATENT APPLICATION

(11) **EP 4 461 814 A2**
(43) Date of publication of application: **13.11.2024**
(21) Application number: 24200416.6
(22) Date of filing: 11.10.2019
(51) Int. Cl.: C12N 15/864

(54) **GENERATION OF IMPROVED HUMAN PAH FOR TREATMENT OF SEVERE PKU BY LIVER-DIRECTED GENE REPLACEMENT THERAPY**

(30) Priority: 12.10.2018 US 201862744944 P
(62) Divisional of application: 19797895.0
(71) Applicant: Genzyme Corporation, Cambridge, MA 02142 (US)
(72) Inventor: KYOSTIO-MOORE, Sirkka, Bridgewater, 08807 (US); MANAVALAN, Partha, Bridgewater, 08807 (US)
(74) Representative: Mathys & Squire

(57) **Abstract**

Provided herein are variant phenylalanine hydroxylase (PAH) polypeptides which are more stable and have greater activity than wild-type human PAH. Also provided are methods to treat phenylketonuria (PKU) and/or to reduce levels of phenylalanine in an individual in need thereof. Further provided herein are expression cassettes, vectors (e.g., rAAV vectors), viral particles, pharmaceutical compositions and kits for expressing the variant PAH polypeptide in an individual in need thereof.

## Description

### CROSS-REFERENCE TO RELATED APPLICATIONS

This application claims the priority benefit of U.S. Provisional Application No. 62/744,944, filed October 12, 2018, which is hereby incorporated by reference in its entirety.

### SUBMISSION OF SEQUENCE LISTING ON ASCII TEXT FILE

The content of the following submission on ASCII text file is incorporated herein by reference in its entirety: a computer readable form (CRF) of the Sequence Listing (file name: 159792016640SEQLIST.TXT, date recorded: October 16, 2019, size: 33 KB).

### FIELD OF THE INVENTION

The present disclosure relates to variant phenylalanine hydroxylase polypeptides. In some aspects, the disclosure relates to compositions and methods for treating phenylketonuria using gene therapy.

### BACKGROUND

Phenylketonuria (PKU) is a genetic deficiency of liver enzyme phenylalanine hydroxylase (PAH) that catalyzes hydroxylation of phenylalanine (Phe) to tyrosine (Tyr). This disease is the most common inborn error of amino acid metabolism, with an overall incidence of 1:10-15,000 in North America, and it is detected by newborn screening programs in most developed countries. In the absence of any treatment, the severe form of PKU leads to highly elevated blood Phe levels that are neurotoxic and associated with severe mental retardation (Kochhar 2012, Ho 2014, Blau 2015). The affected protein, PAH, is a multi-domain protein consisting of N-terminal regulatory (1-117), central catalytic (118-410) and C-terminal tetramerization domains (411-452) (Flydal 2013). To date over 560 disease-causing mutations have been mapped to each domain with the catalytic region being the most frequently affected site (Erlandsen 2003). The homo-multimeric enzyme is subject to complex regulation with phosphorylation and allosteric activation by substrate Phe binding to the N-terminal domain which fine-tunes PAH enzyme activity by altering various conformational and multimerization states of the enzyme (Knappskog 1996, Jaffe 2013, Arturo 2016).

The current treatment for PKU is a life-long dietary restriction of Phe using a low protein diet and liquid medical formula (Kochhar 2012, Ho 2014, Blau 2015). Although efficacious, the poor taste of the medical food and the severe limitations on food choices make adherence to the diet difficult and non-compliance increases steadily during childhood, and by late teens nearly 80% of patients have higher than recommended blood Phe levels (Waisbren 2007, Thomas 2017). There is also emerging evidence that despite good adherence to Phe-restricted diet, many patients experience deficiencies in various neurocognitive and neuropsychiatric functions as well as have a high incidence of attention deficit-hyperactivity disorder (ADHD). While the reasons for this are unclear, potential explanations include amino acid imbalances in brain, nutritional deficiencies in certain vitamins and trace elements as well as fluctuations in blood Phe levels normally maintained stable by liver PAH activity (Cleary 2013, Gonzales 2016, Vogel 2017). Interestingly, treatment of patients with milder forms of PKU with a synthetic form of a cofactor tetrahydrobiopterin (BH4) (Sapropterin dihydrochloride) has been shown efficacious not only in lowering of blood Phe levels, but also has demonstrated improvement in neurological outcomes such as reduction in ADHD symptoms (Burton 2015). This therapy increases residual PAH enzyme activity by acting as a pharmacological chaperone and hence can provide partial correction of the genetic defect by providing normal Phe regulated PAH activity (Blau 2015). Another therapy recently approved consists of an enzyme substitution therapy using a PEGylated form of bacterial phenylalanine ammonia lyase (PAL) that metabolizes Phe into trans-cinnamic acid. This therapy provides significant reduction in blood Phe levels but appears to be less efficacious on neurological endpoints (Longo 2014). It remains unclear whether this or any other therapies based on mainly lowering blood Phe levels in the absence of correcting the PAH function as a regulator of systemic Phe levels and a producer of Tyr can address the cognitive and neuropsychiatric issues observed even in diet compliant PKU patients.

All references cited herein, including patent applications and publications, are incorporated by reference in their entirety.

### BRIEF SUMMARY OF THE INVENTION

The present invention is based at least in part on the Inventors discovery of improved variants of hPAH and in particular variant-1 (V1) that contains four amino acid changes resulting in improved protein stability and enzyme activity compared to the endogenous hPAH. Delivery of cDNA encoding this hPAH-V1 variant to the liver using clinically relevant doses of rAAV improved various disease endpoints in the PAH^{enu2} mice, a model of human PKU, and was more efficacious than the unmodified hPAH. Thus, rAAV vectors encoding this novel variant could provide a path for PKU gene therapy by allowing efficacy with reduced vector doses.

In some aspects, the invention provides a variant phenylalanine hydroxylase (PAH) polypeptide comprising two amino acid substitutions, wherein the amino acid substitutions are at sites selected from M180, K199, S250, and G256 of a wild-type human PAH polypeptide. In some aspects, the invention provides a variant phenylalanine hydroxylase (PAH) polypeptide comprising three amino acid substitutions, wherein the amino acid substitutions are at sites selected from M180, K199, S250, and G256 of a wild-type human PAH polypeptide. In some aspects, the invention provides a variant phenylalanine hydroxylase (PAH) polypeptide comprising four amino acid substitutions at sites M180, K199, S250, and G256 of a wild-type human PAH polypeptide. In some embodiments, the amino acid substitution comprises one of more of M180T, K199P, S250P, and G256A. In some embodiments, the amino acid substitution comprises K199P, S250P, and G256A; M180T, S250P, and G256A; M180T, K199P, and G256A; or M180T, K199P, and S250P. In some embodiments, the amino acid substitution comprises M180T, K199P, S250P, and G256A. In some embodiments, the variant PAH polypeptide further comprises H264P, G272A, G272P, P275L, P279Q, G272P and P275L, or T323R and F327T amino acid substitutions. In some embodiments, the wild-type human PAH polypeptide comprises the amino acid sequence of SEQ ID NO: 1. In some embodiments, the variant PAH polypeptide is a human PAH polypeptide. In some embodiments, the variant PAH polypeptide comprises an amino acid sequences that is at least about 80% identical to the amino acid sequence of SEQ ID NO:3. In some embodiments, the variant PAH polypeptide comprises the amino acid sequence of SEQ ID NO:3. In some embodiments, the variant PAH polypeptide further comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide. In some embodiments, the variant PAH polypeptide further comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide. In some embodiments, the variant PAH polypeptide further comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide. In some embodiments, the variant PAH polypeptide comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide.

In some aspects, the invention provides a variant PAH polypeptide, wherein the variant PAH polypeptide comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide. In some aspects, the invention provides a variant PAH polypeptide comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide.

In some embodiments, the variant PAH polypeptide comprises an N-terminal truncation. In some embodiments, the N-terminal truncation comprises a truncation of the N-terminal regulatory domain. In some embodiments, the N-terminal truncation comprises a truncation of amino acid residues 1-102 of the wild-type PAH polypeptide. In some embodiments, the variant PAH polypeptide comprises a C-terminal truncation. In some embodiments, the C-terminal truncation comprises a truncation of the tetramerization domain. In some embodiments, the C-terminal truncation comprises a truncation of amino acid residues 429-452 of the wild-type PAH polypeptide. In some embodiments, the variant PAH polypeptide comprises an amino acid sequence corresponding to amino acid residues 103-428 of the wild-type PAH polypeptide.

In some embodiments, the variant PAH polypeptide comprises one or more amino acid substitutions to eliminate potential protease cleavage sites. In some embodiments, the one or more amino acid substitutions to eliminate potential protease cleavage sites are located at positions 270-295 and/or 380-405 of the wild type PAH polypeptide.

In some embodiments, the variant PAH polypeptide is fused to a liver targeting polypeptide. In some embodiments, the liver targeting polypeptide is a HGF or fragments thereof or glycoproteins that bind to hepatocyte asialoglycoprotein receptor. In some embodiments, the variant PAH polypeptide is PEGylated and/or nitrosylated. In some embodiments, the variant PAH polypeptide is comprises a I374C amino acid substitution, wherein the cys residue at position 374 is nitrosylated.

In some embodiments the invention provides a composition comprising the variant PAH polypeptide as described herein. In some embodiments, the composition further comprises a pharmaceutically acceptable carrier.

In some aspects, the invention provides an isolated nucleic acid encoding the variant PAH polypeptide as described herein. In some embodiments, the nucleic acid encoding the variant PAH polypeptide is operably linked to a promoter. In some embodiments, the promoter is selected from a cytomegalovirus (CMV) immediate early promoter, an RSV LTR, a MoMLV LTR, a phosphoglycerate kinase- 1 (PGK) promoter, a simian virus 40 (SV40) promoter, a CK6 promoter, a transthyretin promoter (TTR), a mTTR482 promoter, a mA1MB2-mTTR482 promoter, a TK promoter, a tetracycline responsive promoter (TRE), an HBV promoter, an hAAT promoter, a LSP promoter, an LP1 promoter, a chimeric liver-specific promoter (LSP), an E2F promoter, a telomerase (hTERT) promoter; a cytomegalovirus enhancer/chicken beta-actin/Rabbit β-globin promoter (CAG) promoter, an elongation factor 1-alpha promoter (EFl-alpha) promoter, a human β-glucuronidase promoter, a chicken β-actin (CBA) promoter, a modified chicken β-actin (CBA) promoter or SEQ ID NO: 17, a retroviral Rous sarcoma virus (RSV) LTR promoter, a dihydrofolate reductase promoter, and a 13-actin promoter. In some embodiments, the promoter is an LP1 promoter or an mA1MB2-mTTR482 promoter. In some embodiments, the nucleic acid further comprises a polyadenylation signal. In some embodiments, the polyadenylation signal is a bovine growth hormone polyadenylation signal, an SV40 polyadenylation signal, or a HSV TK polyadenylation signal. In some embodiments, the nucleic acid further comprises an intron. In some embodiments, the intron is a chicken β-actin (CBA)/rabbit β-globin hybrid intron. In some embodiments, the intron is a modified chicken β-actin (CBA)/rabbit β-globin hybrid intron of SEQ ID NO: 15. In some embodiments, the nucleic acid further comprises one or more ITRs. In some embodiments, the nucleic acid further comprises a stuffer nucleic acid. In some embodiments, the stuffer nucleic acid is optimized to remove ATG sequences. In some embodiments, the stuffer nucleic acid is an A1AT intron stuffer sequence of SEQ ID NO: 16.

In some aspects, the invention provides an isolated nucleic acid encoding a human PAH polypeptide, wherein the nucleic acid is codon-optimized. In some embodiments, the nucleic acid sequence is at least 80% identical to the nucleic acid sequence of SEQ ID NO: 14. In some embodiments, the nucleic acid comprises the nucleic acid sequence of SEQ ID NO: 14. In some embodiments, the nucleic acid is an mRNA. In some aspects, the invention provides a composition comprising the nucleic acid as described herein. In some embodiments, the composition further comprises a pharmaceutically acceptable carrier.

In some aspects, the invention provides a vector comprising the isolated nucleic acid as described herein. In some embodiments, the vector is a recombinant adeno-associated virus (rAAV) vector. In some embodiments, the rAAV vector comprises the nucleic acid as described herein which is flanked by one or more AAV inverted terminal repeat (ITR) sequences. In some embodiments, the nucleic acid is flanked by two AAV ITRs. In some embodiments, the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAVDJ, a goat AAV, bovine AAV, or mouse AAV serotype ITRs. In some embodiments, the AAV ITRs are AAV2 ITRs. In some embodiments, the rAAV vector comprises 5' to 3' an AAV2 ITR, a promoter, an intron, nucleic acid encoding a PAH polypeptide, a stuffer nucleic acid, a polyadenylation signal, and an AAV2 ITR. In some embodiments, the promoter is a m1A1MB2-mTTR482 promoter or an LP1 promoter. In some embodiments, the intron is a chicken β-actin (CBA)/rabbit β-globin hybrid intron. In some embodiments, the PAH polypeptide is the variant PAH polypeptide as described herein. In some embodiments, the nucleic acid encoding the PAH polypeptide is the codon optimized nucleic acid. In some embodiments, the stuffer nucleic acid comprises nucleic acid from an intron of the human alpha 1 antitrypsin gene. In some embodiments, the intron of the human alpha 1 antitrypsin gene has been mutated to remove ATG sequences. In some embodiments, the polyadenylation signal is a bovine growth hormone polyadenylation signal. In some embodiments, the vector is a self-complimenting vector. In some embodiments, the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length. In some embodiments, the first nucleic acid sequence and the second nucleic acid sequence are linked by a mutated AAV ITR, wherein the mutated AAV ITR comprises a deletion of the D region and comprises a mutation of the terminal resolution sequence.

In some aspects, the invention provides a rAAV particle comprising the rAAV vector as described herein. In some embodiments, the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid. In some embodiments, the AAV viral particle comprises an engineered AAV capsid. In some embodiments, the engineered AAV capsid is a DJ capsid or an LK03 capsid. In some embodiments, the ITR and the capsid of the rAAV viral particle are derived from the same AAV serotype. In some embodiments, the ITR and the capsid of the rAAV viral particles are derived from different AAV serotypes. In some embodiments, the rAAV viral particle comprises AAV8 capsid. In some embodiments, the rAAV viral particle comprises an AAV8 capsid, and wherein the vector comprises AAV2 ITRs.

In some embodiments, the AAV viral particle comprises a capsid that has at least 85% sequence identity with an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV1 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV2 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV3 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV4 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV5 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV6 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV7 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV8 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV9 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV11 serotype capsid, such as at least 87%, 90%, 91%, 92%, 93%, 94%, 95%, 96%, 97%, 98%, or 99% sequence identity. In some embodiments, the AAV viral particle comprises a capsid that has at least 85% sequence identity with an AAV12 serotype capsid, such as at least 87%, 90%, 91%, 92%, 93%, 94%, 95%, 96%, 97%, 98%, or 99% sequence identity.

In some embodiments, the AAV viral particle comprises a hybrid capsid. In some embodiments, the hybrid capsid is an AAV1/AAV6, AAV2/AAV6, AAV3/AAV6, AAV4/AAV6, AAV5/AAV6, AAV7/AAV6, AAV8/AAV6, AAV9/AAV6, AAV10/AAV6, AAV11/AAV6, AAV12/AAV6, AAV1/AAV8, AAV2/AAV8, AAV3/AAV8, AAV4/AAV8, AAV5/AAV8, AAV7/AAV8, AAV9/AAV8, AAV10/AAV8, AAV11/AAV8, AAV12/AAV8, or AAV1/AAV6/AAV8 hybrid capsid. In some embodiments, the hybrid capsid is an AAV8/AAV6 hybrid capsid. In some embodiments, the hybrid capsid is an AAV1/AAV6/AAV8 hybrid capsid.

In some embodiments, the AAV viral particle comprises a hybrid capsid. In some embodiments, the hybrid capsid is an AAV1/AAV6, AAV2/AAV6, AAV3/AAV6, AAV4/AAV6, AAV5/AAV6, AAV7/AAV6, AAV8/AAV6, AAV9/AAV6, AAV10/AAV6, AAV11/AAV6, AAV12/AAV6, AAV1/AAV8, AAV2/AAV8, AAV3/AAV8, AAV4/AAV8, AAV5/AAV8, AAV7/AAV8, AAV9/AAV8, AAV10/AAV8, AAV11/AAV8, AAV12/AAV8, or AAV1/AAV6/AAV8 hybrid capsid. In some embodiments, the hybrid capsid is an AAV8/AAV6 hybrid capsid. In some embodiments, the hybrid capsid is an AAV1/AAV6/AAV8 hybrid capsid.

In some embodiments, the hybrid capsid comprises an amino acid sequence that has at least 95% sequence identity with a portion of AAV1 capsid sequence, such as at least 96%, 97%, 98%, or 99% sequence identity. In some embodiments, the hybrid capsid comprises an amino acid sequence that has at least 95% sequence identity with a portion of AAV2 capsid sequence, such as at least 96%, 97%, 98%, or 99% sequence identity. In some embodiments, the hybrid capsid comprises an amino acid sequence that has at least 95% sequence identity with a portion of AAV3 capsid sequence, such as at least 96%, 97%, 98%, or 99% sequence identity. In some embodiments, the hybrid capsid comprises an amino acid sequence that has at least 95% sequence identity with a portion of AAV4 capsid sequence, such as at least 96%, 97%, 98%, or 99% sequence identity. In some embodiments, the hybrid capsid comprises an amino acid sequence that has at least 95% sequence identity with a portion of AAV5 capsid sequence, such as at least 96%, 97%, 98%, or 99% sequence identity. In some embodiments, the hybrid capsid comprises an amino acid sequence that has at least 95% sequence identity with a portion of AAV6 capsid sequence, such as at least 96%, 97%, 98%, or 99% sequence identity. In some embodiments, the hybrid capsid comprises an amino acid sequence that has at least 95% sequence identity with a portion of AAV7 capsid sequence, such as at least 96%, 97%, 98%, or 99% sequence identity. In some embodiments, the hybrid capsid comprises an amino acid sequence that has at least 95% sequence identity with a portion of AAV8 capsid sequence, such as at least 96%, 97%, 98%, or 99% sequence identity. In some embodiments, the hybrid capsid comprises an amino acid sequence that has at least 95% sequence identity with a portion of AAV9 capsid sequence, such as at least 96%, 97%, 98%, or 99% sequence identity. In some embodiments, the hybrid capsid comprises an amino acid sequence that has at least 95% sequence identity with a portion of AAV10 capsid sequence, such as at least 96%, 97%, 98%, or 99% sequence identity. In some embodiments, the hybrid capsid comprises an amino acid sequence that has at least 95% sequence identity with a portion of AAV12 capsid sequence, such as at least 96%, 97%, 98%, or 99% sequence identity. In some embodiments, the hybrid capsid comprises an amino acid sequence that has at least 95% sequence identity with a portion of AAV8 capsid sequence, such as at least 96%, 97%, 98%, or 99% sequence identity. In some embodiments, the portion comprises at least 100 amino acids, such as at least 150, 200, 250, 300, 350, or 400 amino acids.

In some aspects, the invention provides a composition comprising the rAAV particle as described herein. In some embodiments, the composition further comprises a pharmaceutically acceptable carrier.

In some aspects, the invention provides a cell comprising the isolated nucleic acid as described herein. In some aspects, the invention provides a method of producing a variant PAH polypeptide, the method comprising culturing the cell described above under conditions to produce the variant PAH polypeptide. In some embodiments, the method further comprising the step of purifying the variant PAH polypeptide.

In some aspects, the invention provides a method for treating phenylketonuria in an individual in need thereof, comprising administering to the individual the variant PAH polypeptide as described herein or the composition as described herein. In some aspects, the invention provides a method for treating phenylketonuria in an individual in need thereof, comprising administering to the individual nucleic acid encoding the variant PAH polypeptide as described herein or the nucleic acid as described herein. In some aspects, the invention provides a method for treating phenylketonuria in an individual in need thereof, comprising administering to the individual the rAAV vector as described herein. In some aspects, the invention provides a method for treating phenylketonuria in an individual in need thereof, comprising administering to the individual the rAAV particle as described herein. In some embodiments, the invention provides a method for treating phenylketonuria in an individual in need thereof, comprising administering to the individual the composition as described herein. In some embodiments, the invention provides a method for treating phenylketonuria in an individual in need thereof, comprising administering to the individual the cell as described herein. In some embodiments, the individual lacks PAH activity.

In some aspects, the invention provides a method for reducing the level of phenylalanine in the blood of in an individual in need thereof, comprising administering to the individual the variant PAH polypeptide as described herein. In some aspects, the invention provide a method for reducing the level of phenylalanine in the blood of in an individual in need thereof, comprising administering to the individual nucleic acid encoding the variant PAH polypeptide as described herein or the nucleic acid as described herein. In some aspects, the invention provides a method for reducing the level of phenylalanine in the blood of in an individual in need thereof, comprising administering to the individual the rAAV vector as described herein. In some aspects, the invention provides a method for reducing the level of phenylalanine in the blood of in an individual in need thereof, comprising administering to the individual the rAAV particle as described herein. In some aspects, the invention provides a method for reducing the level of phenylalanine in the blood of an individual in need thereof, comprising administering to the individual the composition as described herein. In some embodiments, the level of phenylalanine in the blood of the individual prior to treatment is elevated compared to the level of phenylalanine in the blood of peer-matched control individuals. In some aspects the invention provides a method for reducing the level of phenylalanine in the blood of an individual in need thereof, comprising administering to the individual the cell as described herein.

In some embodiments, the nucleic acid, rAAV vector, rAAV particle, composition or cell is administered intravenously, intraarterially, intrahepatically, intraportally, intraperitoneal, or subcutaneously. In some embodiments, the administration is in combination with another therapy. In some embodiments, the another therapy is treatment with tetrahydribiopterin, treatment with phenylalanine ammonia lyase (PAL) or PEGylated PAL, or a phenylalanine-restricted diet.

In some aspects, the invention provides a method for making a PAH polypeptide comprising culturing the cell as described herein under conditions produce the PAH polypeptide. In some embodiments, the method further comprises purifying the PAH polypeptide.

In some aspects, the invention provides a kit comprising the variant PAH polypeptide as described herein. In some embodiments, the invention provides a kit comprising the nucleic acid as described herein, the rAAV vector as described herein, the rAAV particle as described herein, or the composition as described herein. In some embodiments, the kit further comprises instructions for use; buffers and/or pharmaceutically acceptable excipients; and/or bottles, vials and/or syringes.

In some aspects, the invention provides an expression cassette for expressing a transgene in a liver cell, wherein the expression cassette comprises a transgene operably linked to a promoter and enhancer, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer. In some embodiments, the mTTR promoter is a mTTR482 promoter. In some embodiments, the enhancer is 5' to the mTTR promoter.

In some aspects, the invention provides an expression cassette for expressing a transgene in a liver cell, wherein the expression cassette comprises a transgene operably linked to a promoter and a 3' element, wherein the promoter comprises a mouse transthyretin (mTTR) promoter and the 3' element is an albumin 3' element (3'Alb) or an albumin 3' element linked to a human alpha 1 antitrypsin scaffold/matrix attachment region (SMAR) (3'AlbSMAR). In some embodiments, the mTTR promoter is an mTTR482 promoter. In some embodiments, the 3' element is located 3' to the transgene.

In some aspects, the invention provides an expression cassette for expressing a transgene in a liver cell, wherein the expression cassette comprises a transgene operably linked to a promoter and enhancer and a 3' element, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer; and wherein the 3' element is an albumin 3' element (3'Alb) or an albumin 3' element linked to a human alpha 1 antitrypsin scaffold/matrix attachment region (SMAR) (3'AlbSMAR). In some embodiments, the mTTR promoter is an mTTR482 promoter. In some embodiments, the enhancer is 5' to the mTTR promoter. In some embodiments, the 3' element is located 3' to the transgene.

In some embodiments, the expression cassette further comprises an intron. In some embodiments, the intron is a chicken β-actin/rabbit β-globin hybrid intron. In some embodiments, the expression cassette further comprises a polyadenylation signal. In some embodiments, the polyadenylation signal is a bovine growth hormone polyadenylation signal.

In some embodiments, the transgene encodes a PAH polypeptide or a variant PAH polypeptide. In some embodiments, the invention provides a vector comprising the expression cassette as described herein. In some embodiments, the vector is a recombinant adeno-associated virus (rAAV) vector.

In some embodiments, the invention provides an rAAV vector comprising the expression cassette as described herein is flanked by one or more AAV inverted terminal repeat (ITR) sequences. In some embodiments, the expression cassette is flanked by two AAV ITRs. In some embodiments, the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs. In some embodiments, the AAV ITRs are AAV2 ITRs.

In some embodiments, the vector is a self-complimenting vector. In some embodiments, the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length. In some embodiments, the first nucleic acid sequence and the second nucleic acid sequence are linked by a mutated AAV ITR, wherein the mutated AAV ITR comprises a deletion of the D region and comprises a mutation of the terminal resolution sequence.

In some embodiments, the invention provides an rAAV particle comprising the rAAV vector as described herein. In some embodiments, the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid. In some embodiments, the AAV viral particle comprises an engineered AAV capsid. In some embodiments, the engineered AAV capsid is a DJ capsid or an LK03 capsid. In some embodiments, the ITR and the capsid of the rAAV viral particle are derived from the same AAV serotype. In some embodiments, the ITR and the capsid of the rAAV viral particles are derived from different AAV serotypes. In some embodiments, the invention provides a composition comprising the rAAV particle as described herein. In some embodiments, the composition further comprises a pharmaceutically acceptable carrier.

### BRIEF DESCRIPTION OF THE DRAWINGS

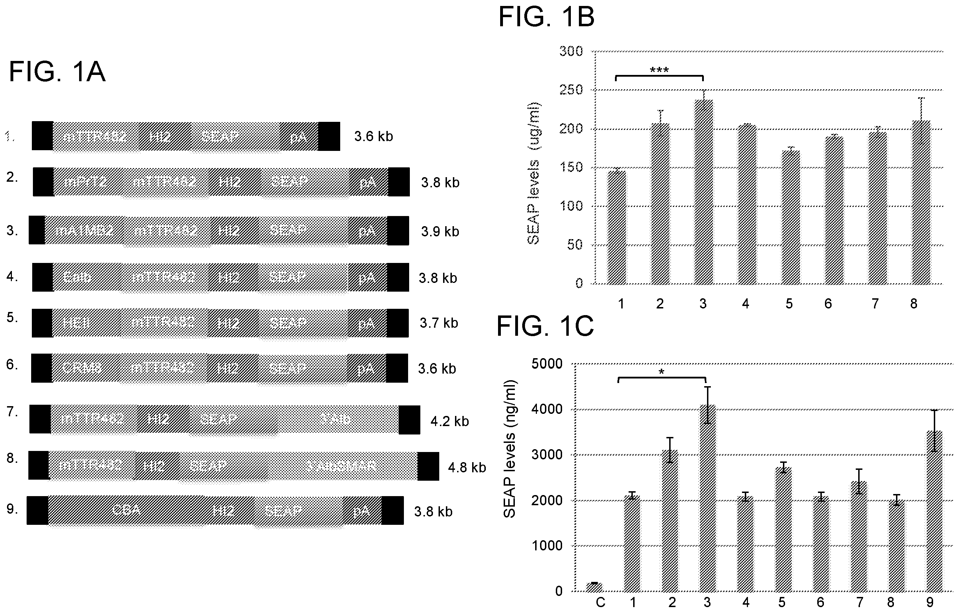

**FIGS. 1A-1C** show optimization of liver promoter. **FIG. 1A** shows a diagram of previral plasmid constructs. Construct mTTR482 (#1) was modified by addition of various liver enhancer elements (constructs #2-6) or 3' stability elements (constructs #7-8). CBA promoter containing construct was used for comparison. All constructs contained flanking ITRs, hybrid intron and a SEAP reporter. Constructs were then transfected into Huh7 cells **(****FIG. 1B****)** or HepG2 cells **(****FIG. 1C****).** Levels of secreted SEAP activity was measured 72 hrs post transfection and normalized by b-galactosidase levels from a co-transfected LacZ plasmid. Each evaluation contained n=3-4/plasmids.

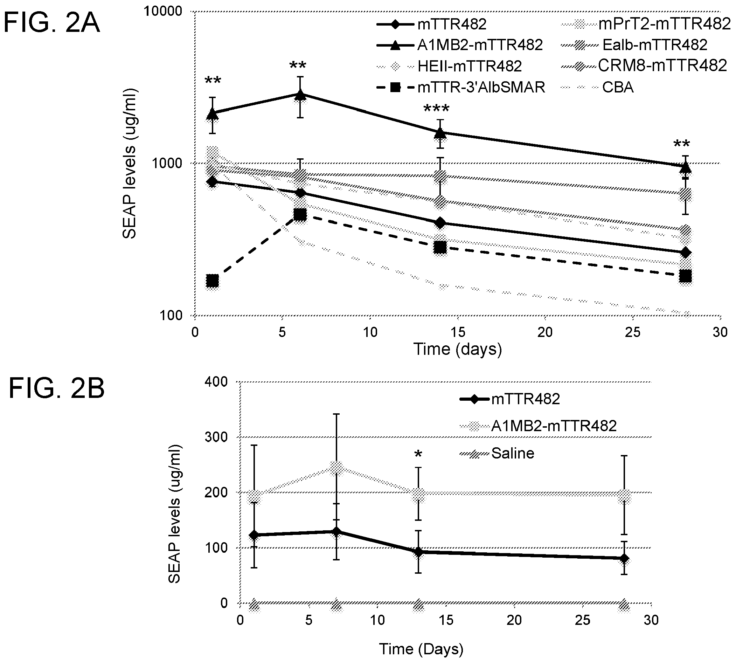

**FIGs. 2A and 2B** show the evaluation of liver promoters by SEAP production in vivo. **FIG. 2A** shows SEAP levels in normal C57BL/6 mice. **FIG. 2B** shows SEAP levels in PAH^{enu2} mice. In both experiments, plasmid vectors were delivered by high volume injection and plasma was measured for SEAP activity at various time-points. Each treatment group contained n=3-6 animals.

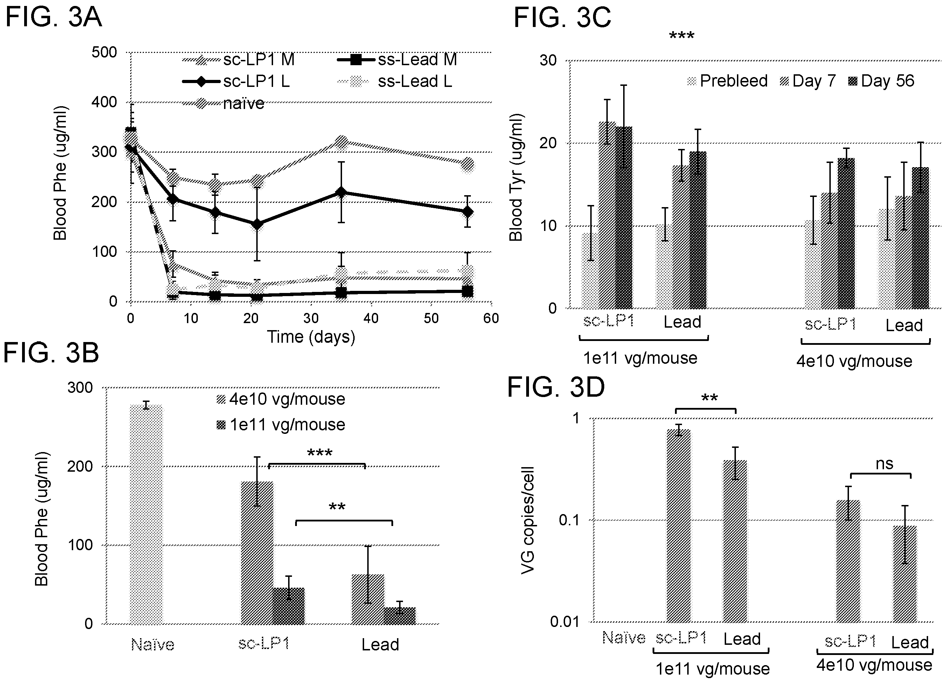

**FIGs. 3A-3D** show a comparison of liver promoter performance in PAH^{enu2} mice. rAAV8 vectors expressing mPAH from sc-LP1 or ss-A1MB2-mTTR promoter (lead) were administered into male PAH^{enu2} mice at 4e10 (L) or 1e11 (M) VG/mouse. **FIG. 3A** shows blood Phe levels during the 56-day time course. Levels are average of n=8/group except for naive animals at n=2. **FIG. 3B** shows blood Phe levels at day 56. Plasma Phe levels in animals treated with the two constructs administered at 4e10 or 1e11 VG/mouse or untreated animals are shown. **FIG. 3C** shows blood Tyr levels. Levels were measured before vector administration (prebleed) and 7 and 56 days post vector administration. Each value is an average of n=8/group. **FIG. 3D** shows vector genomes in liver on day 56. Vector copies were determined by qPCR and average of n=5/group is shown.

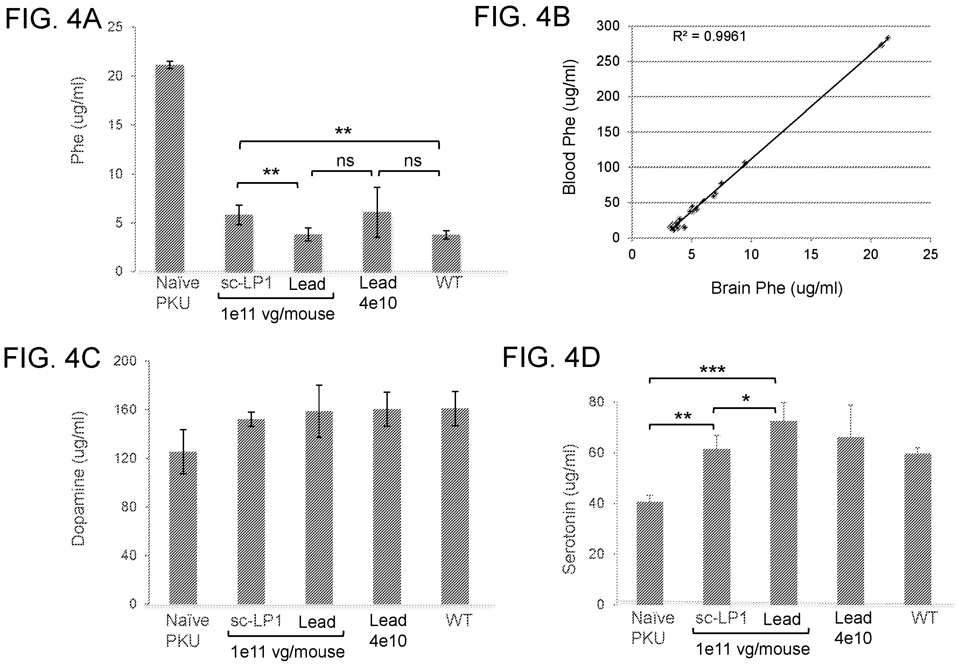

**FIGs. 4A-4D** show analysis of brains of PAH^{enu2} mice. Five animals from untreated, ss-mA1MB2-mTTR (lead) construct (4e10 and 1e11 vg/mouse) and sc-LP1 (1e11 vg/mouse) treated PAH^{enu2} mice were sacrificed 56 days post treatment, perfused with PBS and brains were collected for analysis. Balb/C mice were used as wild-type controls (WT). **FIG. 4A** shows brain Phe levels. **FIG. 4B** shows the correlation of blood and brain Phe levels. **FIG. 4C** show brain dopamine levels. **FIG. 4D** shows brain serotonin levels. All values represent an average of n=5/group (except n=2 for untreated PAH^{enu2} mice). Study was performed as described in FIGs 3A-3C.

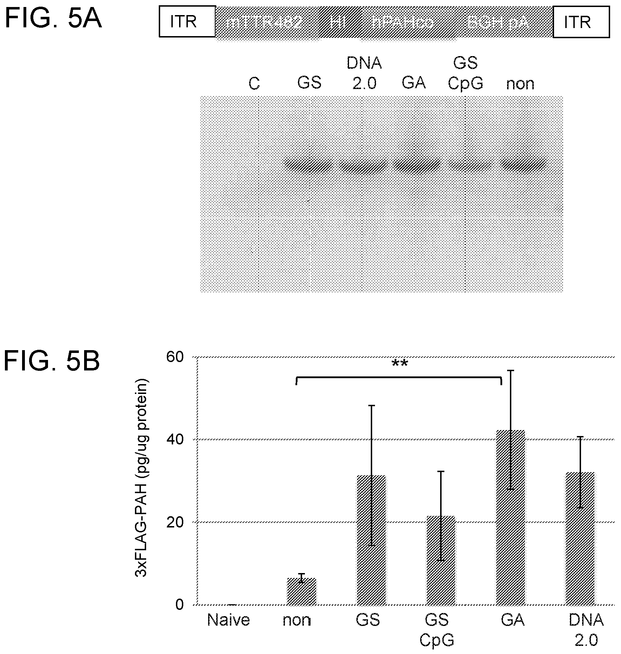

**FIGs. 5A and 5B** show codon-optimization of human PAH cDNA. cDNA encoding FLAG-tagged hPAH were cloned into mTTR482-hPAH-BGHpA expression plasmid. hPAH production was tested both in vitro and in vivo. **FIG. 5A** shows FLAG-hPAH levels in Huh7 cells. Plasmids were transfected and cell lysates were prepared 48 h later. PAH protein levels in cell lysates were analyzed by western blot. **FIG. 5B** shows FLAG-hPAH levels in livers of C57BL/6 mice. Plasmids were administered by hydrodynamic injections and livers were collected 24 h later. FLAG-tagged hPAH levels were quantitated in liver lysates using FLAG-ELISA and normalized by total protein. Values represent average on n=4-5 animals/group. Abbreviations: C, negative control plasmid; GS, Genscript, GA, GeneArt, GS CpG, Genscript sequence with CpGs removed; non, original hPAH DNA sequence; naive, untreated animals.

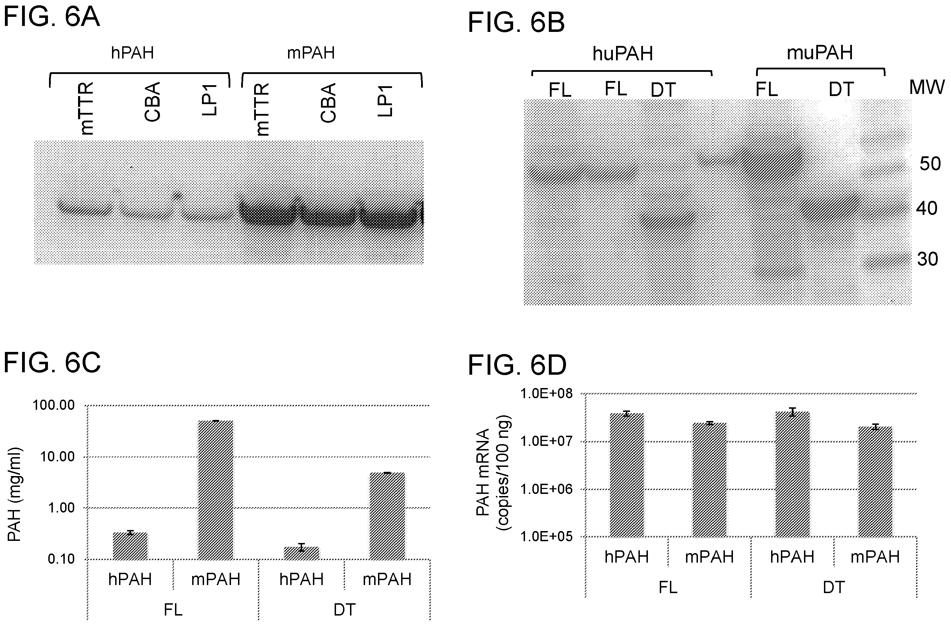

**FIG. 6A** is a Western blot showing expression of human phenylalanine hydroxylase (hPAH) and mouse phenylalanine hydroxylase (mPAH) under the control of different promoters (mTTR482, CBA, and LP1). Expression plasmids were transfected into Huh7 cells and cell lysates were analyzed two days later for FLAG-PAH levels using antibody to FLAG.
**FIG. 6B** is a Western blot showing a comparison of full-length (FL) and double truncated (DT) human and mouse PAH expression *in vitro.* Analysis as in FIG 6A.
**FIG. 6C** is a comparison of hPAH and mPAH protein levels for both full length (FL) and double truncated (DT) forms. Quantitation of FLAG-tagged proteins by ELISA was performed on Huh7 cell lysates after transfection with expression plasmids.
**FIG. 6D** is a comparison of hPAH and mPAH mRNA levels. RNA analysis by qPCR was performed on material used in FIG. 6C.

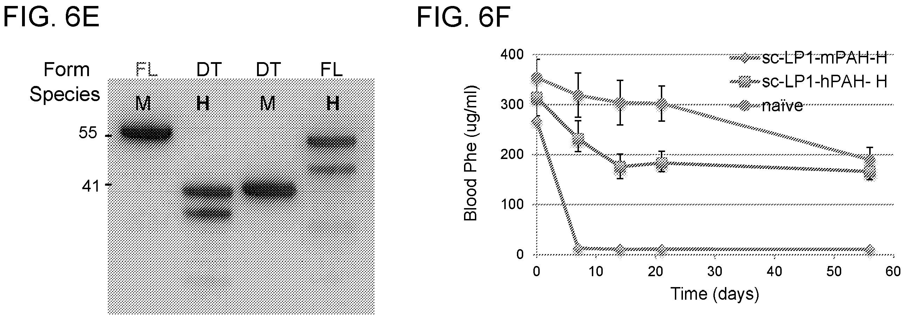

**FIG. 6E** is a Western blot showing analysis of purified hPAH and mPAH (both generated as full length and double truncated forms. Proteins were purified by FLAG-affinity column and run in SDS-PAGE gel followed by detection with anti-FLAG antibody
**FIG. 6F** shows efficacy of rAAV8 vectors encoding hPAH or mPAH in the PKU mouse model. Both hPAH and mPAH were expressed from self-complementary vectors with LP1 promoter (sc-LP1). Efficacy was measured as reduction of blood Phe levels after single IV injection into PAH^{enu2} mice.

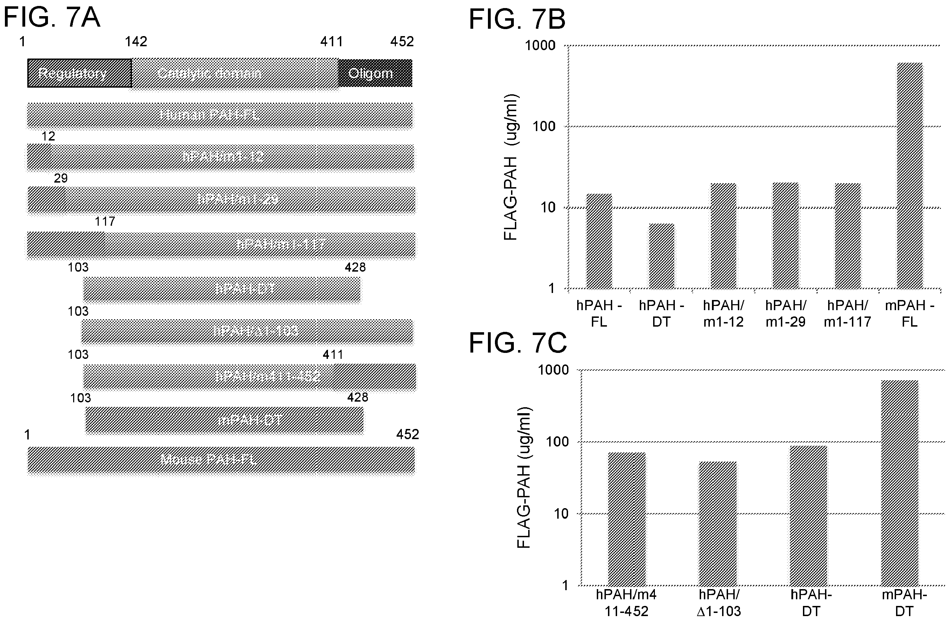

**FIGS. 7A-7C** show the generation of mouse/human hybrid PAH constructs. **FIG. 7A** shows a diagram of the mouse/ human hybrid PAH constructs. Regions derived from human (green) and mouse (gray) PAH are indicated. **FIG. 7B** shows the effect of replacing the N-terminal region of hPAH on protein expression levels. **FIG. 7C** shows the effect of changes in the C- terminal region of hPAH on protein expression levels. Experiments were performed by transfecting plasmids with CBA-PAH expression cassettes into 293T cells. After 48 hrs, cells were collected for quantitation of FLAG-PAH by FLAG ELISA.

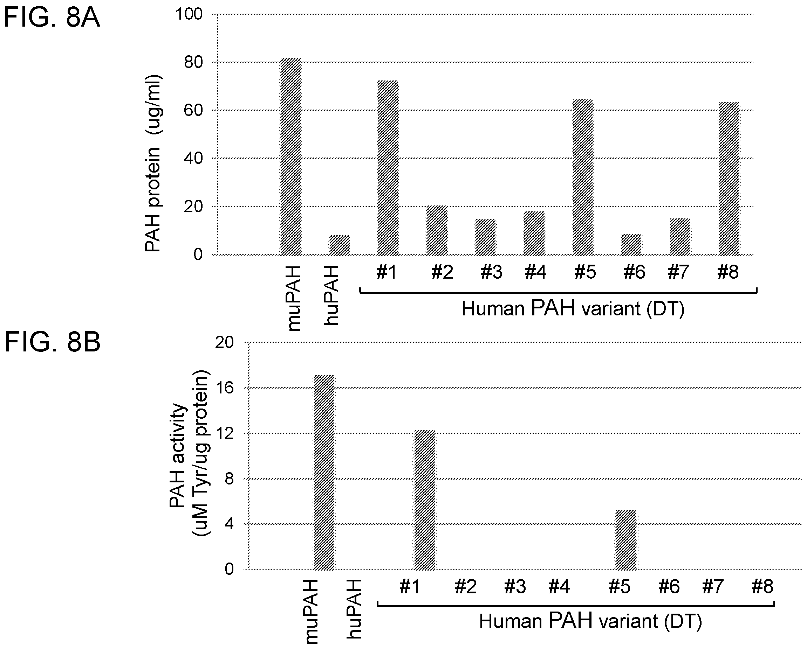

**FIGS. 8A & 8B** show the screening of double truncated hPAH (hPAH-DT) variants *in vitro.* **FIG. 8A** shows protein expression levels of plasmid-expressed hPAH protein variants (#1 to #8). **FIG. 8B** shows PAH activity levels of plasmid-expressed hPAH protein variants. All variants were transfected into 293 cells and expressed from plasmid with a CBA promoter. Results from the variant plasmids are compared to analogous plasmids expressing human or mouse PAH.

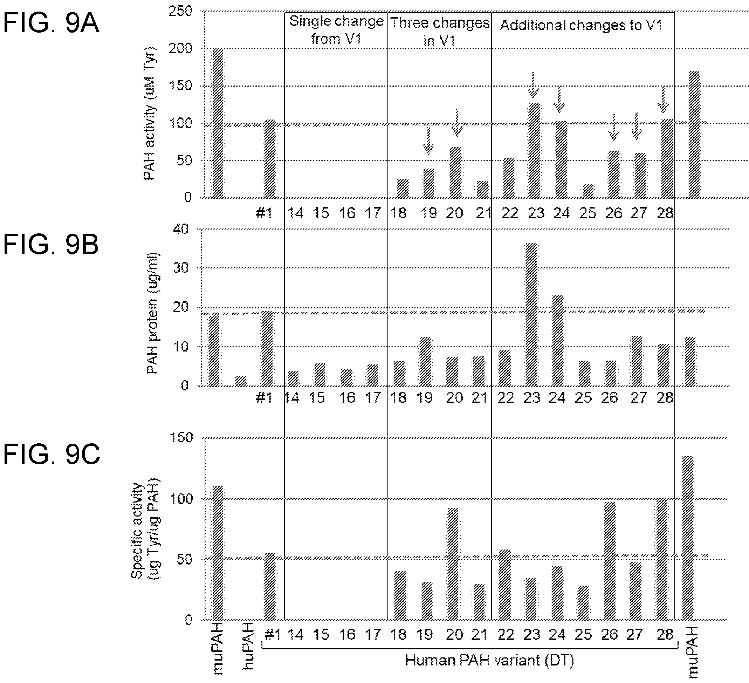

**FIGS. 9A-9C** show the characterization of hPAH-V1-DT derivatives for PAH production *in vitro.* **FIG. 9A** shows PAH activity levels of hPAH-V1-DT derivatives. **FIG. 9B** shows PAH protein levels of hPAH-V1-DT derivatives. **FIG. 9C** shows specific PAH activity of hPAH-V1-DT derivatives. Results are based on analysis of cell lysates from transfected 293 cells.

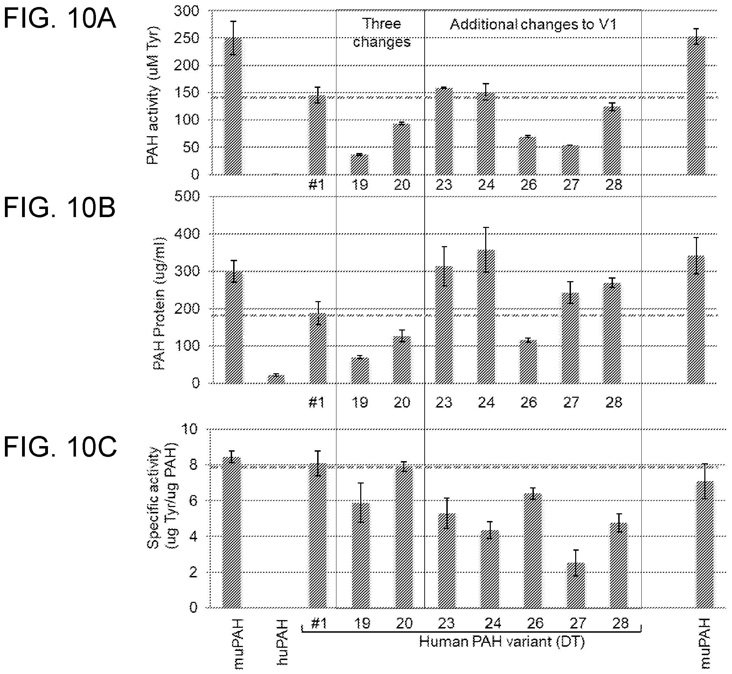

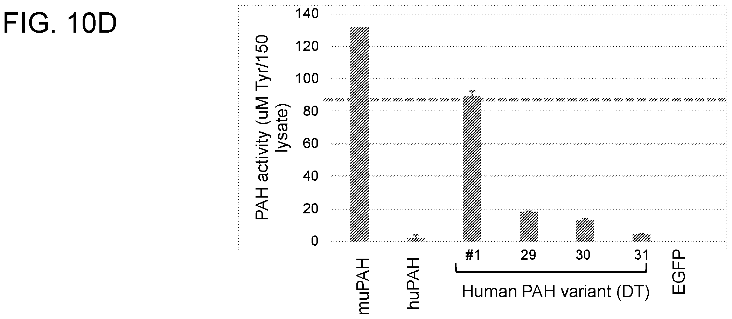

**FIGS. 10A-10C** show a repeat analysis of hPAH-V1-DT derivatives for PAH production *in vitro.* **FIG. 10A** shows PAH activity levels of selected hPAH-V1-DT derivatives. **FIG. 10B** shows PAH protein levels of hPAH-V1-DT derivatives. **FIG. 10C** shows specific PAH activity of hPAH-V1-DT derivatives. Data was generated as described in FIG. 8A and 8B. **FIGS 10D** shows PAH activity of three double-mutant version of variant-1. Data was generated as described in FIG. 8A and 8B.

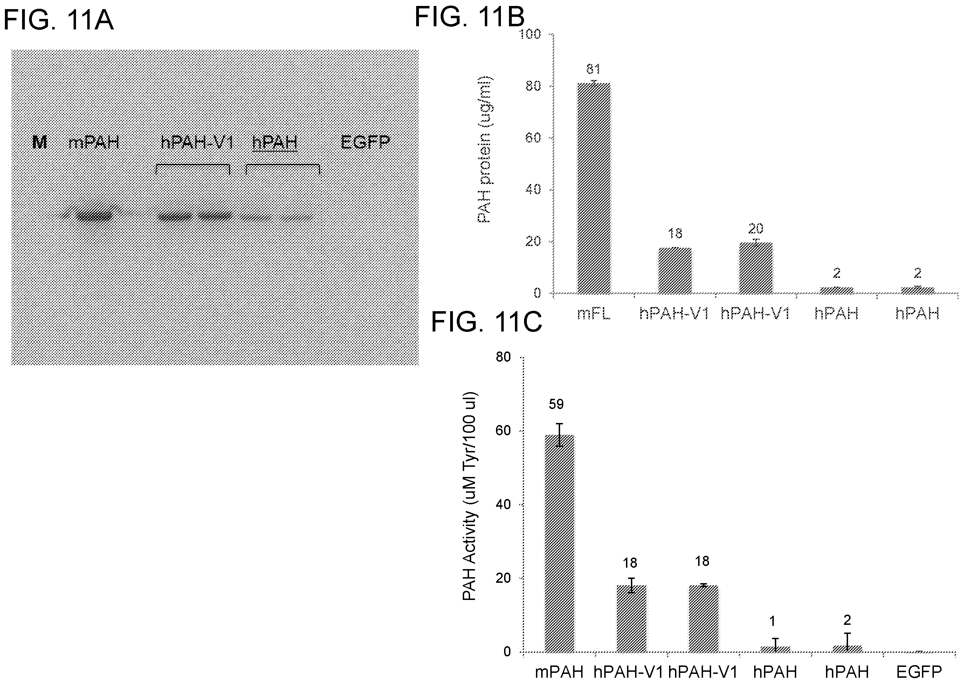

**FIGS. 11A-11C** show an *in vitro* comparison of full-length hPAH-V1 to mouse PAH (mPAH) and human PAH (hPAH). **FIG. 11A** is a Western blot analysis of protein expression. **FIG. 11B** shows PAH protein levels by FLAG-ELISA. **FIG. 11C** shows PAH activity levels. Data was generated by transfecting expression plasmids with liver promoter A1MB2-mTTR482 and encoding full-length PAH proteins into Huh7 followed by analysis of cell lysates. M indicates marker lane.

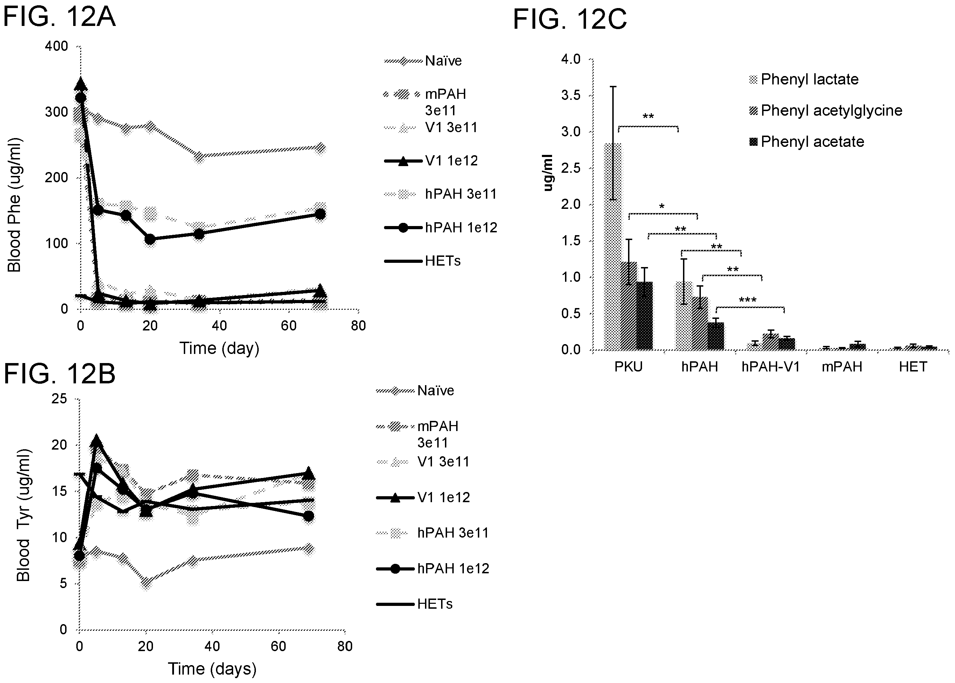

**FIGS. 12A-12C** show a comparison of efficacy of rAAV vectors expressing full-length hPAH-V1 to that of mouse and human PAH *in* PAH^{enu2} mice. **FIG. 12A** shows blood phenylalanine levels. **FIG. 12B** shows blood tyrosine levels. **FIG 12C** shows blood phenylalanine metabolite levels. P values are indicated as * is P <0.05), ** is P <0.01 and *** is P <0.001. All treatment groups consisted of AAV8 vector expressing FLAG-tagged PAH from a liver promoter. Vectors were injected by IV route at 3e11 (hPAH, hPAH-V1 and mPAH) or 1e12 vg/mouse (hPAH and hPAH-V1) on day 0. Naive PAH^{enu2} mice and heterozygous (HETs) were used as negative and positive controls, respectively.

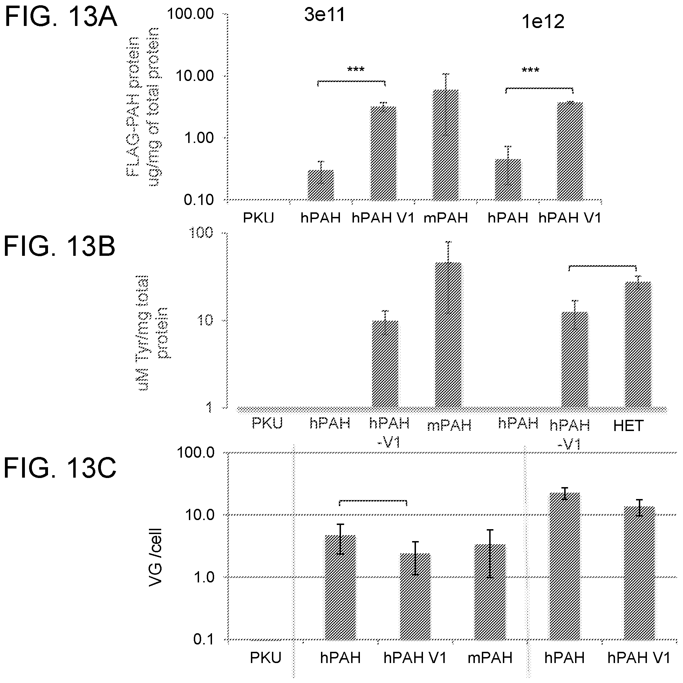

**FIGS. 13A-13C** show a quantitation of rAAV vector genomes and 3x-FLAG-PAH levels in liver. **FIG. 13A** shows PAH protein levels. **FIG. 13B** shows PAH activity levels. **FIG. 13C** shows vector genome copies in liver. Experiment was performed as described in FIGs 12A-12C and liver collected 69 days after vector administration. In each panel, data for 3e11 VG/mouse treatment groups (hPAH, hPAH-V1 and mPAH) and 1e12 vg/mouse treatment groups (hPAH, hPAH-V1) is shown. P value, *** is <0.001.

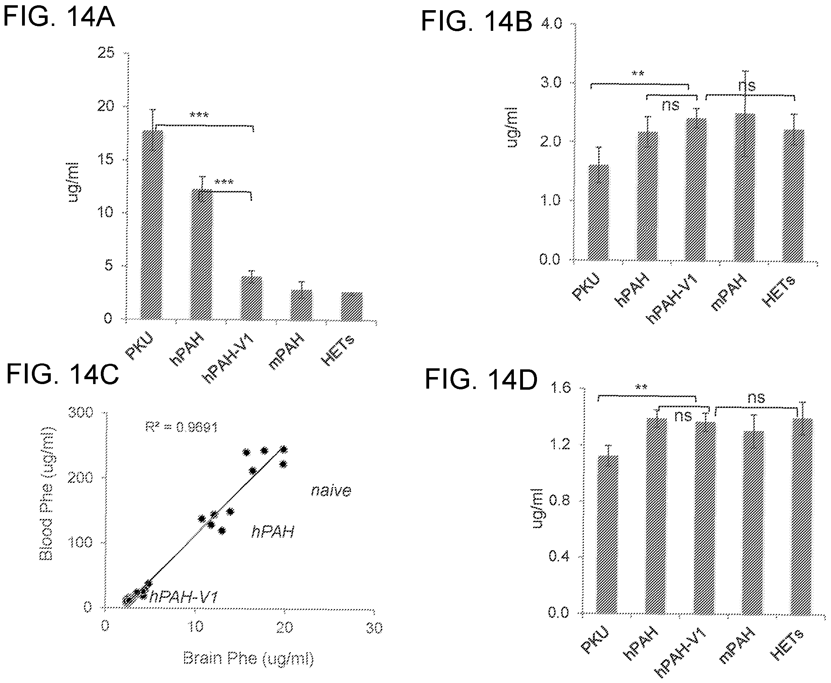

**FIGS. 14A-14D** show a quantitation of various amino acid levels in brain. **FIG. 14A** shows brain phenylalanine levels. **FIG. 14B** shows brain tyrosine levels. **FIG. 14C** shows the correlation between brain and blood phenylalanine levels. **FIG. 14D** shows brain tryptophan levels. Experiment was performed as described in FIGs 12A-12C. Data shown is from treatment groups dosed at 3e11 VG/mouse (n=5/group) (day 69). P value, *** is <0.001.

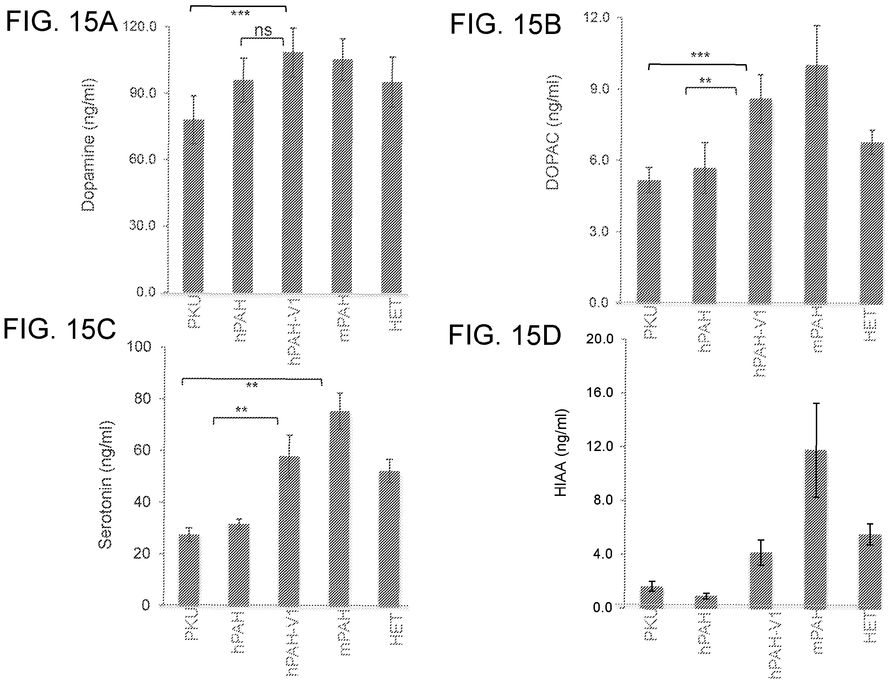

**FIGS. 15A-15D** show neurotransmitter levels in brain. **FIG. 15A** shows brain dopamine levels. **FIG. 15B** shows brain levels of DOPAC, a dopamine metabolite. **FIG. 15C** shows brain serotonin levels. **FIG. 15D** shows brain levels of HIAA, a serotonin metabolite. Experiment was performed as described in FIGs 12A-12C and values are from 3e11 VG/mouse cohorts (day 69). P value, ** is P <0.01 and *** is P <0.001.

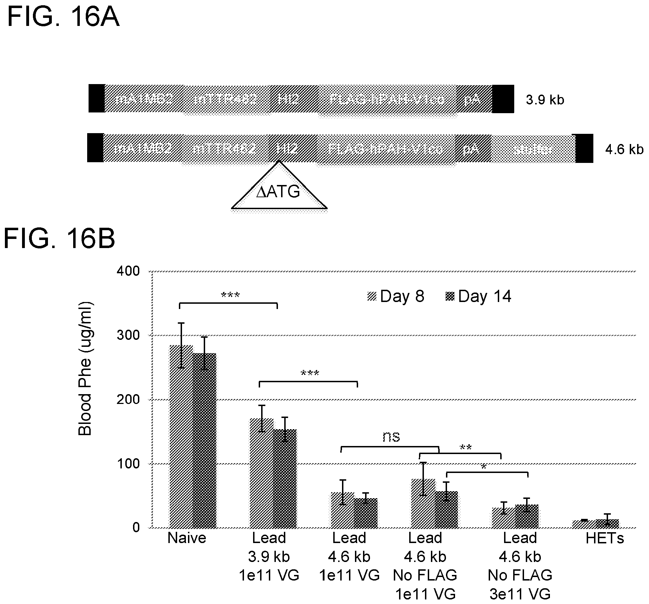

**FIGS. 16A-16B** show a comparison of 3.8 and 4.6 kb A1MB2-mTTR-hPAV-V1 vector genomes for efficacy. **FIG. 16A** shows a diagram of vectors. **FIG. 16B** shows efficacy testing in PAH^{enu2} mice. AAV8 vectors expressing hPAH-V1 from liver promoter (lead) were administered at 1e11 vg/mouse once and efficacy was measured as blood Phe levels. These vectors were also compared to 4.6 kb vectors with hPAH-V1 without the N-terminal tag (tested at 1e11 and 3e11 vg/mouse).

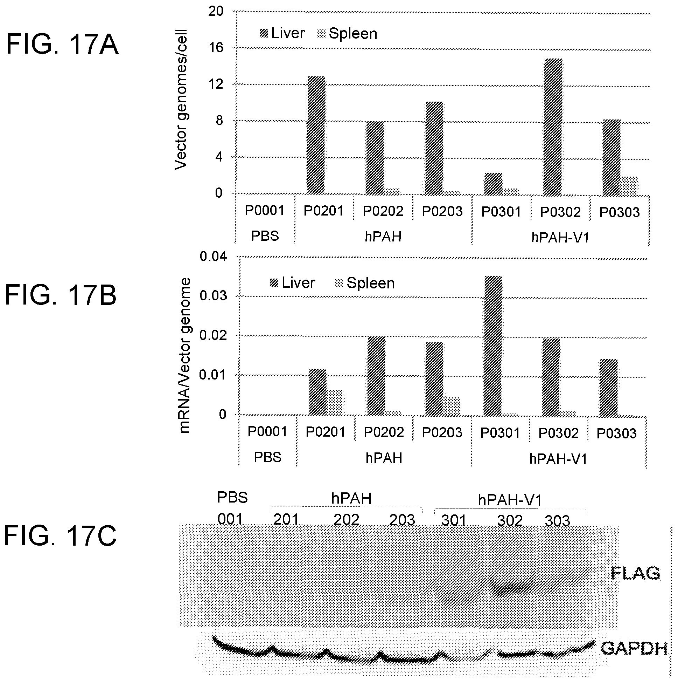

**FIGS 17A-17C** show in vivo comparison of hPAH-V1 to hPAH production in non-human primate liver after delivery with rAAV vectors. **FIG 17A** shows levels of rAAV vector genomes in livers of each individual animal. **FIG 17B** shows vector-derived mRNA levels in liver and spleen of each treated animal. The mRNA levels were normalized to vector genome copies in each animal. **FIG 17C** shows detection of FLAG-tagged PAH protein in liver homogenates. Equal lysate loading was confirmed with detection of housekeeping protein GAPDH. The experiment was performed by IV administration of 5e12 g/kg of rAAV vectors expressing FLAG-tagged PAH or PAH-V1 from A1MB2-mTTR promoter. Tissues were collected 2 weeks later for analysis of vector genomes, vector derived mRNA and PAH protein in liver.

### DETAILED DESCRIPTION

In some aspects, the invention described herein provides a variant of a human PAH (hPAH-V1) polypeptide comprising at least two four amino acid changes that confer higher PAH protein and/or activity levels *in vitro* and *in vivo* compared to wild-type hPAH. In other aspects, the invention described herein provides an improved variant of human PAH (hPAH-V1) comprising at least two, three or four amino acid changes that confer higher PAH protein and/or activity levels *in vitro* and *in vivo* compared to wild-type hPAH. In some aspects, the invention provides nucleic acid encoding said variants of a human PAH. In some aspects, the invention provides expression cassettes, recombinant adeno-associated virus (rAAV) vectors and viral particles and pharmaceutical compositions comprising the variant PAH polypeptide of the present disclosure. In further aspects, the invention provides methods for treating phenylketonuria (PKU); for example, by increasing PAH activity, increasing tyrosine and tryptophan transport into the brain, and normalizing brain neurotransmitter levels including dopamine and serotonin. In yet further aspects, the invention provides kits for treating PKU in an individual with the variant PAH of the present disclosure.

### I. General Techniques

The techniques and procedures described or referenced herein are generally well understood and commonly employed using conventional methodology by those skilled in the art, such as, for example, the widely utilized methodologies described in Molecular Cloning: A Laboratory Manual (Sambrook et al., 4th ed., Cold Spring Harbor Laboratory Press, Cold Spring Harbor, N.Y., 2012); Current Protocols in Molecular Biology (F.M. Ausubel, et al. eds., 2003); the series Methods in Enzymology (Academic Press, Inc.); PCR 2: A Practical Approach (M.J. MacPherson, B.D. Hames and G.R. Taylor eds., 1995); Antibodies, A Laboratory Manual (Harlow and Lane, eds., 1988); Culture of Animal Cells: A Manual of Basic Technique and Specialized Applications (R.I. Freshney, 6th ed., J. Wiley and Sons, 2010); Oligonucleotide Synthesis (M.J. Gait, ed., 1984); Methods in Molecular Biology, Humana Press; Cell Biology: A Laboratory Notebook (J.E. Cellis, ed., Academic Press, 1998); Introduction to Cell and Tissue Culture (J.P. Mather and P.E. Roberts, Plenum Press, 1998); Cell and Tissue Culture: Laboratory Procedures (A. Doyle, J.B. Griffiths, and D.G. Newell, eds., J. Wiley and Sons, 1993-8); Handbook of Experimental Immunology (D.M. Weir and C.C. Blackwell, eds., 1996); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Ausubel et al., eds., J. Wiley and Sons, 2002); Immunobiology (C.A. Janeway et al., 2004); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane, Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 2011).

### II. Definitions

A "vector," as used herein, refers to a recombinant plasmid or virus that comprises a nucleic acid to be delivered into a host cell, either *in vitro* or *in vivo.*

The term "polynucleotide" or "nucleic acid" as used herein refers to a polymeric form of nucleotides of any length, either ribonucleotides or deoxyribonucleotides. Thus, this term includes, but is not limited to, single-, double- or multi-stranded DNA or RNA, genomic DNA, cDNA, DNA-RNA hybrids, or a polymer comprising purine and pyrimidine bases, or other natural, chemically or biochemically modified, non-natural, or derivatized nucleotide bases. The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidates and thus can be an oligodeoxynucleoside phosphoramidate (P-NH₂) or a mixed phosphoramidate- phosphodiester oligomer. In addition, a double-stranded polynucleotide can be obtained from the single stranded polynucleotide product of chemical synthesis either by synthesizing the complementary strand and annealing the strands under appropriate conditions, or by synthesizing the complementary strand de novo using a DNA polymerase with an appropriate primer.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and multimers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present disclosure, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification.

A "recombinant viral vector" refers to a recombinant polynucleotide vector comprising one or more heterologous sequences (*i.e.,* nucleic acid sequence not of viral origin). In the case of recombinant AAV vectors, the recombinant nucleic acid is flanked by at least one and in embodiments two, inverted terminal repeat sequences (ITRs).

A "recombinant AAV vector (rAAV vector)" refers to a polynucleotide vector comprising one or more heterologous sequences (*i.e.,* nucleic acid sequence not of AAV origin) that are flanked by at least one, and in embodiments two, AAV inverted terminal repeat sequences (ITRs). Such rAAV vectors can be replicated and packaged into infectious viral particles when present in a host cell that has been infected with a suitable helper virus (or that is expressing suitable helper functions) and that is expressing AAV rep and cap gene products (*i.e.* AAV Rep and Cap proteins). When a rAAV vector is incorporated into a larger polynucleotide *(e.g.,* in a chromosome or in another vector such as a plasmid used for cloning or transfection), then the rAAV vector may be referred to as a "pro-vector" which can be "rescued" by replication and encapsidation in the presence of AAV packaging functions and suitable helper functions. An rAAV vector can be in any of a number of forms, including, but not limited to, plasmids, linear artificial chromosomes, complexed with lipids, encapsulated within liposomes, and encapsidated in a viral particle, particularly an AAV particle. A rAAV vector can be packaged into an AAV virus capsid to generate a "recombinant adeno-associated viral particle (rAAV particle)".

"Heterologous" means derived from a genotypically distinct entity from that of the rest of the entity to which it is compared or into which it is introduced or incorporated. For example, a polynucleotide introduced by genetic engineering techniques into a different cell type is a heterologous polynucleotide (and, when expressed, can encode a heterologous polypeptide). Similarly, a cellular sequence (*e.g.*, a gene or portion thereof) that is incorporated into a viral vector is a heterologous nucleotide sequence with respect to the vector.

The term "transgene" refers to a polynucleotide that is introduced into a cell and is capable of being transcribed into RNA and optionally, translated and/or expressed under appropriate conditions. In aspects, it confers a desired property to a cell into which it was introduced, or otherwise leads to a desired therapeutic or diagnostic outcome.

"Chicken β-actin (CBA) promoter" refers to a polynucleotide sequence derived from a chicken β-actin gene (*e.g., Gallus gallus* beta actin, represented by GenBank Entrez Gene ID 396526). As used herein, "chicken β-actin promoter" may refer to a promoter containing a cytomegalovirus (CMV) early enhancer element, the promoter and first exon and intron of the chicken β-actin gene, and the splice acceptor of the rabbit beta-globin gene, such as the sequences described in Miyazaki, J. et al. (1989) Gene 79(2):269-77. As used herein, the term "CAG promoter" may be used interchangeably. As used herein, the term "CMV early enhancer/chicken beta actin (CAG) promoter" may be used interchangeably.

The terms "genome particles (gp)," "genome equivalents," or "genome copies" as used in reference to a viral titer, refer to the number of virions containing the recombinant AAV DNA genome, regardless of infectivity or functionality. The number of genome particles in a particular vector preparation can be measured by procedures such as described in the Examples herein, or for example, in Clark et al. (1999) Hum. Gene Ther., 10:1031-1039; Veldwijk et al. (2002) Mol. Ther., 6:272-278.

The term "vector genome (vg)" as used herein may refer to one or more polynucleotides comprising a set of the polynucleotide sequences of a vector, *e.g.,* a viral vector. A vector genome may be encapsidated in a viral particle. Depending on the particular viral vector, a vector genome may comprise single-stranded DNA, double-stranded DNA, or single-stranded RNA, or double-stranded RNA. A vector genome may include endogenous sequences associated with a particular viral vector and/or any heterologous sequences inserted into a particular viral vector through recombinant techniques. For example, a recombinant AAV vector genome may include at least one ITR sequence flanking a promoter, a stuffer, a sequence of interest (*e.g*., an RNAi), and a polyadenylation sequence. A complete vector genome may include a complete set of the polynucleotide sequences of a vector. In some embodiments, the nucleic acid titer of a viral vector may be measured in terms of vg/mL. Methods suitable for measuring this titer are known in the art (*e.g*., quantitative PCR).

The terms "infection unit (iu)," "infectious particle," or "replication unit," as used in reference to a viral titer, refer to the number of infectious and replication-competent recombinant AAV vector particles as measured by the infectious center assay, also known as replication center assay, as described, for example, in McLaughlin et al. (1988) J. Virol., 62:1963-1973.

The term "transducing unit (tu)" as used in reference to a viral titer, refers to the number of infectious recombinant AAV vector particles that result in the production of a functional transgene product as measured in functional assays such as described in Examples herein, or for example, in Xiao et al. (1997) Exp. Neurobiol., 144:113-124; or in Fisher et al. (1996) J. Virol., 70:520-532 (LFU assay).

An "inverted terminal repeat" or "ITR" sequence is a term well understood in the art and refers to relatively short sequences found at the termini of viral genomes which are in opposite orientation.

An "AAV inverted terminal repeat (ITR)" sequence, a term well-understood in the art, is an approximately 145-nucleotide sequence that is present at both termini of the native single-stranded AAV genome. The outermost 125 nucleotides of the ITR can be present in either of two alternative orientations, leading to heterogeneity between different AAV genomes and between the two ends of a single AAV genome. The outermost 125 nucleotides also contains several shorter regions of self-complementarity (designated A, A', B, B', C, C' and D regions), allowing intrastrand base-pairing to occur within this portion of the ITR.

A "terminal resolution sequence" or "trs" is a sequence in the D region of the AAV ITR that is cleaved by AAV rep proteins during viral DNA replication. A mutant terminal resolution sequence is refractory to cleavage by AAV rep proteins.

"AAV helper functions" refer to functions that allow AAV to be replicated and packaged by a host cell. AAV helper functions can be provided in any of a number of forms, including, but not limited to, helper virus or helper virus genes which aid in AAV replication and packaging. Other AAV helper functions are known in the art such as genotoxic agents.

A "helper virus" for AAV refers to a virus that allows AAV (which is a defective parvovirus) to be replicated and packaged by a host cell. A helper virus provides "helper functions" which allow for the replication of AAV. A number of such helper viruses have been identified, including adenoviruses, herpesviruses and, poxviruses such as vaccinia and baculovirus. The adenoviruses encompass a number of different subgroups, although Adenovirus type 5 of subgroup C (Ad5) is most commonly used. Numerous adenoviruses of human, non-human mammalian and avian origin are known and are available from depositories such as the ATCC. Viruses of the herpes family, which are also available from depositories such as ATCC, include, for example, herpes simplex viruses (HSV), Epstein-Barr viruses (EBV), cytomegaloviruses (CMV) and pseudorabies viruses (PRV). Examples of adenovirus helper functions for the replication of AAV include E1A functions, E1B functions, E2A functions, VA functions and E4orf6 functions. Baculoviruses available from depositories include *Autographa californica* nuclear polyhedrosis virus.

A preparation of rAAV is said to be "substantially free" of helper virus if the ratio of infectious AAV particles to infectious helper virus particles is at least about 10²:l; at least about 10⁴:l, at least about 10⁶:l; or at least about 10⁸:l or more. In some embodiments, preparations are also free of equivalent amounts of helper virus proteins (*i.e.,* proteins as would be present as a result of such a level of helper virus if the helper virus particle impurities noted above were present in disrupted form). Viral and/or cellular protein contamination can generally be observed as the presence of Coomassie staining bands on SDS gels (*e.g.,* the appearance of bands other than those corresponding to the AAV capsid proteins VP1, VP2 and VP3).

"Percent (%) sequence identity" with respect to a reference polypeptide or nucleic acid sequence is defined as the percentage of amino acid residues or nucleotides in a candidate sequence that are identical with the amino acid residues or nucleotides in the reference polypeptide or nucleic acid sequence, after aligning the sequences and introducing gaps, if necessary, to achieve the maximum percent sequence identity, and not considering any conservative substitutions as part of the sequence identity. Alignment for purposes of determining percent amino acid or nucleic acid sequence identity can be achieved in various ways that are within the skill in the art, for instance, using publicly available computer software programs, for example, those described in Current Protocols in Molecular Biology (Ausubel et al., eds., 1987), Supp. 30, section 7.7.18, Table 7.7.1, and including BLAST, BLAST-2, ALIGN or Megalign (DNASTAR) software. A preferred alignment program is ALIGN Plus (Scientific and Educational Software, Pennsylvania). Those skilled in the art can determine appropriate parameters for measuring alignment, including any algorithms needed to achieve maximal alignment over the full length of the sequences being compared. For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows: 100 times the fraction X/Y, where X is the number of amino acid residues scored as identical matches by the sequence alignment program in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows: 100 times the fraction W/Z, where W is the number of nucleotides scored as identical matches by the sequence alignment program in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

An "isolated" molecule (*e.g.,* nucleic acid or protein) or cell means it has been identified and separated and/or recovered from a component of its natural environment.

An "effective amount" is an amount sufficient to effect beneficial or desired results, including clinical results (*e.g.,* amelioration of symptoms, achievement of clinical endpoints, and the like). An effective amount can be administered in one or more administrations. In terms of a disease state, an effective amount is an amount sufficient to ameliorate, stabilize, or delay development of a disease.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

As used herein, "treatment" is an approach for obtaining beneficial or desired clinical results. For purposes of this disclosure, beneficial or desired clinical results include, but are not limited to, alleviation of symptoms, diminishment of extent of disease, stabilized (*e.g.,* not worsening) state of disease, preventing spread (*e.g.,* metastasis) of disease, delay or slowing of disease progression, amelioration or palliation of the disease state, and remission (whether partial or total), whether detectable or undetectable. "Treatment" can also mean prolonging survival as compared to expected survival if not receiving treatment.

As used herein, the term "prophylactic treatment" refers to treatment, wherein an individual is known or suspected to have or be at risk for having a disorder but has displayed no symptoms or minimal symptoms of the disorder. An individual undergoing prophylactic treatment may be treated prior to onset of symptoms.

As used herein, "phenylalanine hydroxylase (PAH)" is an enzyme (EC 1.14.16.1) that catalyzes the hydroxylation of the aromatic side-chain of phenylalanine to generate tyrosine. PAH is a monooxygenase that uses tetrahydrobiopterin (BH4, a pteridine cofactor) and a non-heme iron for catalysis. During the reaction, molecular oxygen is heterolytically cleaved with sequential incorporation of one oxygen atom into BH4 and phenylalanine substrate. The hydroxylation of phenylalanine to tyrosine and is the rate-limiting step in phenylalanine catabolism and a deficiency of this enzyme activity results in the autosomal recessive disorder phenylketonuria. PAH may also be referred to as PH, PKU, or PKU1. PAH is a multi-domain protein consisting of N-terminal regulatory (1-117), central catalytic (118-410) and C-terminal tetramerization domains (411-452). Human PAH is provided in GenBank; for example, NM_000277, NM_00877, NM_001354304, NP_000268, NP_032803, NP_001341233, AAA60082.1, AAH26251.1, AAC51772.1, and AAL78816.1 (GI: 18765885). An example of a human PAH is provided as SEQ ID NO:1.

As used herein, "Phenylketonuria (PKU)" refers to a genetic deficiency of liver enzyme phenylalanine hydroxylase (PAH). In the absence of any treatment, the severe form of PKU leads to highly elevated blood Phe levels that are neurotoxic and associated with severe mental retardation.

"mTTR promoter" refers to a polynucleotide sequence derived from the murine transthyretin gene. An example of a mTTR promoter, mTTR482, is provided by Kyostio-Moore, (2016) and Nambiar (2017).

"Modified prothrombin enhancer (mPrT2)" refers to two copies of polynucleotide sequence derived from a human prothrombin gene. An example of a mPrT2 enhancer is provided by (McEachern 2006, Jacobs 2008). An example of a mPrT2 sequence is provided by SEQ ID NO:7.

"Modified alpha1-microbikunin (mA1MB2)" refers to two copies of polynucleotide sequence derived from a human alpha1-microglobulin/bikunin gene. An example of a mA1MB2 is enhancer by (McEachern 2006, Jacobs 2008). An example of a mA1MB2 sequence is provided by SEQ ID NO:8.

"Modified mouse albumin enhancer (mEalb)" refers to a polynucleotide sequence derived from the murine albumin gene. An example of a mEalb enhancer is provided by (Kramer 2003). An example of a mEalb sequence is provided by SEQ ID NO:9.

"Hepatitis B virus enhancer II (HE11)" refers to a polynucleotide sequence derived from hepatitis B virus, located upstream of the PreCore promoter. An example of a hEII enhancer is provided by (Kramer 2003). An example of a HEII sequence is provided by SEQ ID NO: 10.

"CRM8" refers to a cis-acting regulatory module derived from a polynucleotide sequence from the human Serpina1 gene (Chuah 2014). An example of a CRM8 sequence is provided by SEQ ID NO: 11.

"Alb 3'" refers to a polynucleotide sequence 3' to the coding region of the human albumin gene. An example of an Alb 3' element is provided by Wooddell (2008). An example of an Alb 3' sequence is provided by SEQ ID NO: 12. "Alb37SMAR" refers to Alb3' linked to a scaffold/matrix attachment region of the human alpha1-antitrypsin gene (AF156542). An example of a Alb37SMAR sequence is provided by SEQ ID NO:13.

Reference to "about" a value or parameter herein includes (and describes) embodiments that are directed to that value or parameter *per se.* For example, description referring to "about X" includes description of "X."

As used herein, the singular form of the articles "a," "an," and "the" includes plural references unless indicated otherwise.

It is understood that aspects and embodiments of the disclosure described herein include "comprising," "consisting," and/or "consisting essentially of' aspects and embodiments.

### III. PAH Variants

In some aspects, the invention provides variant PAH polypeptides that confer higher PAH expression and/or activity levels when expressed in an individual. In some embodiments, the variant PAH polypeptide comprises at least two amino acid substitutions, wherein the amino acid substitutions are at sites selected from M180, K199, S250, S251, H264, G256, G272, G275, P279, T323 and F327 of a wild-type human PAH polypeptide. In some embodiments, the variant PAH polypeptide comprises at least two amino acid substitutions at H264 and G275. In some embodiments, the variant PAH polypeptide comprises at least three amino acid substitutions, wherein the amino acid substitutions are at sites selected from M180, K199, S250, and G256 of a wild-type human PAH polypeptide. In some embodiments, the variant PAH polypeptide comprises four amino acid substitutions at sites M180, K199, S250, and G256 of a wild-type human PAH polypeptide. In some embodiments, the variant PAH polypeptide comprises five amino acid substitutions at M180, K199, S250, S251, G256, G272 and P279. In some embodiments, the variant PAH polypeptide comprised six amino acid substitutions at M180, K199, S250, G256, T323 and F327. In some embodiments, the variant PAH polypeptide comprises at least two amino acid substitutions of H264P and G275H. In some embodiments, the variant PAH polypeptide comprises at least three amino acid substitutions selected from M180T, K199P, S250P, and G256A. In some embodiments, the variant PAH polypeptide comprises amino acid substitution comprising K199P, S250P, and G256A; M180T, S250P, and G256A; M180T, K199P, and G256A; or M180T, K199P, and S250P. In some embodiments, the variant PAH polypeptide further comprises amino acid substitutions H264P, G272A, G272P, P275L, P279Q, G272P and P275L, or T323R and F327T amino acid substitutions. In some embodiments, the variant PAH polypeptide is any one of the variant PAH polypeptides presented in Tables 1-3. In some embodiments, the variant PAH polypeptide comprises amino acid substitutions M180T, K199P, S250P, and G256A and comprises further amino acid substitutions while maintaining at least about the phenylalanine hydroxylase activity of wild-type PAH. In some embodiments, the location of the amino acid substitutions is based on wild-type human PAH polypeptide; for example, the human PAH polypeptide comprising the amino acid sequence of SEQ ID NO: 1.

In some embodiments, the variant PAH polypeptide is a human PAH polypeptide. In some embodiments, the variant PAH polypeptide comprises the amino acid sequence of SEQ ID NO:3. In some embodiments, variant PAH polypeptide comprises an amino acid sequences that is at least about any of 80%, 85%, 90%, 95% or 99% identical to the amino acid sequence of SEQ ID NO:3. In some embodiments, the variant PAH polypeptide comprising an amino acid sequences that is at least about any of 80%, 85%, 90%, 95% or 99% identical to the amino acid sequence of SEQ ID NO:3 has at least about 25%, 50%, 75%, 100%, or greater than 100% the phenylalanine hydroxylase activity of wild-type PAH. In some embodiments, the variant PAH polypeptide comprising an amino acid sequences that is at least about any of 80%, 85%, 90%, 95% or 99% identical to the amino acid sequence of SEQ ID NO:3 has at least about 25%, 50%, 75%, 100%, or greater than 100% the phenylalanine hydroxylase activity of wild-type PAH of SEQ ID NO: 1.

In some embodiments, the variant PAH polypeptide is a truncated PAH polypeptide that maintains phenylalanine hydroxylase activity. In some embodiments, the truncated PAH polypeptide comprise an N-terminal truncation. In some embodiments, the N-terminal truncation is a truncation of part or all of the N-terminal regulatory domain. In some embodiments, the N-terminal truncation is a deletion of amino acid residue 1 to about amino acid residue 102 of the wild-type human PAH polypeptide (e.g., the PAH polypeptide of SEQ ID NO: 1). In some embodiments, the truncated PAH polypeptide comprises a C-terminal truncation. In some embodiments, the C-terminal truncation is a truncation of part or all of the tetramerization domain. In some embodiments, the C-terminal truncation is a deletion of amino acid residues from about 429 to 452 of the wild-type human PAH polypeptide (*e.g.,* the PAH polypeptide of SEQ ID NO: 1). In some embodiments, the variant PAH polypeptide comprises an N-terminal truncation and a C-terminal truncation. In some embodiments, the truncated PAH polypeptide comprises a truncation of part or all of the N-terminal regulatory sequence and part of all of the tetramerization domain. In some embodiments, the truncated PAH polypeptide comprises a deletion of amino acid residue 1 to about amino acid residue 102 and from about 429 to about 452 of the wild-type human PAH polypeptide (*e.g.,* the PAH polypeptide of SEQ ID NO: 1). In some embodiments, the variant PAH polypeptide comprises an amino acid sequence corresponding to amino acid residues from about 102 to about 428 of the wild-type PAH polypeptide (*e.g.,* the PAH polypeptide of SEQ ID NO: 1). In some embodiments, the variant PAH polypeptide comprises an amino acid sequence corresponding to amino acid residues from 102 to 428 of the wild-type PAH polypeptide (*e.g.,* the PAH polypeptide of SEQ ID NO: 1). In some embodiments, the truncated PAH polypeptide further comprises four amino acid substitutions at sites M180, K199, S250, and G256 of a wild-type human PAH polypeptide (*e.g.,* the PAH polypeptide of SEQ ID NO: 1). In some embodiments, the truncated PAH polypeptide comprises four amino acid substitutions selected from M180T, K199P, S250P, and G256A. In some embodiments, the truncated variant PAH polypeptide further comprises any one of the combinations of amino acid substitutions presented in Tables 1-3. In some embodiments, the truncated PAH polypeptide has at least about 25%, 50%, 75%, 100%, or greater than 100% the phenylalanine hydroxylase activity of wild-type PAH *(e.g.,* the PAH polypeptide of SEQ ID NO:1).

In some embodiments, the variant PAH polypeptide comprises one or more amino acid substitutions at one or more of the following sites: G33, G46, G103, G139, G148, G188, G218, G239, G247, G257, G272, G289, G307, G312, G332, G337, G344, G352, or G442. In some embodiments, the variant PAH polypeptide comprises one of the following amino acid substitutions: G33A, G46(A,P), G103A, G139(A,P), G148A, G188A, G218A, G239A, G247A, G257A, G272A, G289A, G307A, G312A, G332A, G337A, G344A, G352A, or G442A. In some embodiments, the variant #1 PAH polypeptide further comprises one or more amino acid substitutions at one or more of the following sites: G33, G46, G103, G139, G148, G188, G218, G239, G247, G257, G272, G289, G307, G312, G332, G337, G344, G352, or G442. In some embodiments, variant #1 PAH polypeptide comprises one or more of the following amino acid substitutions: G33A, G46(A,P), G103A, G139(A,P), G148A, G188A, G218A, G239A, G247A, G257A, G272A, G289A, G307A, G312A, G332A, G337A, G344A, G352A, or G442A.

In some embodiments, the variant PAH polypeptide (*e.g.,* variant #1 PAH polypeptide) comprises one or more of the following amino acid substitutions: P9G, G10V, G12S, K184R, K192R, S196A, Y206H, H220R, Q336E, E360D, I374C, N376E, N401T, I421V, I441V, S446H. In some embodiments, the variant PAH polypeptide (*e.g.,* variant #1) further comprises a serine residue (Ser453) at the C-terminus of human PAH.

In some embodiments, the variant PAH polypeptide (*e.g.,* variant #1 PAH polypeptide) comprises one or more of the following amino acid substitutions: F240W, A246P, G247A, Y268W, C284F, T323R, F327Y, E319P, I306(Y,F), K113P, G188A, F191Y, T193R, Y206H, G337P, N376P.

In some embodiments, the variant PAH polypeptide (*e.g.,* variant #1 PAH polypeptide) comprises any amino acid substitutions to eliminate potential protease cleavage sites. In some embodiments, the amino acid substitutions to eliminate potential protease cleavage sites are within PAH fragments 270 -295 and 380 - 405.

In some embodiments, the variant PAH polypeptide (*e.g.,* variant #1 PAH polypeptide) comprises post-translational modifications to enhance the stability of human PAH. In some embodiments, the variant PAH polypeptide comprises a post-translational modification such as pegylation and nitrosylations of Cys residues in particular I374C by external nitrosylating agents.

### III. Nucleic Acids

In some aspects, the invention provides nucleic acid encoding a variant PAH polypeptide that confers higher PAH activity levels when expressed in an individual. In some embodiments, the nucleic acid encodes a variant PAH polypeptide comprises at least three amino acid substitutions, wherein the amino acid substitutions are at sites selected from M180, K199, S250, and G256 of a wild-type human PAH polypeptide. In some embodiments, the nucleic acid encodes a variant PAH polypeptide comprising four amino acid substitutions at sites M180, K199, S250, and G256 of a wild-type human PAH polypeptide. In some embodiments, the nucleic acid encodes a variant PAH polypeptide comprising at least three amino acid substitutions selected from M180T, K199P, S250P, and G256A. In some embodiments, the nucleic acid encodes a variant PAH polypeptide comprising amino acid substitutions M180T, K199P, S250P, and G256A. In some embodiments, the nucleic acid encodes a variant PAH polypeptide comprising amino acid substitution comprises K199P, S250P, and G256A; M180T, S250P, and G256A; M180T, K199P, and G256A; or M180T, K199P, and S250P. In some embodiments, the nucleic acid encodes a variant PAH polypeptide comprising amino acid substitutions M180T, K199P, S250P, and G256A. In some embodiments, the nucleic acid encodes a variant PAH polypeptide further comprising amino acid substitutions H264P, G272A, G272P, P275L, P279Q, G272P and P275L, or T323R and F327T amino acid substitutions. In some embodiments, the nucleic acid encodes a variant PAH polypeptide that is any one of the variant PAH polypeptides presented in Tables 1-3. In some embodiments, the nucleic acid encodes a variant PAH polypeptide comprising amino acid substitutions M180T, K199P, S250P, and G256A and comprises further amino acid substitutions while maintaining at least about the phenylalanine hydroxylase activity of wild-type PAH. In some embodiments, the location of the amino acid substitutions encoded by the nucleic acid is based on wild-type human PAH polypeptide; for example, the human PAH polypeptide comprising the amino acid sequence of SEQ ID NO: 1.

In some embodiments, the nucleic acid encodes a variant human PAH polypeptide. In some embodiments, the nucleic acid encodes a variant PAH polypeptide comprising the amino acid sequence of SEQ ID NO:3. In some embodiments, the nucleic acid encodes a variant PAH polypeptide comprising an amino acid sequences that is at least about any of 80%, 85%, 90%, 95% or 99% identical to the amino acid sequence of SEQ ID NO:3. In some embodiments, the nucleic acid encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH. In some embodiments, the nucleic acid encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH of SEQ ID NO: 1.

In some embodiments, the nucleic acid encodes a variant PAH polypeptide that is a truncated PAH polypeptide that maintains phenylalanine hydroxylase activity. In some embodiments, the nucleic acid encodes a truncated PAH polypeptide comprising an N-terminal truncation. In some embodiments, the nucleic acid encodes a N-terminal truncation that is a truncation of part or all of the N-terminal regulatory domain. In some embodiments, the nucleic acid encodes a N-terminal truncation comprising a deletion of amino acid residue 1 to about amino acid residue 102 of the wild-type human PAH polypeptide (*e.g.,* the PAH polypeptide of SEQ ID NO: 1). In some embodiments, the nucleic acid encodes a truncated PAH polypeptide comprising a C-terminal truncation. In some embodiments, the nucleic acid encodes a C-terminal truncation that is a truncation of part or all of the tetramerization domain. In some embodiments, the nucleic acid encodes a C-terminal truncation comprising a deletion of amino acid residues from about 429 to 452 of the wild-type human PAH polypeptide (*e.g.,* the PAH polypeptide of SEQ ID NO: 1). In some embodiments, the nucleic acid encodes a variant PAH polypeptide comprising an N-terminal truncation and a C-terminal truncation. In some embodiments, the truncated PAH polypeptide comprises a truncation of part or all of the N-terminal regulatory sequence and part of all of the tetramerization domain. In some embodiments, the truncated PAH polypeptide comprises a deletion of amino acid residue 1 to about amino acid residue 102 and from about 429 to about 452 of the wild-type human PAH polypeptide (*e.g.,* the PAH polypeptide of SEQ ID NO: 1). In some embodiments, the nucleic acid encodes a variant PAH polypeptide comprising an amino acid sequence corresponding to amino acid residues from about 102 to about 428 of the wild-type PAH polypeptide (*e.g.,* the PAH polypeptide of SEQ ID NO: 1). In some embodiments, the nucleic acid encodes a variant PAH polypeptide comprises an amino acid sequence corresponding to amino acid residues from 102 to 428 of the wild-type PAH polypeptide (*e.g.,* the PAH polypeptide of SEQ ID NO: 1). In some embodiments, the nucleic acid encodes a truncated PAH polypeptide further comprising four amino acid substitutions at sites M180, K199, S250, and G256 of a wild-type human PAH polypeptide (*e.g.,* the PAH polypeptide of SEQ ID NO: 1). In some embodiments, the nucleic acid encodes a truncated PAH polypeptide comprising four amino acid substitutions selected from M180T, K199P, S250P, and G256A. In some embodiments, the nucleic acid encoding a truncated variant PAH polypeptide further comprises any one of the combinations of amino acid substitutions presented in Tables 1-3. In some embodiments, the nucleic acid encodes a truncated PAH polypeptide has at least about 25%, 50%, 75%, 100%, or greater than 100% the phenylalanine hydroxylase activity of wild-type PAH (*e.g.,* the PAH polypeptide of SEQ ID NO:1).

In some embodiments, the nucleic acid encoding the variant PAH polypeptide is operably linked to a promoter. In some embodiments, the promoter is selected from a cytomegalovirus (CMV) immediate early promoter, an RSV LTR, a MoMLV LTR, a phosphoglycerate kinase- 1 (PGK) promoter, a simian virus 40 (SV40) promoter, a CK6 promoter, a mouse transthyretin promoter (mTTR), a mTTR482 promoter, a mA1MB2-mTTR482 promoter, a TK promoter, a tetracycline responsive promoter (TRE), an HBV promoter, an hAAT promoter, a LSP promoter, an LP1 promoter, a chimeric liver-specific promoter (LSP), an E2F promoter, a telomerase (hTERT) promoter; a cytomegalovirus enhancer/chicken beta-actin/Rabbit β-globin promoter (CAG) promoter, an elongation factor 1-alpha promoter (EFl-alpha) promoter, a human β-glucuronidase promoter, a chicken β-actin (CBA) promoter, a retroviral Rous sarcoma virus (RSV) LTR promoter, a dihydrofolate reductase promoter, and a 13-actin promoter. In some embodiments, the promoter is an LP1 promoter or an mA1MB2-mTTR482 promoter.

In some embodiments, the nucleic acid further comprises a polyadenylation signal. In some embodiments, the polyadenylation signal is a bovine growth hormone polyadenylation signal, an SV40 polyadenylation signal, or a HSV TK pA. In some embodiments, the polyadenylation signal is a synthetic polyadenylation signal as described in Levitt, N et al. (1989), Genes Develop. 3:1019-1025.

In some embodiments, the nucleic acid further comprises an intron. A variety of introns for use in the invention are known to those of skill in the art, and include the MVM intron, the F IX truncated intron 1, the β-globin SD/immunoglobin heavy chain SA, the adenovirus SD/immunoglobin SA, the SV40 late SD/SA (19S/16S), and the hybrid adenovirus SD/IgG SA. (Wu *et al.* 2008, Kurachi *et al.,* 1995, Choi *et al.* 2014, Wong *et al.,* 1985, Yew *et al.* 1997, Huang and Gorman (1990). In some embodiments, the intron is a chicken β-actin (CBA)/rabbit β-globin hybrid intron. In some embodiments, intron is a chicken β-actin (CBA)/rabbit β-globin hybrid promoter and intron where all the ATG sites are removed to minimize false translation start sites (SEQ ID NO:15).

In some embodiments, a nucleic acid may include a (one or more) stuffer nucleic acids. In some embodiments, the stuffer nucleic acid may comprise a sequence that encodes a reporter polypeptide. As will be appreciated by those of skill in the art, the stuffer nucleic acid may be located in a variety of regions within the nucleic, and may be comprised of a continuous sequence (*e.g.,* a single stuffer nucleic acid in a single location) or multiple sequences (*e.g.,* more than one stuffer nucleic acid in more than one location (*e.g.,* 2 locations, 3 locations, *etc.*) within the nucleic acid. In some embodiments, the stuffer nucleic acid may be located downstream of the nucleic acid sequence encoding the variant PAH polypeptide. In embodiments, the stuffer nucleic acid may be located upstream of the nucleic acid sequence encoding the variant PAH polypeptide (*e.g.,* between the promoter and nucleic acid sequence encoding the variant PAH polypeptide). As will also be appreciated by those of skill in the art a variety of nucleic acids may be used as a stuffer nucleic acid. In some embodiments, the stuffer nucleic acid comprises all or a portion of a human alpha-1-antitrypsin (AAT) stuffer sequence or a C16 P1 chromosome 16 P1 clone (human C16) stuffer sequence. In some embodiments, the stuffer sequence comprises all or a portion of a gene. For example, the stuffer sequence comprises a portion of the human AAT sequence. One skilled in the art would recognize that different portions of a gene (*e.g.,* the human AAT sequence) can be used as a stuffer fragment. For example, the stuffer fragment may be from the 5' end of the gene, the 3' end of the gene, the middle of a gene, a non-coding portion of the gene (*e.g.,* an intron), a coding region of the gene (*e.g.* an exon), or a mixture of non-coding and coding portions of a gene. One skilled in the art would also recognize that all or a portion of stuffer sequence may be used as a stuffer sequence. In some embodiments, the stuffer sequence is modified to remove internal ATG codons. In some embodiments, the stuffer sequence comprises the nucleotide sequence of SEQ ID NO: 16.

In some embodiments, the isolated nucleic acid encoding a human PAH polypeptide is codon-optimized. In some embodiments, the isolated nucleic acid encoding a human PAH polypeptide is codon optimized for expression in a particular cell, such as a eukaryotic cell. Eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon of the native sequence with codons that are more frequently or most frequently used in the genes of that host cell while maintaining the native amino acid sequence. Various species exhibit particular bias for certain codons of a particular amino acid. Codon usage tables are readily available, for example, at the "Codon Usage Database", and these tables can be adapted in a number of ways (see, *e.g.,* Nakamura, Y. et al. (2000) Nucleic Acids Res. 28:292). Computer algorithms for codon optimizing a particular sequence for expression in a particular host cell are also available, such as Gene Forge (Aptagen; Jacobus, Pa.), DNA2.0, GeneArt (GA) or Genscript (GS) and a GS algorithm combined with reduction in CpG content. In some embodiments, nucleic acid encoding the PAH polypeptide is codon optimized using the GA algorithm. In some embodiments, the nucleic acid sequence is at least 80% identical to the nucleic acid sequence of SEQ ID NO: 14. In some embodiments, the nucleic acid comprises the nucleic acid sequence of SEQ ID NO:14.

### IV. Liver specific expression cassettes

In some aspects, the invention provides expression cassettes for expressing a transgene in a liver cell, wherein the expression cassette comprises a transgene operably linked to a promoter and enhancer, wherein the promoter comprises a mouse transthyretin (mTTR) promoter and the enhancer comprises one or two modified prothrombin enhancers (mPrT2), one or two modified alpha1-microbikunin enhancers (mA1MB2), a modified mouse albumin enhancer (mEalb), a hepatitis B virus enhancer II (HE11) or a CRM8 enhancer. In some embodiments, the mTTR promoter is a mTTR482 promoter. In some embodiments, the enhancer is 5' to the mTTR promoter. In some embodiments, the transgene encodes a variant PAH polypeptide as described herein.

In some embodiments, the invention provides expression cassettes for expressing a transgene in a liver cell, wherein the expression cassette comprises a transgene operably linked to a promoter and a 3' element, wherein the promoter comprises a mouse transthyretin (mTTR) promoter and the 3' element is an albumin 3' element (3'Alb) or an albumin 3' element linked to a human alpha 1 antitrypsin scaffold/matrix attachment region (SMAR) (3'AlbSMAR). In some embodiments, the mTTR promoter is a mTTR482 promoter. In some embodiments, the 3' element is located 3' to the transgene. In some embodiments, the transgene encodes a variant PAH polypeptide as described herein.

In some embodiments, the invention provides expression cassettes for expressing a transgene in a liver cell, wherein the expression cassette comprises a transgene operably linked to a promoter and enhancer and a 3' element, wherein the promoter comprises a mouse transthyretin (mTTR) promoter and the enhancer comprises one or two modified prothrombin enhancers (mPrT2), one or two modified alpha1-microbikunin enhancers (mA1MB2), a modified mouse albumin enhancer (mEalb), a hepatitis B virus enhancer II (HE11) or a CRM8 enhancer; and wherein the 3' element is an albumin 3' element (3'Alb) or an albumin 3' element linked to a human alpha 1 antitrypsin scaffold/matrix attachment region (SMAR) (3'AlbSMAR). In some embodiments, the mTTR promoter is a mTTR482 promoter. In some embodiments, the enhancer is 5' to the mTTR promoter. In some embodiments, the 3' element is located 3' to the transgene. In some embodiments, the transgene encodes a variant PAH polypeptide as described herein.

In some embodiments, the expression cassette further comprises an intron. In some embodiments the intron is an MVM intron, a F IX truncated intron 1, a β-globin SD/immunoglobin heavy chain SA, an adenovirus SD/immunoglobin SA, a SV40 late SD/SA (19S/16S), or a hybrid adenovirus SD/IgG SA. In some embodiments, the intron is a chicken β-actin (CBA)/rabbit β-globin hybrid intron.

In some embodiments, the expression cassette further comprises a polyadenylation signal. In some embodiments, the polyadenylation signal is a bovine growth hormone polyadenylation signal, an SV40 polyadenylation signal, or a HSV TK pA.

In some embodiments, the expression cassette is incorporated into a vector. In some embodiments, the expression cassette is incorporated into a viral vector. In some embodiments, the viral vector is an rAAV vector as described herein.

### V. Vectors and Viral Particles

In certain aspects, the nucleic acid encoding a variant PAH polypeptide is contained in a vector. In some embodiments, the present invention contemplates the use of a recombinant viral genome for introduction of nucleic acid sequences encoding the variant PAH polypeptide for packaging into a viral particle, *e.g.,* a viral particle described below. The recombinant viral genome may include any element to establish the expression of the variant PAH polypeptide, for example, a promoter, an ITR, a ribosome binding element, terminator, enhancer, selection marker, intron, polyA signal, and/or origin of replication. Exemplary viral genome elements and delivery methods for viral particles are described in greater detail below.

### Non-viral Delivery Systems

Conventional non-viral gene transfer methods may also be used to introduce nucleic acids into cells or target tissues. Non-viral vector delivery systems include DNA plasmids, naked nucleic acid, and nucleic acid complexed to a delivery system. For example, the vector may be complexed to a lipid (*e.g.,* a cationic or neutral lipid), a liposome, a polycation, a nanoparticle, or an agent that enhances the cellular uptake of nucleic acid. The vector may be complexed to an agent suitable for any of the delivery methods described herein. In some embodiments, the nucleic acid comprises one or more viral ITRs (*e.g.,* AAV ITRs).

### Viral Particles

In some embodiments, the vector comprising nucleic acid encoding the variant PAH polypeptide is a recombinant adeno-associated virus (rAAV) vector, a recombinant adenoviral vector, a recombinant lentiviral vector or a recombinant herpes simplex virus (HSV) vector.

### rAA Vparticles

In some embodiments, the vector is a recombinant AAV (rAAV) vector. In some embodiments, the nucleic acid encoding the variant PAH polypeptide is flanked by one or more AAV inverted terminal repeat (ITR) sequences. In some embodiments, the viral particle is a recombinant AAV particle comprising a nucleic acid comprising nucleic acid encoding the variant PAH polypeptide flanked by one or two ITRs. In some embodiments, the nucleic acid encoding the variant PAH polypeptide is flanked by two AAV ITRs.

In some embodiments, the nucleic acid encoding the variant PAH polypeptide of the present disclosure operatively linked components in the direction of transcription, control sequences including transcription initiation and termination sequences, thereby forming an expression cassette. The expression cassette is flanked on the 5' and 3' end by at least one functional AAV ITR sequence. By "functional AAV ITR sequences" it is meant that the ITR sequences function as intended for the rescue, replication and packaging of the AAV virion. *See* Davidson et al., PNAS, 2000, 97(7)3428-32; Passini et al., J. Virol., 2003, 77(12):7034-40; and Pechan et al., Gene Ther., 2009, 16:10-16, all of which are incorporated herein in their entirety by reference. For practicing some aspects of the invention, the recombinant vectors comprise at least all of the sequences of AAV essential for encapsidation and the physical structures for infection by the rAAV. AAV ITRs for use in the vectors of the invention need not have a wild-type nucleotide sequence (*e.g.,* as described in Kotin, Hum. Gene Ther., 1994, 5:793-801), and may be altered by the insertion, deletion or substitution of nucleotides or the AAV ITRs may be derived from any of several AAV serotypes. More than 40 serotypes of AAV are currently known, and new serotypes and variants of existing serotypes continue to be identified. *See* Gao et al., PNAS, 2002, 99(18): 11854-6; Gao et al., PNAS, 2003, 100(10):6081-6; and Bossis et al., J. Virol., 2003, 77(12):6799-810.

Use of any AAV serotype is considered within the scope of the present invention. In some embodiments, a rAAV vector is a vector derived from an AAV serotype, including without limitation, AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, a goat AAV, bovine AAV, or mouse AAV ITRs or the like. In some embodiments, the nucleic acid in the AAV comprises an ITR of AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, a goat AAV, bovine AAV, or mouse AAV or the like. In certain embodiments, the AAV ITRs are AAV2 ITRs.

In some embodiments, a vector may include a stuffer nucleic acid. In some embodiments, the stuffer nucleic acid may encode a green fluorescent protein. In some embodiments, the stuffer nucleic acid may be located 3' to the nucleic acid encoding a variant PAH polypeptide of the present disclosure.

In some aspects, the invention provides viral particles comprising a recombinant self-complementing genome. In some embodiments, the vector is a self-complementary vector. AAV viral particles with self-complementing genomes and methods of use of self-complementing AAV genomes are described in US Patent Nos. 6,596,535; 7,125,717; 7,765,583; 7,785,888; 7,790,154; 7,846,729; 8,093,054; and 8,361,457; and Wang Z., et al., (2003) Gene Ther 10:2105-2111, each of which are incorporated herein by reference in its entirety. A rAAV comprising a self-complementing genome will quickly form a double stranded DNA molecule by virtue of its partially complementing sequences (*e.g.,* complementing coding and non-coding strands of a transgene). In some embodiments, the invention provides an AAV viral particle comprising an AAV genome, wherein the rAAV genome comprises a first heterologous polynucleotide sequence (*e.g.,* the coding strand of the variant PAH polypeptide of the invention) and a second heterologous polynucleotide sequence (*e.g.,* the noncoding or antisense strand of the variant PAH polypeptide of the present disclosure) wherein the first heterologous polynucleotide sequence can form intrastrand base pairs with the second polynucleotide sequence along most or all of its length.

In some embodiments, the first heterologous polynucleotide sequence and a second heterologous polynucleotide sequence are linked by a sequence that facilitates intrastrand basepairing; *e.g.,* a hairpin DNA structure. Hairpin structures are known in the art, for example in siRNA molecules. In some embodiments, the first heterologous polynucleotide sequence and a second heterologous polynucleotide sequence are linked by a mutated ITR (*e.g.,* the right ITR). The mutated ITR comprises a deletion of the D region comprising the terminal resolution sequence. As a result, on replicating an AAV viral genome, the rep proteins will not cleave the viral genome at the mutated ITR and as such, a recombinant viral genome comprising the following in 5' to 3' order will be packaged in a viral capsid: an AAV ITR, the first heterologous polynucleotide sequence including regulatory sequences, the mutated AAV ITR, the second heterologous polynucleotide in reverse orientation to the first heterologous polynucleotide and a third AAV ITR.

In some embodiments, the first heterologous nucleic acid sequence and a second heterologous nucleic acid sequence are linked by a mutated ITR *(e.g.,* the right ITR). In some embodiments, the ITR comprises the polynucleotide sequence 5'-CACTCCCTCTCTGCGCGCT CGCTCGCTCACTGAGGCCGGGCGACCAAAGGTCGCCCACGCCCGGGCTTTGCCCGG GCG - 3' (SEQ ID NO: 17). The mutated ITR comprises a deletion of the D region comprising the terminal resolution sequence. As a result, on replicating an AAV viral genome, the rep proteins will not cleave the viral genome at the mutated ITR and as such, a recombinant viral genome comprising the following in 5' to 3' order will be packaged in a viral capsid: an AAV ITR, the first heterologous polynucleotide sequence including regulatory sequences, the mutated AAV ITR, the second heterologous polynucleotide in reverse orientation to the first heterologous polynucleotide and a third AAV ITR.

In some embodiments, the vector is encapsidated in a viral particle. In some embodiments, the viral particle is a recombinant AAV viral particle comprising a recombinant AAV vector. Different AAV serotypes are used to optimize transduction of particular target cells or to target specific cell types within a particular target tissue (*e.g.,* an ocular tissue). A rAAV particle can comprise viral proteins and viral nucleic acids of the same serotype or a mixed serotype. For example, in some embodiments a rAAV particle can comprise AAV2 capsid proteins of the invention and at least one AAV2 ITR or it can comprise AAV2 capsid proteins and at least one AAV1 ITR. Any combination of AAV serotypes for production of a rAAV particle is provided herein as if each combination had been expressly stated herein. In some embodiments, the invention provides rAAV particles comprising an AAV2 capsid of the invention. In some embodiments, the invention provides rAAV particles comprising an AAVrh8R capsid of the invention.

In some embodiments, the rAAV particles comprise an AAV1 capsid, an AAV2 capsid, an AAV3 capsid, an AAV4 capsid, an AAV5 capsid, an AAV6 capsid (*e.g.,* a wild-type AAV6 capsid, or a variant AAV6 capsid such as ShH10, as described in U.S. PG Pub. 2012/0164106), an AAV7 capsid, an AAV8 capsid, an AAVrh8 capsid, an AAVrh8R capsid, an AAV9 capsid (*e.g.,* a wild-type AAV9 capsid, or a modified AAV9 capsid as described in U.S. PG Pub. 2013/0323226), an AAV10 capsid, an AAVrh10 capsid, an AAV11 capsid, an AAV12 capsid, a tyrosine capsid mutant, a heparin binding capsid mutant, an AAV2R471A capsid, an AAVAAV2/2-7m8 capsid, an AAV DJ capsid (*e.g.,* an AAV-DJ/8 capsid, an AAV-DJ/9 capsid, or any other of the capsids described in U.S. PG Pub. 2012/0066783), an AAV2 N587A capsid, an AAV2 E548A capsid, an AAV2 N708A capsid, an AAV V708K capsid, a goat AAV capsid, an AAV1/AAV2 chimeric capsid, a bovine AAV capsid, a mouse AAV capsid, a rAAV2/HBoV1 capsid, or an AAV capsid described in U.S. Pat. No. 8,283,151 or International Publication No. WO/2003/042397. In some embodiments, a mutant capsid protein maintains the ability to form an AAV capsid. In some embodiments, the rAAV particle comprises AAV5 tyrosine mutant capsid (Zhong L. et al., (2008) Proc Natl Acad Sci USA 105(22):7827-7832. In further embodiments, the rAAV particle comprises capsid proteins of an AAV serotype from Clades A-F (Gao, et al., J. Virol. 2004, 78(12):6381). In some embodiments, the rAAV particle comprises an AAV1 capsid protein or mutant thereof. In other embodiments, the rAAV particle comprises an AAV2 capsid protein or mutant thereof. In some embodiments, the AAV serotype is AAV1, AAV2, AAV5, AAV6, AAV7, AAV8, AAVrh8, AAVrh8R, AAV9, AAV10, or AAVrh10. In some embodiments, the rAAV particle comprises an AAV serotype 1 (AAV1) capsid. In some embodiments, the rAAV particle comprises an AAV serotype 2 (AAV2) capsid. In some embodiments, the recombinant AAV viral particle comprises an AAV1, AAV2, AAV8, AAVrh8R, AAV9, and/or AAVrh10 capsid. In some embodiments, the AAV1, AAV2, AAV8, AAVrh8R, AAV9, and/or AAVrh10 capsid comprises a tyrosine mutation or a heparan binding mutation, *e.g.,* as described below. In some embodiments, the capsid is a liver targeting capsid; for example but not limited to, a LK03 capsid, a HSC15 capsid, or a 17 capsid. In some embodiments, the capsid is an engineered AAV capsid (*e.g.,* a shuffled capsid). Examples of engineered AAV capsids include, but are not limited to DJ (Grimm D et al., J Virol. 2008, 82:5887-911), LK03 (Lisowski L et al., Nature, 2014, 506:382-6) and HSC15 and HSC17 (Smith LJ et al., Mol Ther, 2014 Sep;22(9):1625-34).

The capsid of AAV (*e.g.,* AAV2, AAV8 *etc.*) is known to include three capsid proteins: VP1, VP2, and VP3. These proteins contain significant amounts of overlapping amino acid sequence and unique N-terminal sequences. An AAV2 capsid includes 60 subunits arranged by icosahedral symmetry (Xie, Q., et al. (2002) Proc. Natl. Acad. Sci. USA 99(16):10405-10). VP1, VP2, and VP3 have been found to be present in a 1:1:10 ratio.

In some embodiments, the rAAV particle comprises a) a rAAV capsid comprising rAAV capsid proteins comprising one or more amino acid substitutions at one or more positions that interacts with a heparan sulfate proteoglycan, and b) a rAAV vector comprising the heterologous nucleic acid and at least one AAV inverted terminal repeat.

In some embodiments, the rAAV particles comprise one or more amino acid substitutions of capsid proteins that reduce or ablate binding of the rAAV particle to the heparan sulfate proteoglycan, and/or wherein the one or more amino acid substitutions is at position 484, 487, 532, 585 or 588, numbering based on VP1 numbering of AAV2. As used herein, "numbering based on VP1 of AAV2" refers to the amino acid of the recited capsid protein corresponding to the recited amino acid of VP1 of AAV2. For example, if one or more amino acid substitutions are at position 347, 350, 390, 395, 448, 451, 484, 487, 527, 532, 585 and/or 588, numbering based on VP1 of AAV2, then the one or more amino acid substitutions are at the amino acid(s) of the recited capsid protein corresponding to amino acids 347, 350, 390, 395, 448, 451, 484, 487, 527, 532, 585 and/or 588 of VP1 of AAV2. In some embodiments, the one or more amino acid substitutions is at position 484, 487, 532, 585 or 588 of VP1 of AAV2. In some embodiments, the one or more amino acid substitutions is at position 484, 487, 532, 585 or 588 of VP1 of AAV3, numbering based on VP1 of AAV2. In some embodiments, the one or more amino acid substitutions is at position 485, 488, 528, 533, 586 or 589, numbering based on VP1 numbering of AAVrh8R. In some embodiments, one or more amino acids at position(s) corresponding to amino acids 585 and/or 588 (numbering based on VP1 of AAV2) are replaced by arginine residues (*e.g.,* S586 and/or T589 for AAV1 or AAV6; S586 and/or A589 for AAV9; A586 and/or T589 for AAVrh8R; Q588 and/or T591 for AAV8; and Q588 and/or A591 for AAVrh10). In other embodiments, one or more amino acids (*e.g.,* arginine or lysine) at position(s) corresponding to amino acids 484, 487, 527 and/or 532 (numbering based on VP1 of AAV2) are replaced by non-positively charged amino acid(s) such as alanine (*e.g.,* R485, R488, K528, and/or K533 for AAV1 or AAV6; R485, R488, K528, and/or R533 for AAV9 or AAVrh8R; and R487, R490, K530, and/or R535 for AAV8 or AAVrh10).

### Production of AAV particles

Numerous methods are known in the art for production of rAAV vectors, including transfection, stable cell line production, and infectious hybrid virus production systems which include adenovirus-AAV hybrids, herpesvirus-AAV hybrids (Conway, JE et al., (1997) J. Virology 71(11):8780-8789) and baculovirus-AAV hybrids (Urabe, M. et al., (2002) Human Gene Therapy 13(16): 1935-1943; Kotin, R. (2011) Hum Mol Genet. 20(R1): R2-R6). rAAV production cultures for the production of rAAV virus particles all require; 1) suitable host cells, 2) suitable helper virus function, 3) AAV rep and cap genes and gene products; 4) a nucleic acid (such as a therapeutic nucleic acid) flanked by at least one AAV ITR sequences (*e.g.,* an AAV genome encoding a variant PAH polypeptide); and 5) suitable media and media components to support rAAV production. In some embodiments, the suitable host cell is a primate host cell. In some embodiments, the suitable host cell is a human-derived cell lines such as HeLa, A549, 293, or Perc.6 cells. In some embodiments, the suitable helper virus function is provided by wild-type or mutant adenovirus (such as temperature sensitive adenovirus), herpes virus (HSV), baculovirus, or a plasmid construct providing helper functions. In some embodiments, the AAV rep and cap gene products may be from any AAV serotype. In general, but not obligatory, the AAV rep gene product is of the same serotype as the ITRs of the rAAV vector genome as long as the rep gene products may function to replicated and package the rAAV genome. Suitable media known in the art may be used for the production of rAAV vectors. These media include, without limitation, media produced by Hyclone Laboratories and JRH including Modified Eagle Medium (MEM), Dulbecco's Modified Eagle Medium (DMEM), custom formulations such as those described in U.S. Patent No. 6,566,118, and Sf-900 II SFM media as described in U.S. Patent No. 6,723,551, each of which is incorporated herein by reference in its entirety, particularly with respect to custom media formulations for use in production of recombinant AAV vectors. In some embodiments, the AAV helper functions are provided by adenovirus or HSV. In some embodiments, the AAV helper functions are provided by baculovirus and the host cell is an insect cell (*e.g., Spodoptera frugiperda* (Sf9) cells).

One method for producing rAAV particles is the triple transfection method. Briefly, a plasmid containing a rep gene and a capsid gene, along with a helper adenoviral plasmid, may be transfected (*e.g.,* using the calcium phosphate method) into a cell line (*e.g.,* HEK-293 cells), and virus may be collected and optionally purified. As such, in some embodiments, the rAAV particle was produced by triple transfection of a nucleic acid encoding the rAAV vector, a nucleic acid encoding AAV rep and cap, and a nucleic acid encoding AAV helper virus functions into a host cell, wherein the transfection of the nucleic acids to the host cells generates a host cell capable of producing rAAV particles.

In some embodiments, rAAV particles may be produced by a producer cell line method (*see* Martin et al., (2013) Human Gene Therapy Methods 24:253-269; U.S. PG Pub. No. US2004/0224411; and Liu, X.L. et al. (1999) Gene Ther. 6:293-299). Briefly, a cell line *(e.g.,* a HeLa, 293, A549, or Perc.6 cell line) may be stably transfected with a plasmid containing a rep gene, a capsid gene, and a vector genome comprising a promoter-heterologous nucleic acid sequence (*e.g.,* a variant PAH polypeptide). Cell lines may be screened to select a lead clone for rAAV production, which may then be expanded to a production bioreactor and infected with a helper virus (*e.g.,* an adenovirus or HSV) to initiate rAAV production. Virus may subsequently be harvested, adenovirus may be inactivated (e.g., by heat) and/or removed, and the rAAV particles may be purified. As such, in some embodiments, the rAAV particle was produced by a producer cell line comprising one or more of nucleic acid encoding the rAAV vector, a nucleic acid encoding AAV rep and cap, and a nucleic acid encoding AAV helper virus functions. As described herein, the producer cell line method may be advantageous for the production of rAAV particles with an oversized genome, as compared to the triple transfection method.

In some embodiments, the nucleic acid encoding AAV rep and cap genes and/or the rAAV genome are stably maintained in the producer cell line. In some embodiments, nucleic acid encoding AAV rep and cap genes and/or the rAAV genome is introduced on one or more plasmids into a cell line to generate a producer cell line. In some embodiments, the AAV rep, AAV cap, and rAAV genome are introduced into a cell on the same plasmid. In other embodiments, the AAV rep, AAV cap, and rAAV genome are introduced into a cell on different plasmids. In some embodiments, a cell line stably transfected with a plasmid maintains the plasmid for multiple passages of the cell line (*e.g.,* 5, 10, 20, 30, 40, 50 or more than 50 passages of the cell). For example, the plasmid(s) may replicate as the cell replicates, or the plasmid(s) may integrate into the cell genome. A variety of sequences that enable a plasmid to replicate autonomously in a cell (*e.g.,* a human cell) have been identified (*see, e.g.,* Krysan, P.J. et al. (1989) Mol. Cell Biol. 9:1026-1033). In some embodiments, the plasmid(s) may contain a selectable marker (*e.g.,* an antibiotic resistance marker) that allows for selection of cells maintaining the plasmid. Selectable markers commonly used in mammalian cells include without limitation blasticidin, G418, hygromycin B, zeocin, puromycin, and derivatives thereof. Methods for introducing nucleic acids into a cell are known in the art and include without limitation viral transduction, cationic transfection (*e.g.,* using a cationic polymer such as DEAE-dextran or a cationic lipid such as lipofectamine), calcium phosphate transfection, microinjection, particle bombardment, electroporation, and nanoparticle transfection (for more details, *see e.g.,* Kim, T.K. and Eberwine, J.H. (2010) Anal. Bioanal. Chem. 397:3173-3178).

In some embodiments, the nucleic acid encoding AAV rep and cap genes and/or the rAAV genome are stably integrated into the genome of the producer cell line. In some embodiments, nucleic acid encoding AAV rep and cap genes and/or the rAAV genome is introduced on one or more plasmids into a cell line to generate a producer cell line. In some embodiments, the AAV rep, AAV cap, and rAAV genome are introduced into a cell on the same plasmid. In other embodiments, the AAV rep, AAV cap, and rAAV genome are introduced into a cell on different plasmids. In some embodiments, the plasmid(s) may contain a selectable marker (*e.g.,* an antibiotic resistance marker) that allows for selection of cells maintaining the plasmid. Methods for stable integration of nucleic acids into a variety of host cell lines are known in the art. For example, repeated selection (*e.g.,* through use of a selectable marker) may be used to select for cells that have integrated a nucleic acid containing a selectable marker (and AAV cap and rep genes and/or a rAAV genome). In other embodiments, nucleic acids may be integrated in a site-specific manner into a cell line to generate a producer cell line. Several site-specific recombination systems are known in the art, such as FLP/FRT (*see, e.g.,* O'Gorman, S. et al. (1991) Science 251:1351-1355), Cre/loxP (*see, e.g.,* Sauer, B. and Henderson, N. (1988) Proc. Natl. Acad. Sci. 85:5166-5170), and phi C31-att (*see, e.g.,* Groth, A.C. et al. (2000) Proc. Natl. Acad. Sci. 97:5995-6000).

In some embodiments, the producer cell line is derived from a primate cell line (*e.g.,* a non-human primate cell line, such as a Vero or FRhL-2 cell line). In some embodiments, the cell line is derived from a human cell line. In some embodiments, the producer cell line is derived from HeLa, 293, A549, or PERC.6^{®} (Crucell) cells. For example, prior to introduction and/or stable maintenance/integration of nucleic acid encoding AAV rep and cap genes and/or the oversized rAAV genome into a cell line to generate a producer cell line, the cell line is a HeLa, 293, A549, or PERC.6^{®} (Crucell) cell line, or a derivative thereof.

In some embodiments, the producer cell line is adapted for growth in suspension. As is known in the art, anchorage-dependent cells are typically not able to grow in suspension without a substrate, such as microcarrier beads. Adapting a cell line to grow in suspension may include, for example, growing the cell line in a spinner culture with a stirring paddle, using a culture medium that lacks calcium and magnesium ions to prevent clumping (and optionally an antifoaming agent), using a culture vessel coated with a siliconizing compound, and selecting cells in the culture (rather than in large clumps or on the sides of the vessel) at each passage. For further description, see, *e.g.,* ATCC frequently asked questions document (available at www.atcc.org/Global/FAQs/9/1/Adapting%20a%20monolayer%20cell%20line%20to%20suspen sion-40.aspx) and references cited therein.

In some aspects, a method is provided for producing any rAAV particle as disclosed herein comprising (a) culturing a host cell under a condition that rAAV particles are produced, wherein the host cell comprises (i) one or more AAV package genes, wherein each said AAV packaging gene encodes an AAV replication and/or encapsidation protein; (ii) a rAAV pro-vector comprising a nucleic acid encoding a heterologous nucleic acid as described herein flanked by at least one AAV ITR, and (iii) an AAV helper function; and (b) recovering the rAAV particles produced by the host cell. In some embodiments, said at least one AAV ITR is selected from the group consisting of AAV1, AAV2, AAV3, AAV4, AAV5, AAV6, AAV7, AAV8, AAVrh8, AAVrh8R, AAV9, AAV10, AAVrh10, AAV11, AAV12, a goat AAV, bovine AAV, or mouse AAV serotype ITRs or the like. For example, in some embodiments, the AAV serotype is AAV1, AAV2, AAV5, AAV6, AAV7, AAV8, AAVrh8, AAVrh8R, AAV9, AAV10, or AAVrh10. In certain embodiments, the nucleic acid in the AAV comprises an AAV2 ITR. In some embodiments, said encapsidation protein is selected from the group consisting of AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV V708K, goat AAV, AAV1/AAV2 chimeric, bovine AAV, or mouse AAV capsid rAAV2/HBoV1 serotype capsid proteins or mutants thereof. In some embodiments, the encapsidation protein is an AAV8 capsid protein. In some embodiments, the rAAV particles comprise an AAV8 capsid and a recombinant genome comprising AAV2 ITRs, and nucleic acid encoding a therapeutic transgene/nucleic acid (*e.g.,* nucleic acid encoding a variant PAH polypeptide).

Suitable rAAV production culture media of the present invention may be supplemented with serum or serum-derived recombinant proteins at a level of 0.5%-20% (v/v or w/v). Alternatively, as is known in the art, rAAV vectors may be produced in serum-free conditions which may also be referred to as media with no animal-derived products. One of ordinary skill in the art may appreciate that commercial or custom media designed to support production of rAAV vectors may also be supplemented with one or more cell culture components know in the art, including without limitation glucose, vitamins, amino acids, and or growth factors, in order to increase the titer of rAAV in production cultures.

rAAV production cultures can be grown under a variety of conditions (over a wide temperature range, for varying lengths of time, and the like) suitable to the particular host cell being utilized. As is known in the art, rAAV production cultures include attachment- dependent cultures which can be cultured in suitable attachment-dependent vessels such as, for example, roller bottles, hollow fiber filters, microcarriers, and packed-bed or fluidized-bed bioreactors. rAAV vector production cultures may also include suspension-adapted host cells such as HeLa, 293, and SF-9 cells which can be cultured in a variety of ways including, for example, spinner flasks, stirred tank bioreactors, and disposable systems such as the Wave bag system.

rAAV vector particles of the invention may be harvested from rAAV production cultures by lysis of the host cells of the production culture or by harvest of the spent media from the production culture, provided the cells are cultured under conditions known in the art to cause release of rAAV particles into the media from intact cells, as described more fully in U.S. Patent No. 6,566,118). Suitable methods of lysing cells are also known in the art and include for example multiple freeze/thaw cycles, sonication, microfluidization, and treatment with chemicals, such as detergents and/or proteases.

In a further embodiment, the rAAV particles are purified. The term "purified" as used herein includes a preparation of rAAV particles devoid of at least some of the other components that may also be present where the rAAV particles naturally occur or are initially prepared from. Thus, for example, isolated rAAV particles may be prepared using a purification technique to enrich it from a source mixture, such as a culture lysate or production culture supernatant. Enrichment can be measured in a variety of ways, such as, for example, by the proportion of DNase-resistant particles (DRPs) or genome copies (gc) present in a solution, or by infectivity, or it can be measured in relation to a second, potentially interfering substance present in the source mixture, such as contaminants, including production culture contaminants or in-process contaminants, including helper virus, media components, and the like.

In some embodiments, the rAAV production culture harvest is clarified to remove host cell debris. In some embodiments, the production culture harvest is clarified by filtration through a series of depth filters including, for example, a grade DOHC Millipore Millistak+ HC Pod Filter, a grade A1HC Millipore Millistak+ HC Pod Filter, and a 0.2 µm Filter Opticap XL1O Millipore Express SHC Hydrophilic Membrane filter. Clarification can also be achieved by a variety of other standard techniques known in the art, such as, centrifugation or filtration through any cellulose acetate filter of 0.2 µm or greater pore size known in the art.

In some embodiments, the rAAV production culture harvest is further treated with Benzonase^{®} to digest any high molecular weight DNA present in the production culture. In some embodiments, the Benzonase^{®} digestion is performed under standard conditions known in the art including, for example, a final concentration of 1-2.5 units/ml of Benzonase^{®} at a temperature ranging from ambient to 37°C for a period of 30 minutes to several hours.

rAAV particles may be isolated or purified using one or more of the following purification steps: equilibrium centrifugation; flow-through anionic exchange filtration; tangential flow filtration (TFF) for concentrating the rAAV particles; rAAV capture by apatite chromatography; heat inactivation of helper virus; rAAV capture by hydrophobic interaction chromatography; buffer exchange by size exclusion chromatography (SEC); nanofiltration; and rAAV capture by anionic exchange chromatography, cationic exchange chromatography, or affinity chromatography. These steps may be used alone, in various combinations, or in different orders. In some embodiments, the method comprises all the steps in the order as described below. Methods to purify rAAV particles are found, for example, in Xiao et al., (1998) Journal of Virology 72:2224-2232; US Patent Numbers 6,989,264 and 8,137,948; and WO 2010/148143.

### VI. Methods of Treatment

Certain aspects of the present disclosure relate to methods of treating phenylketonuria and/or reducing levels of phenylalanine in an individual in need thereof. Phenylketonuria (PKU) is caused by deficiency of phenylalanine hydroxylase (PAH) resulting in elevated blood Phe levels that are toxic to brain and subsequently lead to severe mental disorders without treatment. Current therapy by diet restriction has been efficacious but non-compliance in teens and adults is a major issue.

Efforts to treat PKU have been hampered by low activity of human PAH from gene therapy vectors. The present methods are based in part on the discovery of variant PAH polypeptides with improved protein stability and enzyme activity compared to the endogenous human PAH polypeptide. Treatment of PKU via gene therapy using an rAAV vector encoding the variant PAH polypeptides described herein lead to better reduction of blood and brain Phe levels compared with the vector encoding endogenous human PAH. Furthermore, while both hPAH and hPAH-V1 improved Tyr and Trp transport into brain, only the variant PAH-treated animals had normalized brain neurotransmitter levels including dopamine and serotonin indicating different sensitivities on Phe affected function.

In some embodiments, the invention provides methods to treat PKU comprising administering to an individual in need thereof, a therapeutically effective amount of a variant PAH polypeptide as described herein. In some embodiments, the variant PAH polypeptide comprises at least three amino acid substitutions, wherein the amino acid substitutions are at sites selected from M180, K199, S250, and G256 of a wild-type human PAH polypeptide. In some embodiments, the variant PAH polypeptide comprises four amino acid substitutions at sites M180, K199, S250, and G256 of a wild-type human PAH polypeptide. In some embodiments, the variant PAH polypeptide comprises at least three amino acid substitutions selected from M180T, K199P, S250P, and G256A. In some embodiments, the variant PAH polypeptide comprises amino acid substitutions M180T, K199P, S250P, and G256A. In some embodiments, the variant PAH polypeptide comprises amino acid substitution comprises K199P, S250P, and G256A; M180T, S250P, and G256A; M180T, K199P, and G256A; or M180T, K199P, and S250P. In some embodiments, the variant PAH polypeptide comprises amino acid substitutions M180T, K199P, S250P, and G256A. In some embodiments, the variant PAH polypeptide further comprises amino acid substitutions H264P, G272A, G272P, P275L, P279Q, G272P and P275L, or T323R and F327T amino acid substitutions. In some embodiments, the variant PAH polypeptide is any one of the variant PAH polypeptides presented in Tables 1-3. In some embodiments, the variant PAH polypeptide comprises amino acid substitutions M180T, K199P, S250P, and G256A and comprises further amino acid substitutions while maintaining at least about the phenylalanine hydroxylase activity of wild-type PAH. In some embodiments, the location of the amino acid substitutions is based on wild-type human PAH polypeptide; for example, the human PAH polypeptide comprising the amino acid sequence of SEQ ID NO:1. In some embodiments, the variant PAH polypeptide is a fusion polypeptide. In some embodiments, the variant PAH polypeptide is a fusion polypeptide fused to a tissue-targeting peptide. In some embodiments, the variant PAH polypeptide is a fusion polypeptide fused to a liver-targeting polypeptide. Examples of liver targeting polypeptides include, but are not limited to, fragments of human hepatocyte growth factor (Eavri and Lorberboum-Galkski, J Biol Chem 2007, 282:23402-23409) or glycoproteins binding to hepatocyte asialoglycoprotein receptor (Huang, et al., Bioconjugate Chemistry 2017, 28:283-295).

In some embodiments, the invention provides methods of treating PKU by administering an effective amount of a variant PAH polypeptide of the present disclosure. The variant PAH polypeptide may be administered to a particular tissue of interest, or it may be administered systemically. In some embodiments, an effective amount of variant PAH polypeptide may be administered parenterally. Parenteral routes of administration may include without limitation intravenous, intraperitoneal, intraosseous, intra-arterial, intracerebral, intramuscular, intrathecal, subcutaneous, intracerebroventricular, intrahepatic, and so forth. In some embodiments, an effective amount of the variant PAH polypeptide may be administered through one route of administration. In some embodiments, an effective amount of the variant PAH polypeptide may be administered through a combination of more than one route of administration. In some embodiments, an effective amount of the variant PAH polypeptide is administered to one location. In other embodiments, an effective amount of the variant PAH polypeptide may be administered to more than one location.

In some embodiments, the invention provides methods to treat PKU comprising administering to an individual in need thereof, a therapeutically effective amount of nucleic acid (e.g., DNA or mRNA) encoding a variant PAH polypeptide as described herein. In some embodiments, the nucleic acid encodes a variant PAH polypeptide comprises at least three amino acid substitutions, wherein the amino acid substitutions are at sites selected from M180, K199, S250, and G256 of a wild-type human PAH polypeptide. In some embodiments, the nucleic acid encodes a variant PAH polypeptide comprising four amino acid substitutions at sites M180, K199, S250, and G256 of a wild-type human PAH polypeptide. In some embodiments, the nucleic acid encodes a variant PAH polypeptide comprising at least three amino acid substitutions selected from M180T, K199P, S250P, and G256A. In some embodiments, the nucleic acid encodes a variant PAH polypeptide comprising amino acid substitutions M180T, K199P, S250P, and G256A. In some embodiments, the nucleic acid encodes a variant PAH polypeptide comprising amino acid substitution comprises K199P, S250P, and G256A; M180T, S250P, and G256A; M180T, K199P, and G256A; or M180T, K199P, and S250P. In some embodiments, the nucleic acid encodes a variant PAH polypeptide comprising amino acid substitutions M180T, K199P, S250P, and G256A. In some embodiments, the nucleic acid encodes a variant PAH polypeptide further comprising amino acid substitutions H264P, G272A, G272P, P275L, P279Q, G272P and P275L, or T323R and F327T amino acid substitutions. In some embodiments, the nucleic acid encodes a variant PAH polypeptide that is any one of the variant PAH polypeptides presented in Tables 1-3. In some embodiments, the nucleic acid encodes a variant PAH polypeptide comprising amino acid substitutions M180T, K199P, S250P, and G256A and comprises further amino acid substitutions while maintaining at least about the phenylalanine hydroxylase activity of wild-type PAH. In some embodiments, the location of the amino acid substitutions encoded by the nucleic acid is based on wild-type human PAH polypeptide; for example, the human PAH polypeptide comprising the amino acid sequence of SEQ ID NO: 1.

In some embodiments, the nucleic acid encodes a variant human PAH polypeptide. In some embodiments, the nucleic acid encodes a variant PAH polypeptide comprising the amino acid sequence of SEQ ID NO:3. In some embodiments, the nucleic acid encodes a variant PAH polypeptide comprising an amino acid sequences that is at least about any of 80%, 85%, 90%, 95% or 99% identical to the amino acid sequence of SEQ ID NO:3. In some embodiments, the nucleic acid encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH. In some embodiments, the nucleic acid encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH of SEQ ID NO: 1. In some embodiments, the nucleic acid encoding the variant PAH polypeptide is DNA. In some embodiments, the nucleic acid encoding the variant PAH polypeptide is RNA (e.g., mRNA).

In some embodiments, the nucleic acid encodes a variant PAH polypeptide that is a truncated PAH polypeptide that maintains phenylalanine hydroxylase activity. In some embodiments, the nucleic acid encodes a truncated PAH polypeptide comprising an N-terminal truncation. In some embodiments, the nucleic acid encodes a N-terminal truncation that is a truncation of part or all of the N-terminal regulatory domain. In some embodiments, the nucleic acid encodes a N-terminal truncation comprising a deletion of amino acid residue 1 to about amino acid residue 102 of the wild-type human PAH polypeptide (e.g., the PAH polypeptide of SEQ ID NO: 1). In some embodiments, the nucleic acid encodes a truncated PAH polypeptide comprising a C-terminal truncation. In some embodiments, the nucleic acid encodes a C-terminal truncation that is a truncation of part or all of the tetramerization domain. In some embodiments, the nucleic acid encodes a C-terminal truncation comprising a deletion of amino acid residues from about 429 to 452 of the wild-type human PAH polypeptide (e.g., the PAH polypeptide of SEQ ID NO: 1). In some embodiments, the nucleic acid encodes a variant PAH polypeptide comprising an N-terminal truncation and a C-terminal truncation. In some embodiments, the truncated PAH polypeptide comprises a truncation of part or all of the N-terminal regulatory sequence and part of all of the tetramerization domain. In some embodiments, the truncated PAH polypeptide comprises a deletion of amino acid residue 1 to about amino acid residue 102 and from about 429 to about 452 of the wild-type human PAH polypeptide (e.g., the PAH polypeptide of SEQ ID NO: 1). In some embodiments, the nucleic acid encodes a variant PAH polypeptide comprising an amino acid sequence corresponding to amino acid residues from about 102 to about 428 of the wild-type PAH polypeptide (e.g., the PAH polypeptide of SEQ ID NO: 1). In some embodiments, the nucleic acid encodes a variant PAH polypeptide comprises an amino acid sequence corresponding to amino acid residues from 102 to 428 of the wild-type PAH polypeptide (e.g., the PAH polypeptide of SEQ ID NO: 1). In some embodiments, the nucleic acid encodes a truncated PAH polypeptide further comprising four amino acid substitutions at sites M180, K199, S250, and G256 of a wild-type human PAH polypeptide (e.g., the PAH polypeptide of SEQ ID NO: 1). In some embodiments, the nucleic acid encodes a truncated PAH polypeptide comprising four amino acid substitutions selected from M180T, K199P, S250P, and G256A. In some embodiments, the nucleic acid encoding a truncated variant PAH polypeptide further comprises any one of the combinations of amino acid substitutions presented in Tables 1-3. In some embodiments, the nucleic acid encodes a truncated PAH polypeptide has at least about 25%, 50%, 75%, 100%, or greater than 100% the phenylalanine hydroxylase activity of wild-type PAH *(e.g.,* the PAH polypeptide of SEQ ID NO: 1). In some embodiments, the nucleic acid encoding a variant PAH polypeptide encodes a fusion polypeptide fused to a tissue-targeting peptide. In some embodiments, the variant PAH polypeptide is a fusion polypeptide fused to a liver-targeting peptide. In some embodiments, the nucleic acid encoding the variant PAH polypeptide is DNA. In some embodiments, the nucleic acid encoding the variant PAH polypeptide is RNA (e.g., mRNA).

In some embodiments, the invention provides methods of treating PKU by administering an effective amount of nucleic acid encoding a variant PAH polypeptide of the present disclosure. The nucleic acid encoding a variant PAH polypeptide may be administered to a particular tissue of interest, or it may be administered systemically. In some embodiments, an effective amount of nucleic acid encoding a variant PAH polypeptide may be administered parenterally. Parenteral routes of administration may include without limitation intravenous, intraperitoneal, intraosseous, intra-arterial, intracerebral, intramuscular, intrathecal, subcutaneous, intracerebroventricular, intrahepatic, and so forth. In some embodiments, expression of a variant PAH from tissues beyond liver may require the presence of cofactor BH4 *(e.g.,* delivered systemically or co-expressed from nucleic acid) Ding et al., Mol Ther 2008, 16:673-681. In some embodiments, an effective amount of nucleic acid encoding a variant PAH polypeptide may be administered through one route of administration. In some embodiments, an effective amount of nucleic acid encoding a variant PAH polypeptide may be administered through a combination of more than one route of administration. In some embodiments, an effective amount of nucleic acid encoding a variant PAH polypeptide is administered to one location. In other embodiments, an effective amount of the variant PAH polypeptide may be administered to more than one location. In some embodiments, the nucleic acid encoding the variant PAH polypeptide is DNA. In some embodiments, the nucleic acid encoding the variant PAH polypeptide is RNA (e.g., mRNA).

In some aspects the invention, nucleic acid encoding the variant PAH polypeptide is delivered to the individual by way of a viral vector. Viral vectors for gene therapy are known in the art. In some aspects, the invention provides methods of treating PKU by administering an effective amount of a lentiviral particle encoding a variant PAH polypeptide of the present disclosure. In some aspects, the invention provides methods of treating PKU by administering an effective amount of a rAAV particle encoding a variant PAH polypeptide of the present disclosure. rAAV may be administered to a particular tissue of interest, or it may be administered systemically. In some embodiments, an effective amount of rAAV may be administered parenterally. Parenteral routes of administration may include without limitation intravenous, intraperitoneal, intraosseous, intra-arterial, intracerebral, intramuscular, intrathecal, subcutaneous, intracerebroventricular, intrahepatic, and so forth. In some embodiments, an effective amount of rAAV may be administered through one route of administration. In some embodiments, an effective amount of rAAV may be administered through a combination of more than one route of administration. In some embodiments, an effective amount of rAAV is administered to one location. In other embodiments, an effective amount of rAAV may be administered to more than one location.

An effective amount of rAAV (in some embodiments in the form of particles) is administered, depending on the objectives of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells of the desired tissue type, in some embodiments at least about 20% of the cells of the desired tissue type, in some embodiments at least about 50%, in some embodiments at least about 80%, in some embodiments at least about 95%, in some embodiments at least about 99% of the cells of the desired tissue type. The rAAV composition may be administered by one or more administrations, either during the same procedure or spaced apart by days, weeks, months, or years. One or more of any of the routes of administration described herein may be used. In some embodiments, multiple vectors may be used to treat the human.

Methods to identify cells transduced by AAV viral particles are known in the art; for example, immunohistochemistry or the use of a marker such as enhanced green fluorescent protein can be used to detect transduction of viral particles; for example viral particles comprising a rAAV capsid with one or more substitutions of amino acids.

In some embodiments an effective amount of rAAV particles is administered to more than one location simultaneously or sequentially. In other embodiments, an effective amount of rAAV particles is administered to a single location more than once (e.g., repeated). In some embodiments, multiple injections of rAAV viral particles are no more than one hour, two hours, three hours, four hours, five hours, six hours, nine hours, twelve hours or 24 hours apart.

In some embodiments, the invention provides a method for treating a human with PKU by administering an effective amount of a pharmaceutical composition comprising a recombinant viral vector encoding a variant PAH polypeptide of the present disclosure. In some embodiments, the pharmaceutical composition comprises one or more pharmaceutically acceptable excipients.

In some embodiments, the methods comprise administering an effective amount of a pharmaceutical composition comprising a recombinant viral vector encoding a variant PAH polypeptide of the present disclosure to treat PKU in an individual in need thereof. In some embodiments, the viral titer of the viral particles (e.g., rAAV particles) is at least about any of 5 × 10¹², 6 × 10¹², 7 × 10¹², 8 × 10¹², 9 × 10¹², 10 × 10¹², 11 × 10¹², 15 × 10¹², 20 × 10¹², 25 × 10¹², 30 × 10¹², or 50 × 10¹² genome copies/mL. In some embodiments, the viral titer of the viral particles (e.g., rAAV particles) is about any of 5 × 10¹² to 6 × 10¹², 6 × 10¹² to 7 × 10¹², 7 × 10¹² to 8 × 10¹² , 8 × 10¹² to 9 × 10¹², 9 × 10¹² to 10 × 10¹², 10 × 10¹² to 11 × 10¹², 11 × 10¹² to 15 × 10¹², 15 × 10¹² to 20 × 10¹² , 20 × 10¹² to 25 × 10¹² , 25 × 10¹² to 30 × 10¹² , 30 × 10¹² to 50 × 10¹² , or 50 × 10¹² to 100 × 10¹² genome copies/mL. In some embodiments, the viral titer of the viral particles (e.g., rAAV particles) is about any of 5 × 10¹² to 10 × 10¹², 10 × 10¹² to 25 × 10¹², or 25 × 10¹² to 50 × 10¹²genome copies/mL. In some embodiments, the viral titer of the viral particles (e.g., rAAV particles) is at least about any of 5 × 10⁹, 6 × 10⁹, 7 × 10⁹, 8 × 10⁹, 9 × 10⁹, 10 × 10⁹, 11 × 10⁹, 15 × 10⁹, 20 × 10⁹, 25 × 10⁹, 30 × 10⁹, or 50 × 10⁹ transducing units /mL. In some embodiments, the viral titer of the viral particles (e.g., rAAV particles) is about any of 5 × 10⁹ to 6 × 10⁹, 6 × 10⁹ to 7 × 10⁹, 7 × 10⁹ to 8 × 10⁹, 8 × 10⁹ to 9 × 10⁹, 9 × 10⁹ to 10 × 10⁹, 10 × 10⁹ to 11 × 10⁹, 11 × 10⁹ to 15 × 10⁹, 15 × 10⁹ to 20 × 10⁹, 20 × 10⁹ to 25 × 10⁹, 25 × 10⁹ to 30 × 10⁹, 30 × 10⁹ to 50 × 10⁹ or 50 × 10⁹ to 100 × 10⁹ transducing units /mL. In some embodiments, the viral titer of the viral particles *(e.g.,* rAAV particles) is about any of 5 × 10⁹ to 10 × 10⁹, 10 × 10⁹ to 15 × 10⁹, 15 × 10⁹ to 25 × 10⁹, or 25 × 10⁹ to 50 × 10⁹ transducing units /mL. In some embodiments, the viral titer of the viral particles (e.g., rAAV particles) is at least any of about 5 × 10¹⁰, 6 × 10¹⁰, 7 × 10¹⁰, 8 × 10¹⁰, 9 × 10¹⁰, 10 × 10¹⁰, 11 × 10¹⁰, 15 × 10¹⁰, 20 × 10¹⁰, 25 × 10¹⁰, 30 × 10¹⁰, 40 × 10¹⁰, or 50 × 10¹⁰ infectious units/mL. In some embodiments, the viral titer of the viral particles (e.g., rAAV particles) is at least any of about 5 × 10¹⁰ to 6 × 10¹⁰, 6 × 10¹⁰ to 7 × 10¹⁰, 7 × 10¹⁰ to 8 × 10¹⁰, 8 × 10¹⁰ to 9 × 10¹⁰, 9 × 10¹⁰ to 10 × 10¹⁰, 10 × 10¹⁰ to 11 × 10¹⁰, 11 × 10¹⁰ to 15 × 10¹⁰, 15 × 10¹⁰ to 20 × 10¹⁰, 20 × 10¹⁰ to 25 × 10¹⁰, 25 × 10¹⁰ to 30 × 10¹⁰, 30 × 10¹⁰ to 40 × 10¹⁰, 40 × 10¹⁰ to 50 × 10¹⁰, or 50 × 10¹⁰ to 100 × 10¹⁰ infectious units/mL. In some embodiments, the viral titer of the viral particles (e.g., rAAV particles) is at least any of about 5 × 10¹⁰ to 10 × 10¹⁰, 10 × 10¹⁰ to 15 × 10¹⁰, 15 × 10¹⁰ to 25 × 10¹⁰, or 25 × 10¹⁰ to 50 × 10¹⁰ infectious units/mL.

In some embodiments, the dose of viral particles administered to the individual is at least about any of 1 × 10⁸ to about 6 × 10¹³ genome copies/kg of body weight. In some embodiments, the dose of viral particles administered to the individual is about any of 1 × 10⁸ to about 6 × 10¹³ genome copies/kg of body weight.

In some embodiments, the total amount of viral particles administered to the individual is at least about any of 1 × 10⁹ to about 1 × 10¹⁴ genome copies. In some embodiments, the total amount of viral particles administered to the individual is about any of 1 × 10⁹ to about 1 × 10¹⁴ genome copies.

Compositions of the invention (e.g., recombinant viral particles comprising a vector encoding a variant PAH polypeptide of the present disclosure) can be used either alone or in combination with one or more additional therapeutic agents for treating PKU. The interval between sequential administration can be in terms of at least (or, alternatively, less than) minutes, hours, or days.

An effective amount of rAAV (in some embodiments in the form of particles) is administered, depending on the objectives of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells, in some embodiments at least about 20% of the cells of the desired tissue type, in some embodiments at least about 50%, in some embodiments at least about 80%, in some embodiments at least about 95%, in some embodiments at least about 99% of the cells of the desired tissue type. The rAAV composition may be administered by one or more administrations, either during the same procedure or spaced apart by days, weeks, months, or years. In some embodiments, multiple vectors may be used to treat the mammal (e.g., a human).

In some embodiments, a rAAV composition of the present disclosure may be used for administration to a human. In some embodiments, a rAAV composition of the present disclosure may be used for pediatric administration. Without wishing to be bound to theory, because many of the symptoms of PKU are developmental in nature (e.g., severe mental disorders), it may be particularly advantageous to treat PKU as early in life as possible. In some embodiments, an effective amount of rAAV (in some embodiments in the form of particles) is administered to a patient that is less than one month, less than two months, less than three months, less than four months, less than five months, less than six months, less than seven months, less than eight months, less than nine months, less than ten months, less than eleven months, less than one year, less than 13 months, less than 14 months, less than 15 months, less than 16 months, less than 17 months, less than 18 months, less than 19 months, less than 20 months, less than 21 months, less than 22 months, less than two years, or less than three years old.

In some embodiments, a rAAV composition of the present disclosure may be used for administration to a young adult. In some embodiments, an effective amount of rAAV (in some embodiments in the form of particles) is administered to a patient that is less than 12 years old, less than 13 years old, less than 14 years old, less than 15 years old, less than 16 years old, less than 17 years old, less than 18 years old, less than 19 years old, less than 20 years old, less than 21 years old, less than 22 years old, less than 23 years old, less than 24 years old, or less than 25 years old.

In some embodiments, the invention provides methods to treat PKU comprising administering to an individual in need thereof, a therapeutically effective amount of cells comprising nucleic acid encoding a variant PAH polypeptide as described herein. In some embodiments, the cell comprises nucleic acid encoding a variant PAH polypeptide comprising at least three amino acid substitutions, wherein the amino acid substitutions are at sites selected from M180, K199, S250, and G256 of a wild-type human PAH polypeptide. In some embodiments, the nucleic acid in the cell encodes a variant PAH polypeptide comprising four amino acid substitutions at sites M180, K199, S250, and G256 of a wild-type human PAH polypeptide. In some embodiments, the nucleic acid in the cell encodes a variant PAH polypeptide comprising at least three amino acid substitutions selected from M180T, K199P, S250P, and G256A. In some embodiments, the nucleic acid in the cell encodes a variant PAH polypeptide comprising amino acid substitutions M180T, K199P, S250P, and G256A. In some embodiments, the nucleic acid in the cell encodes a variant PAH polypeptide comprising amino acid substitution comprises K199P, S250P, and G256A; M180T, S250P, and G256A; M180T, K199P, and G256A; or M180T, K199P, and S250P. In some embodiments, the nucleic acid in the cell encodes a variant PAH polypeptide comprising amino acid substitutions M180T, K199P, S250P, and G256A. In some embodiments, the nucleic acid in the cell encodes a variant PAH polypeptide further comprising amino acid substitutions H264P, G272A, G272P, P275L, P279Q, G272P and P275L, or T323R and F327T amino acid substitutions. In some embodiments, the nucleic acid in the cell encodes a variant PAH polypeptide that is any one of the variant PAH polypeptides presented in Tables 1-3. In some embodiments, the nucleic acid in the cell encodes a variant PAH polypeptide comprising amino acid substitutions M180T, K199P, S250P, and G256A and comprises further amino acid substitutions while maintaining at least about the phenylalanine hydroxylase activity of wild-type PAH. In some embodiments, the location of the amino acid substitutions encoded by the nucleic acid in the cell is based on wild-type human PAH polypeptide; for example, the human PAH polypeptide comprising the amino acid sequence of SEQ ID NO: 1.

In some embodiments, the nucleic acid in the cell encodes a variant human PAH polypeptide. In some embodiments, the nucleic acid in the cell encodes a variant PAH polypeptide comprising the amino acid sequence of SEQ ID NO:3. In some embodiments, the nucleic acid in the cell encodes a variant PAH polypeptide comprising an amino acid sequences that is at least about any of 80%, 85%, 90%, 95% or 99% identical to the amino acid sequence of SEQ ID NO:3. In some embodiments, the nucleic acid in the cell encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH. In some embodiments, the nucleic acid in the cell encodes a variant PAH polypeptide comprising an amino acid sequences that is at least about any of 80%, 85%, 90%, 95% or 99% identical to the amino acid sequence of SEQ ID NO:3 and has at least about 25%, 50%, 75%, 100%, or greater than 100% the phenylalanine hydroxylase activity of wild-type PAH of SEQ ID NO:1.

In some embodiments, the nucleic acid in the cell encodes a variant PAH polypeptide that is a truncated PAH polypeptide that maintains phenylalanine hydroxylase activity. In some embodiments, the nucleic acid in the cell encodes a truncated PAH polypeptide comprising an N-terminal truncation. In some embodiments, the nucleic acid in the cell encodes a N-terminal truncation that is a truncation of part or all of the N-terminal regulatory domain. In some embodiments, the nucleic acid in the cell encodes a N-terminal truncation comprising a deletion of amino acid residue 1 to about amino acid residue 102 of the wild-type human PAH polypeptide (e.g., the PAH polypeptide of SEQ ID NO: 1). In some embodiments, the nucleic acid in the cell encodes a truncated PAH polypeptide comprising a C-terminal truncation. In some embodiments, the nucleic acid in the cell encodes a C-terminal truncation that is a truncation of part or all of the tetramerization domain. In some embodiments, the nucleic acid in the cell encodes a C-terminal truncation comprising a deletion of amino acid residues from about 429 to 452 of the wild-type human PAH polypeptide (e.g., the PAH polypeptide of SEQ ID NO: 1). In some embodiments, the nucleic acid in the cell encodes a variant PAH polypeptide comprising an N-terminal truncation and a C-terminal truncation. In some embodiments, the truncated PAH polypeptide comprises a truncation of part or all of the N-terminal regulatory sequence and part of all of the tetramerization domain. In some embodiments, the truncated PAH polypeptide comprises a deletion of amino acid residue 1 to about amino acid residue 102 and from about 429 to about 452 of the wild-type human PAH polypeptide (e.g., the PAH polypeptide of SEQ ID NO: 1). In some embodiments, the nucleic acid in the cell encodes a variant PAH polypeptide comprising an amino acid sequence corresponding to amino acid residues from about 102 to about 428 of the wild-type PAH polypeptide (e.g., the PAH polypeptide of SEQ ID NO: 1). In some embodiments, the nucleic acid in the cell encodes a variant PAH polypeptide comprises an amino acid sequence corresponding to amino acid residues from 102 to 428 of the wild-type PAH polypeptide (e.g., the PAH polypeptide of SEQ ID NO: 1). In some embodiments, the nucleic acid in the cell encodes a truncated PAH polypeptide further comprising four amino acid substitutions at sites M180, K199, S250, and G256 of a wild-type human PAH polypeptide (e.g., the PAH polypeptide of SEQ ID NO: 1). In some embodiments, the nucleic acid in the cell encodes a truncated PAH polypeptide comprising four amino acid substitutions selected from M180T, K199P, S250P, and G256A. In some embodiments, the nucleic acid in the cell encoding a truncated variant PAH polypeptide further comprises any one of the combinations of amino acid substitutions presented in Tables 1-3. In some embodiments, the nucleic acid in the cell encodes a truncated PAH polypeptide has at least about 25%, 50%, 75%, 100%, or greater than 100% the phenylalanine hydroxylase activity of wild-type PAH *(e.g.,* the PAH polypeptide of SEQ ID NO: 1).

In some embodiments, the invention provides methods of treating PKU by administering an effective amount of cells comprising nucleic acid encoding a variant PAH polypeptide of the present disclosure. The cells comprising the nucleic acid encoding a variant PAH polypeptide may be administered to a particular tissue of interest, or it may be administered systemically. In some embodiments, an effective amount of cells comprising the nucleic acid encoding a variant PAH polypeptide may be administered parenterally. Parenteral routes of administration may include without limitation intravenous, intraperitoneal, intraosseous, intra-arterial, intracerebral, intramuscular, intrathecal, subcutaneous, intracerebroventricular, intrahepatic, and so forth. In some embodiments, the cells are encapsulated or in a device. In some embodiments, the PAH expressing cells outside liver may require exogenously added or co-expressed cofactor BH4. In some embodiments, the cells are encapsulated or in a device which further comprises BH4. In some embodiments, an effective amount of cells comprising nucleic acid encoding a variant PAH polypeptide may be administered through one route of administration. In some embodiments, an effective amount of nucleic acid encoding a variant PAH polypeptide may be administered through a combination of more than one route of administration. In some embodiments, an effective amount of nucleic acid encoding a variant PAH polypeptide is administered to one location. In other embodiments, an effective amount of the variant PAH polypeptide may be administered to more than one location.

In some embodiments, the cells comprising nucleic acid encoding a variant PAH polypeptide is a hepatocyte, a muscle cell, fibroblast, an endothelial cell, an epithelial cell, a blood cell, a bone marrow cell, a stem cell, or an induced pluripotent stem cell. In some embodiments, the cell further comprises exogenously added cofactor BH4 and/or coexpressed cofactor BH4.

In some embodiments, the cell is a cell line *(e.g.,* a CHO cell line, a HeLa cell line, *etc*.). In some embodiments, the invention provides methods to produce a variant PAH polypeptide comprising culturing a cell comprising a nucleic acid encoding a variant PAH polypeptide (e.g., an expression vector encoding a variant PAH polypeptide) under conditions to produce the variant PAH polypeptide. In some embodiments, the method to produce a variant PAH polypeptide further comprises one or more steps of purifying the variant PAH polypeptide.

### VII. Kits or Articles of Manufacture

The variant PAH polypeptides, nucleic acids, rAAV vectors, particles, and/or pharmaceutical compositions as described herein may be contained within a kit or article of manufacture, e.g., designed for use in one of the methods of the invention as described herein.

Generally, the system comprises a cannula, one or more syringes (e.g., 1, 2, 3, 4 or more), and one or more fluids *(e.g.,* 1, 2, 3, 4 or more) suitable for use in the methods of the invention.

The syringe may be any suitable syringe, provided it is capable of being connected to the cannula for delivery of a fluid. In some embodiments, the system has one syringe. In some embodiments, the system has two syringes. In some embodiments, the system has three syringes. In some embodiments, the system has four or more syringes. The fluids suitable for use in the methods of the invention include those described herein, for example, one or more fluids each comprising an effective amount of one or more vectors as described herein, and one or more fluids comprising one or more therapeutic agents.

In some embodiments, the kit comprises a single fluid *(e.g.,* a pharmaceutically acceptable fluid comprising an effective amount of the vector). In some embodiments, the kit comprises 2 fluids. In some embodiments, the kit comprises 3 fluids. In some embodiments, the kit comprises 4 or more fluids. A fluid may include a diluent, buffer, excipient, or any other liquid described herein or known in the art suitable for delivering, diluting, stabilizing, buffering, or otherwise transporting a variant PAH polypeptide or rAAV vector composition of the present disclosure. In some embodiments, the kit comprises one or more buffers, e.g., an aqueous pH buffered solution. Examples of buffers may include without limitation phosphate, citrate, Tris, HEPES, and other organic acid buffers.

In some embodiments, the kit comprises a container. Suitable containers may include, e.g., vials, bags, syringes, and bottles. The container may be made of one or more of a material such as glass, metal, or plastic. In some embodiments, the container is used to hold a rAAV composition of the present disclosure. In some embodiments, the container may also hold a fluid and/or other therapeutic agent.

In some embodiments, the kit comprises an additional therapeutic agent with a rAAV composition of the present disclosure. In some embodiments, the rAAV composition and the additional therapeutic agent may be mixed. In some embodiments, the rAAV composition and the additional therapeutic agent may be kept separate. In some embodiments, the rAAV composition and the additional therapeutic agent may be in the same container. In some embodiments, the rAAV composition and the additional therapeutic agent may be in different containers. In some embodiments, the rAAV composition and the additional therapeutic agent may be administered simultaneously. In some embodiments, the rAAV composition and the additional therapeutic agent may be administered on the same day. In some embodiments, the rAAV composition may be administered within one day, two days, three days, four days, five days, six days, seven days, two weeks, three weeks, four weeks, two months, three months, four months, five months, or six months of administration of the additional therapeutic agent.

In some embodiments, the kit comprises a therapeutic agent to transiently suppress the immune system prior to AAV administration. In some embodiments, patients are transiently immune suppressed shortly before and after injection of the virus to inhibit the T cell response to the AAV particles (*e.g., see* Ferreira et al., Hum. Gene Ther. 25:180-188, 2014). In some embodiments, the kit further provides cyclosporine, mycophenolate mofetil, and/or methylprednisolone.

The rAAV particles and/or compositions of the invention may further be packaged into kits including instructions for use. In some embodiments, the kits further comprise a device for delivery (e.g., any type of parenteral administration described herein) of compositions of rAAV particles. In some embodiments, the instructions for use include instructions according to one of the methods described herein. In some embodiments, the instructions are printed on a label provided with (e.g., affixed to) a container. In some embodiments, the instructions for use include instructions for administering to an individual (e.g., a human) an effective amount of rAAV particles, e.g., for treating PKU in an individual.

### VIII. Exemplary Embodiments

Embodiment 1. A variant phenylalanine hydroxylase (PAH) polypeptide comprising two amino acid substitutions, wherein the amino acid substitutions are at sites selected from M180, K199, S250, and G256 of a wild-type human PAH polypeptide.

Embodiment 2. A variant phenylalanine hydroxylase (PAH) polypeptide comprising three amino acid substitutions, wherein the amino acid substitutions are at sites selected from M180, K199, S250, and G256 of a wild-type human PAH polypeptide.

Embodiment 3. A variant phenylalanine hydroxylase (PAH) polypeptide comprising four amino acid substitutions at sites M180, K199, S250, and G256 of a wild-type human PAH polypeptide.

Embodiment 4. The variant PAH polypeptide of any one of embodiments 1-3, wherein the amino acid substitution comprises one of more of M180T, K199P, S250P, and G256A.

Embodiment 5. The variant PAH polypeptide of any one of embodiments 1-4, wherein the amino acid substitution comprises K199P, S250P, and G256A; M180T, S250P, and G256A; M180T, K199P, and G256A; or M180T, K199P, and S250P.

Embodiment 6. The variant PAH polypeptide of any one of embodiments 1-5, wherein the amino acid substitution comprises M180T, K199P, S250P, and G256A.

Embodiment 7. The variant PAH polypeptide of any one of embodiments 1-6, wherein the variant PAH polypeptide further comprises H264P, G272A, G272P, P275L, P279Q, G272P and P275L, or T323R and F327T amino acid substitutions.

Embodiment 8. The variant PAH polypeptide of any one of embodiments 1-7, wherein the wild-type human PAH polypeptide comprises the amino acid sequence of SEQ ID NO:1.

Embodiment 9. The variant PAH polypeptide of any one of embodiments 1-8, wherein the variant PAH polypeptide is a human PAH polypeptide.

Embodiment 10. The variant PAH polypeptide of any one of embodiments 1-9, wherein the variant PAH polypeptide comprises an amino acid sequences that is at least about 80% identical to the amino acid sequence of SEQ ID NO:3.

Embodiment 11. The variant PAH polypeptide of any one of embodiments 1-6, wherein the variant PAH polypeptide comprises the amino acid sequence of SEQ ID NO:3.

Embodiment 12. The variant PAH of any one of embodiments 1-11, wherein the variant PAH polypeptide further comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide.

Embodiment 13. The variant PAH of any one of embodiments 1-12, wherein the variant PAH polypeptide further comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide.

Embodiment 14. The variant PAH of any one of embodiments 1-13, wherein the variant PAH polypeptide further comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide.

Embodiment 15. A variant PAH polypeptide, wherein the variant PAH polypeptide comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide.

Embodiment 16. A variant PAH polypeptide, wherein the variant PAH polypeptide comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide.

Embodiment 17. A variant PAH polypeptide, wherein the variant PAH polypeptide comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide.

Embodiment 18. The variant PAH polypeptide of any one of embodiments 1-17, wherein the variant PAH polypeptide comprises an N-terminal truncation.

Embodiment 19. The variant PAH polypeptide of embodiment 18, wherein the N-terminal truncation comprises a truncation of the N-terminal regulatory domain.

Embodiment 20. The variant PAH polypeptide of embodiments 18 or 19, wherein the N-terminal truncation comprises a truncation of amino acid residues 1-102 of the wild-type PAH polypeptide.

Embodiment 21. The variant PAH polypeptide of any one of embodiments 1-20, wherein the variant PAH polypeptide comprises a C-terminal truncation.

Embodiment 22. The variant PAH polypeptide of embodiment 21, wherein the C-terminal truncation comprises a truncation of the tetramerization domain.

Embodiment 23. The variant PAH polypeptide of embodiments 21 or 22, wherein the C-terminal truncation comprises a truncation of amino acid residues 429-452 of the wild-type PAH polypeptide.

Embodiment 24. The variant PAH polypeptide of any one of embodiments 1-23, wherein the variant PAH polypeptide comprises an amino acid sequence corresponding to amino acid residues 103-428 of the wild-type PAH polypeptide.

Embodiment 25. The variant PAH polypeptide of any one of embodiments 1-24, wherein the variant PAH polypeptide comprises one or more amino acid substitutions to eliminate potential protease cleavage sites.

Embodiment 26. The variant PAH polypeptide of embodiment 25, wherein the one or more amino acid substitutions to eliminate potential protease cleavage sites is located at positions 270-295 and/or 380-405 of the wild type PAH polypeptide.

Embodiment 27. The variant PAH polypeptide of any one of embodiments 1-26, wherein the variant PAH polypeptide is fused to a liver targeting polypeptide.

Embodiment 28. The variant PAH polypeptide of embodiment 27, wherein the liver targeting polypeptide is a HGF or fragments thereof or glycoproteins that bind to hepatocyte asialoglycoprotein receptor.

Embodiment 29. The variant PAH polypeptide of any one of embodiments 1-25, wherein the variant PAH polypeptide is PEGylated and/or nitrosylated.

Embodiment 30. The variant PAH polypeptide of embodiment 26, wherein the variant PAH polypeptide comprises a I374C amino acid substitution, wherein the cys residue at position 374 is nitrosylated.

Embodiment 31. A composition comprising the variant PAH polypeptide of any one of embodiments 1-30.

Embodiment 32. The composition of embodiment 31, wherein the composition further comprises a pharmaceutically acceptable carrier.

Embodiment 33. An isolated nucleic acid encoding the variant PAH polypeptide of any one of embodiments 1-30.

Embodiment 34. The isolated nucleic acid of embodiment 33, wherein the nucleic acid encoding the variant PAH polypeptide is operably linked to a promoter.

Embodiment 35. The isolated nucleic acid of embodiment 34, wherein the promoter is selected from a cytomegalovirus (CMV) immediate early promoter, an RSV LTR, a MoMLV LTR, a phosphoglycerate kinase- 1 (PGK) promoter, a simian virus 40 (SV40) promoter, a CK6 promoter, a transthyretin promoter (TTR), a mTTR482 promoter, a mA1MB2-mTTR482 promoter, a TK promoter, a tetracycline responsive promoter (TRE), an HBV promoter, an hAAT promoter, a LSP promoter, an LP1 promoter, a chimeric liver-specific promoter (LSP), an E2F promoter, a telomerase (hTERT) promoter; a cytomegalovirus enhancer/chicken beta-actin/Rabbit β-globin promoter (CAG) promoter, an elongation factor 1-alpha promoter (EFl-alpha) promoter, a human β-glucuronidase promoter, a chicken β-actin (CBA) promoter, a modified chicken β-actin (CBA) promoter or SEQ ID NO: 17, a retroviral Rous sarcoma virus (RSV) LTR promoter, a dihydrofolate reductase promoter, and a 13-actin promoter.

Embodiment 36. The isolated nucleic acid of embodiment 34 or 35, wherein the promoter is an LP1 promoter or an mA1MB2-mTTR482 promoter.

Embodiment 37. The isolated nucleic acid of any one of embodiments 33-36, wherein the nucleic acid further comprises a polyadenylation signal.

Embodiment 38. The isolated nucleic acid of embodiment 37, wherein the polyadenylation signal is a bovine growth hormone polyadenylation signal, an SV40 polyadenylation signal, or a HSV TK pA.

Embodiment 39. The isolated nucleic acid of any one of embodiments 33-38, wherein the nucleic acid further comprises an intron.

Embodiment 40. The isolated nucleic acid of embodiment 39, wherein the intron is a chicken β-actin (CBA)/rabbit β-globin hybrid intron.

Embodiment 41. The isolated nucleic acid of embodiment 39, wherein the intron is a modified chicken β-actin (CBA)/rabbit β-globin hybrid intron of SEQ ID NO: 15.

Embodiment 42. The isolated nucleic acid of any one of embodiments 33-41, wherein the nucleic acid further comprises one or more ITRs.

Embodiment 43. The isolated nucleic acid of any one of embodiments 33-42, wherein the nucleic acid further comprises a stuffer nucleic acid.

Embodiment 44. The isolated nucleic acid of embodiment 43, wherein the stuffer nucleic acid is optimized to remove ATG sequences.

Embodiment 45. The isolated nucleic acid of embodiment 44, wherein the stuffer nucleic acid is an A1AT intron stuffer sequence of SEQ ID NO: 16.

Embodiment 46. An isolated nucleic acid encoding a human PAH polypeptide, wherein the nucleic acid is codon-optimized.

Embodiment 47. The isolated nucleic acid of embodiment 46, wherein the nucleic acid sequence is at least 80% identical to the nucleic acid sequence of SEQ ID NO: 14.

Embodiment 48. The isolated nucleic acid of embodiment 46, wherein the nucleic acid comprises the nucleic acid sequence of SEQ ID NO: 14.

Embodiment 49. The isolated nucleic acid of embodiment 33, wherein the nucleic acid is an mRNA.

Embodiment 50. A composition comprising the nucleic acid of any one of embodiments 33-49.

Embodiment 51. The composition of embodiment 50, wherein the composition further comprises a pharmaceutically acceptable carrier.

Embodiment 52. A vector comprising the nucleic acid of any one of embodiments 33-49.

Embodiment 53. The vector of embodiment 52, wherein the vector is a recombinant adeno-associated virus (rAAV) vector.

Embodiment 54. An rAAV vector comprising the nucleic acid of any one of embodiments 33-41 or 43-49 flanked by one or more AAV inverted terminal repeat (ITR) sequences.

Embodiment 55. The rAAV vector of embodiment 54, wherein the nucleic acid of any one of embodiments 33-48 is flanked by two AAV ITRs.

Embodiment 56. The rAAV vector of embodiment 54 or 55, wherein the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs.

Embodiment 57. The rAAV vector of any one of embodiments 54-56, wherein the AAV ITRs are AAV2 ITRs.

Embodiment 58. The rAAV vector of embodiment 57, wherein the rAAV vector comprises 5' to 3' an AAV2 ITR, a promoter, an intron, nucleic acid encoding a PAH polypeptide, a stuffer nucleic acid, a polyadenylation signal, and an AAV2 ITR.

Embodiment 59. The rAAV vector of embodiment 58, wherein the promoter is a m1A1MB2-mTTR482 promoter or an LP1 promoter.

Embodiment 60. The rAAV vector of embodiment 58 or 59, wherein the intron is a chicken β-actin (CBA)/rabbit β-globin hybrid intron.

Embodiment 61. The rAAV vector of any one of embodiments 58-59, wherein the PAH polypeptide is the variant PAH polypeptide of any one of embodiments 1-30.

Embodiment 62. The rAAV vector of any one of embodiments 58-60, wherein the nucleic acid encoding the PAH polypeptide is the codon optimized nucleic acid of any one of embodiments 46-48.

Embodiment 63. The rAAV vector of any one of embodiments 58-62, wherein the stuffer nucleic acid comprises nucleic acid from an intron of the human alpha 1 antitrypsin gene.

Embodiment 64. The rAAV vector of embodiment 63, wherein the intron of the human alpha 1 antitrypsin gene has been mutated to remove ATG sequences.

Embodiment 65. The rAAV vector of any one of embodiments 58-64, wherein the polyadenylation signal is a bovine growth hormone polyadenylation signal.

Embodiment 66. The rAAV vector of any one of embodiments 53-65, wherein the vector is a self-complimenting vector.

Embodiment 67. The rAAV vector of embodiment 66, wherein the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length.

Embodiment 68. The rAAV vector of embodiment 67, wherein the first nucleic acid sequence and the second nucleic acid sequence are linked by a mutated AAV ITR, wherein the mutated AAV ITR comprises a deletion of the D region and comprises a mutation of the terminal resolution sequence.

Embodiment 69. A rAAV particle comprising the rAAV vector of any one of embodiments 53-68.

Embodiment 70. The rAAV particle of embodiment 69, wherein the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid.

Embodiment 71. The rAAV particle of embodiment 69, wherein the AAV viral particle comprises an engineered AAV capsid.

Embodiment 72. The rAAV particle of embodiment 71, wherein the engineered AAV capsid is a DJ capsid or an LK03 capsid.

Embodiment 73. The rAAV particle of embodiment 69 or 70, wherein the ITR and the capsid of the rAAV viral particle are derived from the same AAV serotype.

Embodiment 74. The rAAV particle of embodiment 69 or 70, wherein the ITR and the capsid of the rAAV viral particles are derived from different AAV serotypes.

Embodiment 75. The rAAV particle of any one of embodiments 69-70 or 73-74, wherein the rAAV viral particle comprises an AAV8 capsid.

Embodiment 76. The rAAV particle of embodiment 74, wherein the rAAV viral particle comprises an AAV8 capsid, and wherein the vector comprises AAV2 ITRs.

Embodiment 77. A composition comprising the rAAV particle of any one of embodiments 69-76.

Embodiment 78. The composition of embodiment 77, wherein the composition further comprises a pharmaceutically acceptable carrier.

Embodiment 79. A cell comprising the nucleic acid of any one of embodiments 33-49 or the vector of claims 52 or 53 or the rAAV vector of any one of embodiments 54-68.

Embodiment 80. A method of producing a variant PAH polypeptide, the method comprising culturing the cell of embodiment 79 under conditions to produce the variant PAH polypeptide.

Embodiment 81. The methods of embodiment 80, further comprising the step of purifying the variant PAH polypeptide.

Embodiment 82. A method for treating phenylketonuria in an individual in need thereof, comprising administering to the individual the variant PAH polypeptide of any one of embodiments 1-30 or the composition of embodiment 31 or 32.

Embodiment 83. A method for treating phenylketonuria in an individual in need thereof, comprising administering to the individual a nucleic acid encoding the variant PAH polypeptide of any one of embodiments 1-30 or the nucleic acid of embodiment 33, 34 or 49.

Embodiment 84. A method for treating phenylketonuria in an individual in need thereof, comprising administering to the individual the rAAV vector of any one of embodiments 53-68.

Embodiment 85. A method for treating phenylketonuria in an individual in need thereof, comprising administering to the individual the rAAV particle of any one of embodiments 69-76.

Embodiment 86. A method for treating phenylketonuria in an individual in need thereof, comprising administering to the individual the composition of embodiment 31, 32, 50, 51, 77 or 78.

Embodiment 87. A method for treating phenylketonuria in an individual in need thereof, comprising administering to the individual the cell of embodiment 79.

Embodiment 88. The method of any one of embodiments 82-87, wherein the individual lacks PAH activity.

Embodiment 89. A method for reducing the level of phenylalanine in the blood of in an individual in need thereof, comprising administering to the individual the variant PAH polypeptide of any one of embodiments 1-30 or the composition of claim 31 or 32.

Embodiment 90. A method for reducing the level of phenylalanine in the blood of in an individual in need thereof, comprising administering to the individual nucleic acid encoding the variant PAH polypeptide of any one of embodiments 1-30 or the nucleic acid of embodiment 33, 34 or 49.

Embodiment 91. A method for reducing the level of phenylalanine in the blood of in an individual in need thereof, comprising administering to the individual the rAAV vector of any one of embodiments 53-68.

Embodiment 92. A method for reducing the level of phenylalanine in the blood of in an individual in need thereof, comprising administering to the individual the rAAV particle of any one of embodiments 69-76.

Embodiment 93. A method for reducing the level of phenylalanine in the blood of an individual in need thereof, comprising administering to the individual the composition of embodiment 31, 32, 50, 51, 77 or 78.

Embodiment 94. A method for reducing the level of phenylalanine in the blood of an individual in need thereof, comprising administering to the individual the cell of embodiment 79.

Embodiment 95. The method of any one of embodiments 89-94, wherein the level of phenylalanine in the blood of the individual prior to treatment is elevated compared to the level of phenylalanine in the blood of peer-matched control individuals.

Embodiment 96. The method of any one of embodiments 82-95, wherein the variant PAH polypeptide, the nucleic acid, rAAV vector, rAAV particle, composition or cell is administered intravenously, intraarterially, intrahepatically, intraportally, intraperitoneally, or subcutaneously.

Embodiment 97. The method of any one of embodiments 82-96, wherein the administration is in combination with another therapy.

Embodiment 98. The method of embodiment 97, wherein the another therapy is treatment with tetrahydribiopterin, treatment with phenylalanine ammonia lyase (PAL) or pegylated PAL, or a phenylalanine-restricted diet.

Embodiment 99. A method for making a PAH polypeptide comprising culturing the cell of embodiment 79 under conditions produce the PAH polypeptide.

Embodiment 100. The method or embodiment 99, further comprising purifying the PAH polypeptide.

Embodiment 101. A kit comprising the variant PAH polypeptide of any one of embodiments 1-24.

Embodiment 102. A kit comprising the nucleic acid of any one of embodiments 33-46, the rAAV vector of any one of embodiments 53-68, the rAAV particle of any one of embodiments 69-76, or the composition of embodiment 77 or 78.

Embodiment 103. The kit of embodiment 101 or 102, wherein the kit further comprises instructions for use; buffers and/or pharmaceutically acceptable excipients; and/or bottles, vials and/or syringes.

Embodiment 104. An expression cassette for expressing a transgene in a liver cell, wherein the expression cassette comprises a transgene operably linked to a promoter and enhancer, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer.

Embodiment 105. The expression cassette of embodiment 104, wherein the mTTR promoter is a mTTR482 promoter.

Embodiment 106. The expression cassette of embodiment 104 or 105, wherein the enhancer is 5' to the mTTR promoter.

Embodiment 107. An expression cassette for expressing a transgene in a liver cell, wherein the expression cassette comprises a transgene operably linked to a promoter and a 3' element, wherein the promoter comprises a mouse transthyretin (mTTR) promoter and the 3' element is an albumin 3' element (3'Alb) or an albumin 3' element linked to a human alpha 1 antitrypsin scaffold/matrix attachment region (SMAR) (3'AlbSMAR).

Embodiment 108. The expression cassette of embodiment 107, wherein the mTTR promoter is a mTTR482 promoter.

Embodiment 109. The expression cassette of embodiment 107 or 108, wherein the 3' element is located 3' to the transgene.

Embodiment 110. An expression cassette for expressing a transgene in a liver cell, wherein the expression cassette comprises a transgene operably linked to a promoter and enhancer and a 3' element, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer; and wherein the 3' element is an albumin 3' element (3'Alb) or an albumin 3' element linked to a human alpha 1 antitrypsin scaffold/matrix attachment region (SMAR) (3'AlbSMAR).

Embodiment 111. The expression cassette of embodiment 110, wherein the mTTR promoter is a mTTR482 promoter.

Embodiment 112. The expression cassette of embodiment 110 or 111, wherein the enhancer is 5' to the mTTR promoter.

Embodiment 113. The expression cassette of any one of embodiments 110-112, wherein the 3' element is located 3' to the transgene.

Embodiment 114. The expression cassette of any one of embodiments 104-113, wherein the expression cassette further comprises an intron.

Embodiment 115. The expression cassette of embodiment 114, wherein the intron is a chicken β-actin/rabbit β-globin hybrid intron.

Embodiment 116. The expression cassette of any one of embodiments 104-115, wherein the expression cassette further comprises a polyadenylation signal.

Embodiment 117. The expression cassette of embodiment 116, wherein the polyadenylation signal is a bovine growth hormone polyadenylation signal.

Embodiment 118. The expression cassette of any one of embodiments 104-117, wherein the transgene encodes a PAH polypeptide or a variant PAH polypeptide.

Embodiment 119. The expression cassette of embodiment 118, wherein the variant PAH polypeptide is the variant PAH polypeptide of any one of embodiments 1-30.

Embodiment 120. A vector comprising the expression cassette of any one of embodiments 104-119.

Embodiment 121. The vector of embodiment 120, wherein the vector is a recombinant adeno-associated virus (rAAV) vector.

Embodiment 122. An rAAV vector comprising the expression cassette of any one of embodiments 104-119 is flanked by one or more AAV inverted terminal repeat (ITR) sequences.

Embodiment 123. The rAAV vector of embodiment 122, wherein the expression cassette of any one of embodiments 104-118 is flanked by two AAV ITRs.

Embodiment 124. The rAAV vector of embodiment 122 or 123, wherein the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs.

Embodiment 125. The rAAV vector of any one of embodiments 122-124, wherein the AAV ITRs are AAV2 ITRs.

Embodiment 126. The rAAV vector of any one of embodiments 122-125, wherein the vector is a self-complimenting vector.

Embodiment 127. The rAAV vector of embodiment 126, wherein the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length.

Embodiment 128. The rAAV vector of embodiment 127, wherein the first nucleic acid sequence and the second nucleic acid sequence are linked by a mutated AAV ITR, wherein the mutated AAV ITR comprises a deletion of the D region and comprises a mutation of the terminal resolution sequence.

Embodiment 129. A rAAV particle comprising the rAAV vector of any one of embodiments 122-127.

Embodiment 130. The rAAV particle of embodiment 129, wherein the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid.

Embodiment 131. The rAAV particle of embodiment 130, wherein the AAV viral particle comprises an engineered AAV capsid.

Embodiment 132. The rAAV particle of embodiment 131, wherein the engineered AAV capsid is a DJ capsid or an LK03 capsid.

Embodiment 133. The rAAV particle of embodiment 131 or 132, wherein the ITR and the capsid of the rAAV viral particle are derived from the same AAV serotype.

Embodiment 134. The rAAV particle of embodiment 131 or 132, wherein the ITR and the capsid of the rAAV viral particles are derived from different AAV serotypes.

Embodiment 135. A composition comprising the rAAV particle of any one of embodiments 129-134.

Embodiment 136. The composition of embodiment 135, wherein the composition further comprises a pharmaceutically acceptable carrier.

Embodiment 137. A cell comprising the expression cassette of any one of embodiments 104-119 or the vector of any one of embodiments 120-128.

Embodiment 138. A method for treating phenylketonuria in an individual in need thereof, comprising administering to the individual the expression cassette of any one of embodiments 104-119.

Embodiment 139. A method for treating phenylketonuria in an individual in need thereof, comprising administering to the individual the rAAV vector of any one of embodiments 122-128.

Embodiment 140. A method for treating phenylketonuria in an individual in need thereof, comprising administering to the individual the rAAV particle of any one of embodiments 129-134.

Embodiment 141. A method for treating phenylketonuria in an individual in need thereof, comprising administering to the individual the composition of embodiment 135 or 136.

Embodiment 142. A method for treating phenylketonuria in an individual in need thereof, comprising administering to the individual the cell of embodiment 137.

Embodiment 143. The method of any one of embodiments 138-142, wherein the individual lacks PAH activity.

Embodiment 144. A method for reducing the level of phenylalanine in the blood of in an individual in need thereof, comprising administering to the individual the expression cassette of any one of embodiments 104-119.

Embodiment 145. A method for reducing the level of phenylalanine in the blood of in an individual in need thereof, comprising administering to the individual the rAAV vector of any one of embodiments 122-128.

Embodiment 146. A method for reducing the level of phenylalanine in the blood of in an individual in need thereof, comprising administering to the individual the rAAV particle of any one of embodiments 129-134.

Embodiment 147. A method for reducing the level of phenylalanine in the blood of an individual in need thereof, comprising administering to the individual the composition of embodiment 135 or 136.

Embodiment 148. A method for reducing the level of phenylalanine in the blood of an individual in need thereof, comprising administering to the individual the cell of embodiment 137.

Embodiment 149. The method of any one of embodiments 144-148, wherein the level of phenylalanine in the blood of the individual prior to treatment is elevated compared to the level of phenylalanine in the blood of peer-matched control individuals.

Embodiment 150. The method of any one of embodiments 138-149, wherein the nucleic acid, rAAV vector, rAAV particle, composition or cell is administered intravenously, intraarterially, intrahepatically, intraportally, intraperitoneally, or subcutaneously.

Embodiment 151. The method of any one of embodiments 138-150, wherein the administration is in combination with another therapy.

Embodiment 152. The method of embodiment 151, wherein the another therapy is treatment with tetrahydribiopterin, treatment with phenylalanine ammonia lyase (PAL) or pegylated PAL, or a phenylalanine-restricted diet.

Embodiment 153. A kit comprising the expression cassette of any one of embodiments 104-119, the rAAV vector of any one of embodiments 122-128, the rAAV particle of any one of embodiments 129-134, or the composition of embodiment 135 or 136.

Embodiment 154. The kit of embodiment 153, wherein the kit further comprises instructions for use; buffers and/or pharmaceutically acceptable excipients; and/or bottles, vials and/or syringes.

### EXAMPLES

The invention will be more fully understood by reference to the following examples. They should not, however be construed as limiting the scope of the invention. It is understood that the examples and embodiments described herein are for illustrative purposes only and that various modification or changes in light thereof will be suggested to persons skilled in the art and are to be included within the spirit and purview of this application and scope of the appended embodiments.

### Example 1. Generation of liver-specific expression cassette

The following study was conducted to develop a strong expression construct for expression of a transgene in the liver of an individual.

### Materials and methods for Examples 1-3

### Construction of PAH expression cassettes and rAAV vectors

To increase liver promoter strength, plasmid mTTR482-HI-hFVIII-BGHpA containing a mouse transthyretin (mTTR) promoter, an endogenous mTTR enhancer and a bovine growth hormone (BGH) polyadenylation (pA) site was used for additional modifications (Kyostio-Moore 2016, Nambiar 2017). In this plasmid, the FVIII cDNA was replaced with cDNA encoding secreted embryonic alkaline phosphatase (SEAP) and the existing intron was replaced with a 1069 bp chicken b-actin (CBA)/rabbit beta-globin hybrid intron. Various liver enhancer sequences were cloned upstream of the mTTR482 enhancer. These included modified prothrombin enhancer (mPrT2, two copies), modified alpha1-microbikunin (mA1MB2, two copies) (McEachern 2006, Jacobs 2008), modified mouse albumin enhancer (mEalb) (Kramer 2003), hepatitis B virus enhancer II (E11) (Kramer 2003), and CRM8 (Chuah 2014). Additionally, two 3' elements, albumin (Alb 3') (Wooddell 2008) and albumin plus human A1AT1 SMAR element (AF156542) (Alb3'/SMAR) were tested and cloned downstream of BGH pA **(****FIG. 1A****).** The modifications in some enhancers consisted of changing the hepatic nuclear factor (HNF) 3 and 4 binding sites to a higher affinity sequence in similar to that performed for mTTR482 previously (Nambiar 2017; Supplement 1).

For generating human or mouse PAH plasmid expression cassettes, the corresponding cDNAs encoding for full-length PAH (amino acids 1-452) were cloned into three expression plasmids. These plasmids contained liver-specific (LP1; Nathwani 2011), A1MB2- mTTR482 or CBA promoters, hybrid intron and pA sequences (BGH or simian virus 40 [SV40] pA) (Nathwani 2011, Kyostio-Moore 2016). All expression plasmids contained flanking AAV2 ITRs. The mTTR482 (Nambiar 2017) used as a backbone to evaluate various cDNAs encoding for hPAH. Four codon-optimized cDNAs were generated by algorithms from DNA2.0, GeneArt (GA) or Genscript (GS) and a GS algorithm combined with reduction in CpG content. In each expression plasmid the PAH protein was tagged with a N-terminal 22-amino acid 3xFLAG-peptide (MDYKDHDGDYKDHDIDYKDDDDK (SEQ ID NO: 18) for detection and quantitation purpose.

Selected AAV2 ITR containing plasmids with liver promoter, hybrid intron, PAH cDNA and BGHpA were used for rAAV vector production. rAAV vectors with AAV8 serotype capsid were generated using triple transfection method followed by CsCl2 purification (Univ. Massachusetts Gene Therapy Core). Vector lots were quantitated by qPCR to BGHpA (Nambiar 2017).

### In vitro cell cultures

All tissue culture reagents were obtained from Irvine Scientific (Santa Ana, CA) or Invitrogen. For transient transfection, human 293 or human liver carcinoma cells (Huh7 or HepG2) (8 × 10⁵ cells/well) were plated on 6-well dishes in Dulbecco's modified Eagle's medium (DMEM) with high glucose, 10% fetal bovine serum (FBS) and 10 ml/L Pen Strep (10 units/ml penicillin and 10 µg/ml streptomycin). Plasmids (2 µg) were transfected with Lipofectamine 2000 (Invitrogen). Cell lysates or culture media were harvested for PAH analysis or SEAP activity, respectively, 48 or 72 h later.

### Quantitation of SEAP activity

Conditioned media or sera were collected and samples were heated at 65°C for 30 min to inactivate endogenous alkaline phosphatases. SEAP activity was measured using Alkaline Phosphatase (ALP)-SL reagents (Sekisui Diagnostics) and samples were incubated for 10 min to 2 h followed by reading absorbance at 405 nm. Human Alkaline Phosphatase HPAP was used as a standard (Calbiochem). SEAP activity was expressed as ug/ml by dividing the sample value by the conversion factor 0.103 mU/ng.

### Quantitation of PAH protein and activity

Whole cell lysates were generated 48 h later by lysing cells in lysis buffer or in RIPA buffer. Additionally, sonication or shearing was used to enhance cell lysis in some experiments. Upon thawing, lysates were spun at 14,000 g for 30 min before assays. Quantitation of FLAG-fusion protein levels were measured by FLAG ELISA (SE002-flag; ABSbio) according to manufacturer's instructions and using either kit standard or in-house purified 3xFLAG-mPAH-FL as protein standard. In vivo samples were normalized by total protein content measured by BCA protein assay kit (Pierce). The enzyme activity of PAH proteins was measured as previously described in Yew *et al.* 2013 with some minor modifications. Western blotting for PAH detection was performed using anti-hPAH antibody (LS-C344145; LSBio) or anti-FLAG antibody (F1804; Sigma) using standard protocols.

### In vivo studies

A colony of BTBR-PAH^{enu2}/J mice were maintained at Taconic (McDonald 1996). Homozygous (HOM) and heterozygous (HETs) male mice were obtained at 8-12 weeks of age and were housed and maintained in accordance with humane guidelines for animal care and use. For SEAP expression plasmid analysis, plasmids (10 µg/mouse) in sterile saline were administered by hydrodynamic high-volume injections into the tail veins of C57BL/6 mice with minimum of n=8 per test article. Recombinant AAV8 vectors were administered by intravenous route via tail vein (6-10 animals/treatment). In most studies, animals were sacrificed 8-10 weeks later by isoflurane anesthesia. Whole blood was collected by retro-orbital sinus into EDTA collection tubes, spun and stored frozen until analysis. Some animals were perfused with PBS via the left cardiac ventricle before tissue collection. Liver samples were collected and frozen until analysis. For brain analyses, whole brain harvested from the cranium, weight, cut sagittally and was frozen at -80°C until analysis.

### Blood and tissue analyses

The plasma Phe and Tyr levels were analyzed by UHPLC-MS/MS, using Transcend II LX4 multiplex system equipped with Dionex Ultimate 3000 HPLC (Thermo Fisher, Waltham, MA USA) hyphenated to an API 4000 triple quadrupole mass spectrometer (AB SCIEX, Framingham, MA USA). L-Phe and L-Tyr (Sigma-Aldrich, St. Louis, MO USA) were used to prepare standard solutions, and labeled L-Phe-13C9, 15N and labeled L-Tyr-13C9, 15N (Sigma-Aldrich) were used as internal standards. The MS/MS detection was carried out in positive ion mode. The analysis was performed using an Acquity BEH C18 column (1.7 µm, 2.1 × 30 mm) with gradient separation, which included a 10 sec hold at 98% mobile phase A (0.1% formic acid in water) followed by a 2-45% mobile phase B (0.1% formic acid in acetonitrile) gradient over 30 sec, an increase to 75% B over 15 sec, a 10 sec wash at 75%B, and re-equilibration at 98% A for 1 min at a 0.5 mL/min flow rate. MS/MS transitions were: 166.1/120.1 for Phe, 182.1/136.1 for Tyr, 176.1/129.1 for labeled Phe, and 192.1/145.1 for labeled Tyr.

For brain neurotransmitter quantitation, brains were processed as described with minor modifications (Kankaanpaa 2001). Briefly, half of sagittally cut, PBS-perfused brains were homogenized in ice-cold lysis buffer (1 mM oxalic acid, 3 mM cysteine, 0.1 M acetic acid) with Bead Ruptor 24 (Omni) at 4.85 m/sec for 20 sec at 4°C . The samples were spun 14K for 10 min and subsequent supernatant frozen. Brain homogenates (200 mg/mL) were then quantitated for L-Dopa, Dopamine, Homovanillic acid (HVA), Serotonin, 5-Hydroxytryptophan (5-HTP), 5-Hydroxyindoleacetic acid (5-HIAA) and Norepinephrine levels by UPLC-MS/MS, using an Acquity UPLC (Waters Corporation, Milford, MA USA) hyphenated to an API 5000 triple quadrupole mass spectrometer (AB SCIEX, Framingham, MA USA). To increase neurotransmitter stability, 300 ng/mL Cysteine in 0.1% formic acid (FA) and acetonitrile was used as the sample diluent buffer. Standards for each analyte (Sigma-Aldrich) and standard solutions were prepared similar to the samples as described above. The MS/MS detection was carried out in positive ion mode (HVA was detected in negative ion mode). The analysis was performed using an Acquity HSS C18 SB (1.7 µm, 2.1 × 100 mm) with gradient separation, which included a 0.5 min hold at 98% mobile phase A (0.1% FA in water) followed by a 2-40% mobile phase B (0.1% FA in acetonitrile) gradient over 3.5 min, an increase to 95% B over 0.1 min, a 0.5 min wash at 95% B, and re-equilibration at 98% A for 2.4 min at a 0.5 mL/min flow rate. The positive ion MS/MS transitions were: 198/152 for L-Dopa, 177/160.1 for Serotonin, 154/137.1 for Dopamine, 192/146 for 5-HIAA, 170.1/107 for Norepinephrine, 221.1/201 for 5-HTP. HVA detection was carried out in negative ion mode, similar to the method described above except that the initial hold phase of the gradient was eliminated. The MS/MS transition for HVA in negative ion mode was 181.1/137.1. The levels were expressed as µg/mL homogenate or uM. Brain Phe and Tyr levels were quantitated as described in the above section.

For liver samples, vector genomes, PAH activity and protein levels were quantitated. Copies of vector genomes were quantitated by qPCR (Martin 2013). Quantitation of FLAG-tagged protein was performed as follows. Liver samples were homogenized in tubes with beads (15-340-153; Fisher Scientific) in 1× PBS or RIPA buffer with 1× protease inhibitors at 5.65 rpm at 4°C for 20-30 sec in homogenizer (Bead Ruptor 24; Omni). Samples were spun at 14,000 g at 4°C for 30 min. Supernatants were then used for PAH quantitation by FLAG ELISA assay and normalized by total protein (BCA protein assay kit; Pierce). PAH protein activity in liver homogenates was performed as described above.

### Results

A liver mTTR482 promoter containing mouse transthyretin core promoter and its 5' enhancer element with modifications in the HNF-3 and -4 binding sites was previously described (Nambiar 2016). A goal of the present study was to further increase promoter strength. Various liver-based enhancers upstream of this promoter or 3' stability elements downstream of BGH pA were added to the expression cassettes and then measured secreted alkaline phosphatase (SEAP) levels as a reporter in two human liver lines in vitro **(****FIG. 1A****).** Transient transfections of the SEAP plasmids into Huh7 and HepG2 cells showed that the expression cassette with mA1MB2 enhancer (#3) resulted in the highest SEAP levels in the culture media and was approximately 2-fold higher than the parental mTTR482 construct in both cell lines **(****FIGs. 1B, 1C****).** In vivo evaluation of previral plasmids by hydrodynamic injections into normal C57BL/6 mice also demonstrated the highest and most sustained SEAP expression with the plasmid containing the mA1MB2 enhancer. SEAP levels were 2.8-, 4.4-, 4- and 3.6-fold higher than detected in animals treated with the parental mTTR482-SEAP plasmid on days 1, 7, 14 and 28, respectively **(****FIG. 2A****).** The mTTR482 previral plasmid with and without the mA1MB2 enhancer in PAH^{enu2} mice was evaluated **(****FIG. 2B****).** The mA1MB2 enhancer containing plasmid generated consistently 2-fold higher plasma SEAP levels than obtained with the parental mTTR482 construct. Though SEAP was stably secreted throughout the 28-day study, the SEAP levels were approximately 10-fold lower in the PKU mice than obtained with these plasmids in C57Bl/6 mice. Based on this data, the mA1MB2-mTTR482 promoter was selected as the liver promoter candidate.

### Example 2. Comparison of optimized liver promoter to LP1 promoter in PAH^{enu2} mice

To validate our optimized liver promoter in PKU disease model, an AAV8/mA1MB2-mPAH vector was generated and compared it to AAV8/scLP1-mPAH comparable to vector used in Yagi *et al.* (2011). Both vectors were administered intravenously at two different doses (4e10 and 1e11, VG/mouse) and blood Phe levels were measured. With both doses, the mA1MB2-mTTR482 vector was more efficacious in reducing blood Phe levels compared to sc-LP1 vector **(****FIGs. 3A, 3B****).** Both vectors increased blood Tyr levels using the higher dose (1e11 vg/mouse); the elevation was already detected at day 7 (first post administration measurement) and was maintained elevated till day 56 (end of study) **(****FIG. 3C****).** Though blood Tyr increase was also detected in the lower dose treated animals, they were not significantly different from the pretreatment values of corresponding animals. Quantitation of viral vector genomes in liver demonstrated 2-to 3-fold higher VG copies with the self-complementary vector on day 56 **(****FIG. 3D****).**

Since brain Phe levels is the main cause for pathology, Phe levels in the brain were measured. With 1e11 dose, only the mA1MB2-mTTR482 construct provided low brain Phe levels similar to that of wild-type brains (HETs) **(****FIG. 4A****).** The reduction of brain Phe levels correlated well with levels of Phe measured in blood **(****FIG. 4B****).** Analysis of brain neurotransmitter levels demonstrated comparable elevation of dopamine with both constructs **(****FIG. 4C****).** Similar efficacy was also seen with the mA1MB2-mTTR482 construct at the lower (4e10) dose. Brain serotonin quantitation demonstrated significantly more serotonin generated with treatment using the mA1MB2-mTTR482 construct compared to that of the sc-LP1 vector (FIG. 4D). In summary, efficient reduction of blood Phe levels with the mA1MB2-mTTR482 construct translated to a robust decrease of brain Phe levels and subsequent enhancement of dopamine and serotonin synthesis in the brains of treated animals.

### Example 3. Evaluation of hPAH production from codon-optimized hPAH cDNAs

Previous published studies have shown poor efficacy with rAAV vectors encoding hPAH in PAH^{enu2} mice. To test whether improved efficacy could be obtained with a better produced hPAH, the effect of various codon usage was tested. For this, four different codon-optimized cDNAs for hPAH were generated based on different algorithms. The resulting sequences were cloned downstream from mTTR482 promoter and hPAH protein levels were evaluated in vitro and in vivo. Plasmid transfection into human Huh7 cells showed little differences among the hPAH cDNAs (all within 2-fold) **(****FIG. 5A****).** When expression plasmids were delivered into livers of normal C57BL/6 mice via hydrodynamic injection, much larger differences were observed among the constructs **(****FIG. 5B****).** In particular, expression plasmid with hPAH cDNA generated by GA algorithm resulted in 7-fold higher FLAG-tagged protein detection in the liver lysates compared to that with plasmid with the endogenous, non-modified (non) sequence. Based on this, the human cDNA generated with GA algorithm was used for subsequent studies.

### Example 4: Comparison of Human PAH and mouse PAH in vitro and in vivo

### Methods

### Plasmid vectors and recombinant AAV generation.

The generation of liver-specific promoters (LP1 and mTTR482), hybrid intron, polyadenylation sites (bovine growth hormone [BGH] and simian virus 40 [SV40]) are known in the art and have been described in Nathwani (2012) and Nambiar (2017). Various plasmid vectors with liver-specific or chicken b-actin (CBA) promoter, hybrid intron, cDNAs encoding full-length (FL) human (h) or mouse (m) PAH and BGH polyA were constructed. Expression cassettes with double-truncated forms of mPAH and hPAH (DT; amino acids 103-428) or with various hybrid constructs were also generated. Amino acid modifications in hPAH-DT and in mPAH were evaluated in plasmid vectors with CBA promoter, hybrid intron, PAH-DT and BGH polyA. Amino acid changes in variants (V) of PAH-DT amino acid sequence were generated by synthesizing altered DNA sequence or introducing changes by site-directed mutagenesis (Genscript). The majority of constructs contained a sequence encoding N-terminal 22-amino acid 3xFLAG-peptide (MDYKDHDG DYKDHDI DYKDDDDK (SEQ ID NO: 18) upstream of PAH cDNA for detection and quantitation purpose.

Selected PAH plasmid vectors with liver-specific promoter (LP1 or mA1MB2-mTTR482), 1069 bp chicken β-actin (CBA)/rabbit β-globin hybrid intron, hPAH cDNA (or variant) cDNA and BGHpA were subcloned into AAV2 ITR containing plasmids. The final hPAH-V1 vector construct additionally contained a modified intron with ATGs removed and a 0.9 kb "filler" sequence from alpha 1 antitrypsin intron (with reduced ATGs) to increase vector genome size to 4.6 kb. All rAAV vectors were generated with AAV8 serotype capsid using triple transfection method followed by CsCl₂ purification. Vector lots were quantitated by qPCR to BGHpA (Martin 2013).

### Purification of PAH proteins.

Transient transfections of Expi293F cells (LifeTechnologies) were performed using Expi293F Expression system kit (LifeTechnologies) in serum-free, suspension culture setting according manufacturer's instructions. pCBA-PAH-BGHpA expression plasmids encoding 3xFLAG-tagged hPAH, hPAH-V1 and mPAH with FL or DT forms were used for transfections. Cells were spun down and whole cell lysates were generated in lysis buffer (50 mM Tris-HCl, pH 7.4, 150 mM NaCl, I mM EDTA and 1% Triton X-100). Flag-tagged PAH proteins were purified using FLAG-affinity column (ANTI-FLAG M2 affinity gel; Sigma-Aldrich). Proteins were eluted using either with 0.1 M glycine-HCl, pH 3.5 or 100 ug/ml FLAG-peptide in TBS (Sigma-Aldrich). Proteins were resuspended in 50 mM Tris-HCl, pH 7.4, 150 mM NaCl (some lots additionally contained 10% mannitol to improve stability, Nascimento 2010). Proteins were analyzed on SDS-PAGE 4-12% Tris-BIS-MES gel and stained by Coomassie Blue staining. Proteins were also analyzed by Western blotting using antibodies against the FLAG peptide or PAH protein.

### Quantitation of PAH protein and activity levels.

Transient transfection of HEK293T cells with plasmids containing FLAG-tagged PAH expression cassettes were performed using Lipofectamine 2000 (LifeTechnologies). Whole cell lysates were generated 48 h later by lysing cells in lysis buffer or in RIPA buffer (Pierce/ThermoFisher). Additionally, sonication or shearing was used to enhance cell lysis in some experiments. Upon thawing, lysates were spun at 14,000 g for 30 min before assays. Quantitation of FLAG-fusion protein levels were measured by FLAG ELISA (SE002-flag; ABSbio) according to manufacturer's instructions and using either kit standard or in-house purified 3xFLAG-mPAH-FL as protein standard. The enzyme activity of PAH proteins was measured as previously described (Yew 2013) with some minor modifications. Western blotting for PAH detection was performed using anti-hPAH antibody (LSBio) or anti-FLAG antibody (Sigma) using standard protocols known in the art

### Results

Production of human and mouse PAH proteins was assessed in Huh7 and 293 cells *in vitro.* For quantitation of hPAH and mPAH production, N-terminal 3xFLAG-tag was included in all expressed proteins. The data showed low levels of hPAH in whole Huh7 cell lysates compared to that of mPAH and was independent of expression cassette used **(****FIG. 6A****).** The lower production of hPAH was shown for the full-length (FL, 1-452) and the double-truncated (DT) version of hPAH compared to that of mPAH **(****FIGS. 6B, 6C****).** The DT version is a shortened and constitutively active form of PAH consisting of amino acids 103 to 428 and hence, lacks both the N-terminal regulatory domain and the C-terminal tetramerization domain of PAH.

Quantitation of mRNA for each construct showed comparable message levels indicating differences in hPAH and mPAH protein levels were post-transcriptional effects **(****FIG. 6D****).** When hPAH and mPAH (FL and DT forms) were purified using FLAG affinity column, the human PAH (both FL and DT forms) showed a very distinct degradation pattern that was not observed for the mouse PAH **(****FIG. 6E****).**

rAAV8/sc-LP1 vectors encoding human and mPAH were tested in PAH^{enu2} mice. Animals treated with the vector encoding mPAH showed normal blood Phe levels while very little reduction in blood Phe levels were observed in mice treated with comparable dose of hPAH vector **(****FIG. 6F****).** The efficacy of sc-LP1 vector encoding mPAH has previously been demonstrated (Yagi 2011).

Taken together, this data demonstrated that the endogenous form of hPAH was less potent than that of mPAH and that this correlated to poor production/stability of the hPAH protein observed *in vitro.*

### Example 5: Generation of improved variants for human PAH

Hybrid hPAH constructs were generated containing various lengths of N-terminal or C-terminal sequences derived from the mPAH **(****FIG. 7A****).** These constructs were tested for FLAG-tagged protein production *in vitro* and showed that neither the replacement of N- or C-terminus of hPAH with the corresponding mouse sequences improved protein levels **(****FIGS. 7B, 7C****).** Hence, the region containing the catalytic core (103-448), DT) was responsible for the stability of mPAH.

To improve the stability of hPAH core region, various modifications were introduced into the hPAH-DT backbone. Various amino acid modifications were introduced into hPAH sequence using the hPAH-DT backbone. The amino acid changes were modeled from amino acid sequences of PAHs in other species including mouse, chicken and bacteria (Table 1). All constructs also contained 3x-FLAG-tag in the flexible N-terminus. The human variants (V; V1-13) were expressed in 293 cells *in vitro* and whole cell lysates were quantitated for levels of PAH protein and activity and compared to that of DT-versions of hPAH and mPAH **(****FIG. 8A****).** The protein quantitation showed that some hPAH variants, such as V1, V5 (one additional change compared to V1) and V8 (two amino acid changes distinct from V1) had increased protein levels compared to the unmodified hPAH. However, when these variants were tested for PAH enzyme activity, only the V1 had significantly improved PAH activity **(****FIG. 8B****).** The V1 consisted of four amino acid changes located in three distinct parts of the catalytic domain, M180T, K199P, S250P and G256A. No changes were observed in constructs V9-13 (data not shown).

**Table 1. Human PAH variant descriptions**

| **Description** | **Changes** |
|---|---|
| Variant #1 | M180T, K199P, S250P, G256A |
| Variant #2 | M180T, K199P, S250P, G256A, G188A, G307A |
| Variant #3 | M180T, K199P, S250P, G256A, E228P, N376P |
| Variant #4 | M180T, K199P, S250P, G256A, E228P, L369P, N376P |
| Variant #5 | M180T, K199P, S250P, G256A, S251D |
| Variant #6 | E228P, L369P, N376P |
| Variant #7 | G272P, P279Q, P275L |
| Variant #8 | H264P, G275H |
| Variant #9 | C344T |
| Variant #10 | M180T, L192R, S196A, T206H, H220R, N376E |
| Variant #11 | M180T, K199P, S250P, G256A, G188A, G192R, S196A, T206H, H220R, N376E |
| Variant #12 | L114E, D116N |
| Variant #13 | L114E, D116N, M180T, G192R, S196A, T206H, H220R, N376E |
| Variant #14 | M180T |
| Variant #15 | K199P |
| Variant #16 | S250P |
| Variant #17 | G256A |
| Variant #18 | K199P, S250P, G256A |
| Variant #19 | M180T, S250P, G256A |
| Variant #20 | M180T, K199P, G256A |
| Variant #21 | M180T, K199P, S250P |

Additional derivatives of V1 were created. The first set included constructs with only one of the amino acid changed (V14-17, Table 1). The PAH activity results showed that all of them had poor PAH levels and activity that was comparable to the unmodified hPAH and hence indicating that none of these changes alone provided any improvement **(****FIGS. 9A-9C****,** **10A-10C****).** The second set of constructs were derivatives of V1 in which one of the four amino acids was reversed back to the human wild-type amino acid.

**Table 2. Human PAH variant-1 derivatives**

| **Description** | **Changes** |
|---|---|
| Variant #22 | M180T, K199P, S250P, G256A, H264P |
| Variant #23 | M180T, K199P, S250P, G256A, G272A |
| Variant #24 | M180T, K199P, S250P, G256A, G272P |
| Variant #25 | M180T, K199P, S250P, G256A, P275L |
| Variant #26 | M180T, K199P, S250P, G256A, P279Q |
| Variant #27 | M180T, K199P, S250P, G256A, G272P, P275L |
| Variant #28 | M180T, K199P, S250P, G256A, T323R, F327T |
| Variant #29 | M180T, G256A |
| Variant #30 | K199P, G256A |
| Variant #31 | S250P, G256A |

Whether three amino acid changes was sufficient (V18-21) was then determined. The best of these, V20 without the S250P change, had approximately 67% of V1 activity **(****FIG. 9A****).** Interestingly, this variant despite having lower overall enzyme activity due to reduction in PAH protein levels had 2-fold improved specific activity compared to the parental V1 **(****FIGS 9B, 9C****).**

A set of double-mutant derivatives of human PAH variant-1 were also tested. Unlike the single mutant series (V14-17), some improvement in activity was obtained with double-mutants V29 and V30, however, not as much as with V1 **(****FIG. 10D****).**

To further improve V1, additional changes were incorporated, some of which were designed to increase specific activity (V22-28, Table 2). Though none of these changes enhanced total PAH activity, some modifications did enhance PAH protein levels or its specific activity. For example, V23 had 2-fold higher PAH protein than that by hPAH-V1.

"Reverse" mutagenesis for mPAH was performed by sequentially changing two or three residues at a time for those present in hPAH within the 103-428 region. The activity testing of five constructs spanning this region showed that none of these alterations resulted in reduction of mPAH production to levels observed for hPAH (data not shown).

**Table 3. Mouse PAH variants**

| **Description** | **Changes** |
|---|---|
| Mouse Variant #1 | E114K, N116D, T180M |
| Mouse Variant #2 | R184K, R192K, A196S |
| Mouse Variant #3 | H206Y, R220H, E336Q |
| Mouse Variant #4 | D360E, C374I, E376N |
| Mouse Variant #5 | T401N, V421I |

An expression cassette encoding the best performing variant (V1) as a full-length protein and expressed from a A1MB2-mTTR promoter was constructed. This construct was compared to comparable mouse and hPAH expression plasmids for PAH protein production and activity after transfecting plasmids into Huh7 cells. The data demonstrated approximately 10-fold enhanced PAH protein production and activity by the hPAH-V1 compared to endogenous hPAH **(****FIGs. 11A-11C****).** All PAH proteins had comparable activity to protein ratio indicating roughly similar specific activity.

Taken together, the data demonstrated the feasibility of improving the characteristics of human PAH by amino acid changes (e.g., 2, 3, 4, 5, or 6 amino acid changes), all of which contributed to the observed improvement.

### Example 6: Correction of hyperphenylalaninemia by PAH-V1

### Methods

### Blood and tissue analyses.

Brain and tissue analyses was performed as described in Examples 1-3.

### Animal experiments.

Animal experiments were performed as described in Examples 1-3.

### Results

To test efficacy of the modified hPAH *in vivo,* an rAAV8 vector encoding hPAH-V1 was generated, as well as analogous vectors with mouse or hPAH (all in FL versions). The vector expressing hPAH-V1 showed a rapid reduction of blood Phe levels as well as increase in blood Tyr in the PAH^{enu2} mouse model of PKU **(****FIGS. 12A, 12B****).** A similar effect was observed with various Phe metabolites measured in blood **(****FIG. 12C****).** Overall, the modified hPAH-V1 was more efficacious for all these endpoints compared to the endogenous hPAH. The improved efficacy of hPAH-V1 directly correlated to improvement in PAH protein levels and activity in liver **(****FIGS. 13A, 13B****).** All treatment groups had comparable vector genome copies in the liver **(****FIG. 13C****).**

Various brain endpoints (performed for 3e11 VG/mouse treatment groups) were analyzed. The data demonstrated significant reduction of Phe levels and increase in Tyr levels in the brains of treated mice compared to those of naive PAH^{enu2} mice **(****FIGS. 14A, 14B****).** The changes in brain Phe levels correlated well with reduction in blood Phe levels and also showed better Phe control by the hPAH-V1 treatment group **(****FIG. 14C****).** Since amino acids Phe, Tyr and Trp all use the same large neutral amino acid transporter (LAT1), a similar improvement was observed in brain Trp levels **(****FIG. 14D****).** Both hPAH and hPAH-V1 resulted in comparable increases in these amino acid levels in brain.

Levels of brain neurotransmitters dopamine and serotonin and their metabolite levels in brains of treated mice were measured. The data showed a significantly higher increase of these neurotransmitters in hPAH-V1 treated animals compared to that of hPAH **(****FIGs. 15A-15D****).**

In summary, the improved properties of hPAH-V1 protein translated to enhanced ability to reduce blood and brain Phe levels as well as to increase Tyr and neurotransmitter levels in the brain. Since comparable vector genomes levels were present in liver, the data indicated higher potency obtained with the hPAH-V1 encoding vector.

A 4.6 kb genome for the mA1MB2-mTTR482 promoter/hPAH-V1 vector was generated. In addition to the stuffer sequence introduced downstream from the BGH pA, this genome contained no "ATG" sequences in the intron sequence **(****FIG. 16A****).** When this vector was tested in the PAH^{enu2} mice, a significantly better efficacy measured as reduction in blood Phe levels was observed when compared to the parental 3.8 kb vector **(****FIG. 16B****).** This experiment also contained a 4.6 kb vector lacking the N-terminal FLAG-tag used to measure PAH protein levels. The data showed that the removal of this tag did not alter the efficacy of the vector genome. Taken together, the modified hPAH-V1 confers improved potency over the endogenous hPAH and hence, is expected to provide efficacy with lower vector dose compared to that of hPAH encoding vector.

### Example 7. Improved hPAH-V1 protein levels in NHP liver

### Materials and methods:

### Construction of recombinant AAV vectors

Vector generation of A1MB2-HI- hPAH and -hPAH-V1 were performed as described in Example 4, except a hybrid capsid was used.

### Animal experiment

Male cynomolgus monkeys (*Macaca fascicularis*) at 2 to 3 years of age (2-4 kg) received 10 mls of test article by slow intravenous infusion (1 ml/min) into saphenous vein. The treatment groups consisted vehicle group (PBS, n=1), rAAV/hPAH (n=3) and rAAV/hPAH-V1 (n=3). The animals were humanely euthanized two weeks later and various tissues were collected and frozen at -80 °C until analysis.

### Quantitation of vector genomes, mRNA and PAH protein

Vector genome and vector derived mRNA copies in liver and spleen were quantitated by qPCR using primer/probes to BGHpA as described in Example 1-3. Levels of human PAH protein in liver homogenates were analyzed by Western blot for FLAG-tag as described in Example 1-3.

### Results:

To test the functionality of the A1MB2-mTTR promoter in NHP liver, both the mRNA and PAH production levels were evaluated two post after vector delivery. On average, comparable vector and vector derived mRNA levels were detected in livers of all vector treated animals (normalized to vector genome copies) **(****FIG 17A,B****).** Liver-specificity of expression was analyzed by comparing vector mRNA in liver and spleen (mRNA levels normalized to vector genome copies in both tissues) and demonstrated higher transcript/VG ratio in liver compared to that of spleen **(****FIG 17B****).** Transduction was also evaluated by testing vector derived FLAG-tagged PAH protein detection in liver. All animals treated with PAH-V1 encoding vector demonstrated the presence of hPAH-V1 of correct size in liver. In contrast, little to no hPAH protein was detected in animals treated with hPAH encoding vector **(****FIG 17C****).** Since both vector genomes and mRNA levels were comparable in both treatment groups, these results indicate better stability of hPAH-V1 protein in NHP liver. These observations were similar to studies performed in mice as well as in vitro studies with human liver cell lines indicating superiority of hPAH-V1 in all test systems.

### REFERENCES

Kochhar JS, et al., Drug Deliv Transl Res 2012, 2:223-237.
Ho G, Christodoulou J. Transl Pediatr 2014, 4:49-62.
Blau N, Longo N. Expert Opin Pharmacother 2015, 16:791-800.
Flydal MI, Martinez A. IUBMB Life 2013, 65:341-349.
Erlandsen H, et al., Pediatrics 2003, 112:1557-1565.
Knappskog PM, et al., Eur J Biochem 1996, 242:813-821.
Jaffe E, et al., Arch Biochem Biophys 2013, 530:73-82.
Arturo EC, et al., PNAS 2016, 113:2394-2399.
Waisbren SE, et al., Mol Genet Metab 2007, 92:63-70.
Thomas J, et al., J Inborn Errors Metabolism & Screening 5:1-9.
Cleary M, et al., Mol Gen Metab 2013, 110:418-423.
Gonzales MJ, et al., SeminPediatr Neurol 2016, 23:332-340.
Vogel KR, et al., J Inherit Metab Dis 2017, 40:227-235.
Burton B, et al., Mol Gen Metab 2015, 114:415-424.
Longo N, et al., Lancet 2014, 384:37-44.
Mochizuki S, et al., Gene Ther 2004, 11:1081-1086.
Ding Z, et al., Gene Therapy 2006, 13:587-593.
Harding CO, et al., Gene Therapy 2006, 13:457-462.
Yagi H, et al., J Gene Med 2011, 13:114-122.
Yagi H, et al., NeuroReport 2012, 23:30-34.
Winn SR, et al., Mol Gen Metabolism 2018, 123:6-20.
Oh H-J, et al., Pediatric Research 2004, 56:278-284.
Nathwani AC, et al., Blood 2006, 107:2653-2661.
Nambiar B, et al., Hum Gene Ther Methods 2017, 28:23-38.
Martin J, et al., Hum Gene Ther Methods 2013;24:253-269.
Yew NS et al., Mol Gen Metab 2013, 109:339-344.
McDonald JD, Charlton CK. Genomics 1996, 39:402-405.
Kyostio-Moore S, et al., Mol Ther Methods Clin Dev 2016, 3:16006.
Nascimento C, et al., Appl Biochem Biotechnol 2010, 162:192-207.
Baker RE, Shiman R. JBiol Chem 19: 9633-9639.
Ledley FD, et al., Biochem J 1990, 267:399-406.
Aquado C, et al., FEBs Letters 2006, 580:1697-1701.
Doskeland AP, Flatmark T. Biochim Biophys Acta 2001, 1547:379-386.
Solstad T, Flatmark T. Eur JBiochem 2000, 267:6302-6310.
Andersen OA, et al., J Mol Bio 2002, 320:1095-1108.
Erlandsen H, et al., J Mol Bio 2002, 320:645-661
Ziegler RJ, et al., Mol Ther 2007, 15:492-500.
Finn JD, et al., Blood 2010, 116:5842-5848.
George LA, et al., N Eng J Med 2017, 377:2215-2227.
Walter JH, etal., The Lancet 2002, 360:55-56.
Anderson PJ, Leuzzi V. Mol Gen Metab 2010, 99:S3-S9.
Garcia MI, et al., Mol Gen Metab 2017, 11:54-58.
Harding CO, et al., Mol Genet Metab 2018, March 31 (abstract)
Thomas J, et al., Mol Genet Metab 2018, March 18 (abstract).
McEachern KA, et al., J Gene Med 2006, 8:719-729.
Jacobs F, etal., Gene Ther 2008, 15:594-603.
Kramer MG, et al., Mol Ther 2003, 7:375-385.
Chuah MK, et al., Mol Ther 2014, 9:1605-1613.
Wooddell CI, et al., Gene Med 2008, 10:551-563.
Nathwani AC, etal., NEJM 2011, 365:2357-2365.
Jiang, H, etal., Blood 2006;108:107-115.
Park JW, et al., Exp Mol Med 2010, 42:105-115.
Charron CE, Lewin AS, Laipis PJ. Mol Ther 2004, 9:S334.
Grimm D, etal., J Virol. 2008, 82:5887-911.
Lisowski L, etal., Nature, 2014, 506:382-6.
Smith LJ, et al., Mol Ther. 2014 Sep;22(9):1625-34.

### SEQUENCES

Human phenylalanine hydroxylase (GenBank AAA60082.1 and AAC51772.1)- amino acid sequence 






 (SEQ ID NO:1)
Human phenylalanine hydroxylase (GenBank AAH26251.1)- amino acid sequence 
MSTAVLENPGLGRKLSDFGQETSYIEDNCNQNGAISLIFSLKEEVGALAKVLRLFEENDVNLTHIESRPS
RLKKDEYEFFTHLDKRSLPALTNIIKILRHDIGATVHELSRDKKKDTVPWFPRTIQELDRFANQILSYGA
ELDADHPGFKDPVYRARRKQFADIAYNYRHGQPIPRVEYMEEGKKTWGTVFKTLKSLYKTHACYEYNHIF
PLLEKYCGFHEDNIPQLEDVSQFLQTCTGFRLRPVAGLLSSRDFLGGLAFRVFHCTQYIRHGSKPMYTPE
PDICHELLGHVPLFSDRSFAQFSQEIGLASLGAPDEYIEKLATIYWFTVEFGLCKQGDSIKAYGAGLLSS
FGELQYCLSEKPKLLPLELEKTAIQNYTVTEFQPLYYVAESFNDAKEKVRNFAATIPRPFSVRYDPYTQR
IEVLDNTQQLKILADSINSEIGILCSALQKIK (SEQ ID NO:2)
Human PAH-V1 amino acid sequence (M180T, K199P, S250P and G256A) 
MSTAVLENPGLGRKLSDFGQETSYIEDNCNQNGAISLIFSLKEEVGALAKVLRLFEENDVNLTHIESRPS
RLKKDEYEFFTHLDKRSLPALTNIIKILRHDIGATVHELSRDKKKDTVPWFPRTIQELDRFANQILSYGA
ELDADHPGFKDPVYRARRKQFADIAYNYRHGQPIPRVEYTEEEKKTWGTVFKTLKSLYPTHACYEYNHIF
PLLEKYCGFHEDNIPQLEDVSQFLQTCTGFRLRPVAGLLPSRDFLAGLAFRVFHCTQYIRHGSKPMYTPE
PDICHELLGHVPLFSDRSFAQFSQEIGLASLGAPDEYIEKLATIYWFTVEFGLCKQGDSIKAYGAGLLSS
FGELQYCLSEKPKLLPLELEKTAIQNYTVTEFQPLYYVAESFNDAKEKVRNFAATIPRPFSVRYDPYTQR
IEVLDNTQQLKILADSINSEIGILCSALQKIK (SEQ ID NO:3)
Human PAH-V1 DNA sequence 
ATGAGCACAGCCGTGCTGGAAAACCCCGGCCTGGGCAGAAAGCTGAGCGACTTCGGCCAGGAAACCAGCTACATCGA
GGACAACTGCAACCAGAACGGCGCCATCAGCCTGATCTTCAGCCTGAAAGAAGAAGTGGGCGCCCTGGCCAAGGTGC
TGCGGCTGTTCGAGGAGAACGACGTGAACCTGACCCACATCGAGAGCCGGCCCAGCAGACTGAAGAAGGACGAGTAC
GAGTTCTTCACCCACCTGGACAAGCGGAGCCTGCCCGCCCTGACCAACATCATCAAGATCCTGCGGCACGACATCGG
CGCCACCGTGCACGAGCTGAGCCGGGACAAGAAAAAGGACACCGTGCCCTGGTTCCCCAGAACCATCCAGGAACTGG
ACAGATTCGCCAACCAGATCCTGTCCTACGGCGCCGAGCTGGATGCCGACCACCCTGGCTTCAAGGACCCCGTGTAC
CGGGCCAGACGGAAGCAGTTCGCCGATATCGCCTACAACTACCGGCACGGCCAGCCCATCCCCAGAGTCGAGTACAC
CGAAGAGGGCAAGAAAACCTGGGGCACCGTGTTCAAGACCCTGAAGTCCCTGTACCCCACCCACGCCTGCTACGAGT
ACAACCACATCTTCCCACTGCTCGAAAAGTACTGCGGCTTCCACGAGGACAATATCCCTCAGCTGGAGGACGTGTCC
CAGTTTCTGCAGACCTGCACCGGCTTCAGACTCAGGCCTGTGGCCGGCCTGCTGCCCAGCAGAGATTTTCTGGCCGG
ACTGGCCTTCCGGGTGTTCCACTGCACCCAGTACATCAGACACGGCAGCAAGCCCATGTACACCCCTGAGCCCGACA
TCTGCCACGAGCTGCTGGGACATGTGCCCCTGTTCAGCGACAGAAGCTTCGCCCAGTTCAGCCAGGAAATCGGCCTG
GCCTCTCTGGGCGCTCCCGACGAGTATATCGAGAAGCTGGCCACCATCTACTGGTTCACCGTGGAATTCGGCCTGTG
CAAGCAGGGCGACAGCATCAAGGCCTATGGCGCCGGACTCCTGTCCAGCTTCGGCGAGCTGCAGTACTGTCTGAGCG
AGAAGCCCAAGCTGCTGCCCCTGGAACTGGAAAAGACCGCCATCCAGAACTACACCGTGACCGAGTTCCAGCCCCTG
TACTACGTGGCCGAGAGCTTCAACGACGCCAAAGAAAAAGTGCGGAACTTCGCCGCCACCATCCCTCGGCCCTTCAG
CGTCAGATACGACCCCTACACCCAGCGGATCGAGGTGCTGGACAACACACAGCAGCTGAAAATTCTGGCCGACTCCA
TCAACAGCGAGATCGGCATCCTGTGCAGCGCCCTGCAGAAAATCAAGTGA (SEQ ID NO:4)
3.8 kb optimized vector genome sequence (ITR-A1MB2-mTTR-HI-hPAH V1-BGHpA-ITR) 
GAGCTCTTGGCCACTCCCTCTCTGCGCGCTCGCTCGCTCACTGAGGCCGGGCGACCAAAGGTCGCCCGACGCCCGGG
CTTTGCCCGGGCGGCCTCAGTGAGCGAGCGAGCGCGCAGAGAGGGAGTGGCCAACTCCATCACTAGGGGTTCCTACG
CGTGGCCCCAGGTTAATTTTTAAAAAGCAGTCAAAGGTCAAAGTGGCCCTTGGCAGCATTTACTCTCTCTATTGACT
TTGGTTAATAATCTCAGGAGCACAAACATTCCTGGAGGCAGGAGAAGAAATCAACATCCTGGACTTATCCTCTGGGC
CTCTCCCCACCTTCGATGGCCCCAGGTTAATTTTTAAAAAGCAGTCAAAGGTCAAAGTGGCCCTTGGCAGCATTTAC
TCTCTCTATTGACTTTGGTTAATAATCTCAGGAGCACAAACATTCCTGGAGGCAGGAGAAGAAATCAACATCCTGGA
CTTATCCTCTGGGCCTCTCCCCACCGATATCTACCTGCTGATCGCCCGGCCCCTGTTCAAACATGTCCTAATACTCT
GTCGGGGCAAAGGTCGGCAGTAGTTTTCCATCTTACTCAACATCCTCCCAGTGTACGTAGGATCCTGTCTGTCTGCA
CATTTCGTAGAGCGAGTGTTCCGATACTCTAATCTCCCGGGGCAAAGGTCGTATTGACTTAGGTTACTTATTCTCCT
TTTGTTGACTAAGTCAATAATCAGAATCAGCAGGTTTGGAGTCAGCTTGGCAGGGATCAGCAGCCTGGGTTGGAAGG
AGGGGGTATAAAAGCCCCTTCACCAGGAGAAGCCGTCACACAGATCCACAAGCTCCTGCTAGCAGTCGCTGCGCGCT
GCCTTCGCCCCGTGCCCCGCTCCGCCGCCTCGCGCCGCCCGCCCCGGCTCTGACTGACCGCGTTACTCCCACAG
GTGAGCGGGCGGGACGGCCCTTCTCCTCCGGGCTGTAATTAGCGCTTGGTTTAATGACGGCTTGTTTCTTTTCTGTG
GCTGCGTGAAAGCCTTGAGGGGCTCCGGGAAGGCCCTTTGTGCGGGGGGAGCGGCTCGGGGGGTGCGTGCGTGTGTG
TGTGCGTGGGGAGCGCCGCGTGCGGCTCCGCGCTGCCCGGCGGCTGTGAGCGCTGCGGGCGCGGCGCGGGGCTTTGT
GCGCTCCGCAGTGTGCGCGAGGGGAGCGCGGCCGGGGGCGGTGCCCCGCGGTGCGGGGGGGGCTGCGAGGGGAACAA
AGGCTGCGTGCGGGGTGTGTGCGTGGGGGGGTGAGCAGGGGGTGTGGGCGCGTCGGTCGGGCTGCAACCCCCCCTGC
ACCCCCCTCCCCGAGTTGCTGAGCACGGCCCGGCTTCGGGTGCGGGGCTCCGTACGGGGCGTGGCGCGGGGCTCGCC
GTGCCGGGCGGGGGGTGGCGGCAGGTGGGGGTGCCGGGCGGGGCGGGGCCGCCTCGGGCCGGGGAGGGCTCGGGGGA
GGGGCGCGGCGGCCCCCGGAGCGCCGGCGGCTGTCGAGGCGCGGCGAGCCGCAGCCATTGCCTTTTATGGTAATCGT
GCGAGAGGGCGCAGGGACTTCCTTTGTCCCAAATCTGTGCGGAGCCGAAATCTGGGAGGCGCCGCCGCACCCCCTCT
AGCGGGCGCGGGGCGAAGCGGTGCGGCGCCGGCAGGAAGGAAATGGGCGGGGAGGGCCTTCGTGCGTCGCCGCGCCG
CCGTCCCCTTCTCCCTCTCCAGCCTCGGGGCTGTCCGCGGGGGGACGGCTGCCTTCGGGGGGGACGGGGCAGGGCGG
GGTTCGGCTTCTGGCGTGTGACCGGCGGCTCTAGAGCCTCTGCTAACCATGTTCATGCCTTCTTCTTTTTCCTACAG
CTCCTGGGCAACGTGCTGGTTATTGTGCTGTCTCATCATTTTGGCAAAGAATTCATTTCGAAGCCGCCACCATGAGC
ACAGCCGTGCTGGAAAACCCCGGCCTGGGCAGAAAGCTGAGCGACTTCGGCCAGGAAACCAGCTACATCGAGGACAA
CTGCAACCAGAACGGCGCCATCAGCCTGATCTTCAGCCTGAAAGAAGAAGTGGGCGCCCTGGCCAAGGTGCTGCGGC
TGTTCGAGGAGAACGACGTGAACCTGACCCACATCGAGAGCCGGCCCAGCAGACTGAAGAAGGACGAGTACGAGTTC
TTCACCCACCTGGACAAGCGGAGCCTGCCCGCCCTGACCAACATCATCAAGATCCTGCGGCACGACATCGGCGCCAC
CGTGCACGAGCTGAGCCGGGACAAGAAAAAGGACACCGTGCCCTGGTTCCCCAGAACCATCCAGGAACTGGACAGAT
TCGCCAACCAGATCCTGTCCTACGGCGCCGAGCTGGATGCCGACCACCCTGGCTTCAAGGACCCCGTGTACCGGGCC
AGACGGAAGCAGTTCGCCGATATCGCCTACAACTACCGGCACGGCCAGCCCATCCCCAGAGTCGAGTACACCGAAGA
GGGCAAGAAAACCTGGGGCACCGTGTTCAAGACCCTGAAGTCCCTGTACCCCACCCACGCCTGCTACGAGTACAACC
ACATCTTCCCACTGCTCGAAAAGTACTGCGGCTTCCACGAGGACAATATCCCTCAGCTGGAGGACGTGTCCCAGTTT
CTGCAGACCTGCACCGGCTTCAGACTCAGGCCTGTGGCCGGCCTGCTGCCCAGCAGAGATTTTCTGGCCGGACTGGC
CTTCCGGGTGTTCCACTGCACCCAGTACATCAGACACGGCAGCAAGCCCATGTACACCCCTGAGCCCGACATCTGCC
ACGAGCTGCTGGGACATGTGCCCCTGTTCAGCGACAGAAGCTTCGCCCAGTTCAGCCAGGAAATCGGCCTGGCCTCT
CTGGGCGCTCCCGACGAGTATATCGAGAAGCTGGCCACCATCTACTGGTTCACCGTGGAATTCGGCCTGTGCAAGCA
GGGCGACAGCATCAAGGCCTATGGCGCCGGACTCCTGTCCAGCTTCGGCGAGCTGCAGTACTGTCTGAGCGAGAAGC
CCAAGCTGCTGCCCCTGGAACTGGAAAAGACCGCCATCCAGAACTACACCGTGACCGAGTTCCAGCCCCTGTACTAC
GTGGCCGAGAGCTTCAACGACGCCAAAGAAAAAGTGCGGAACTTCGCCGCCACCATCCCTCGGCCCTTCAGCGTCAG
ATACGACCCCTACACCCAGCGGATCGAGGTGCTGGACAACACACAGCAGCTGAAAATTCTGGCCGACTCCATCAACA
GCGAGATCGGCATCCTGTGCAGCGCCCTGCAGAAAATCAAGTGACCTAGGTGATCAAGATCTGCTAGCTTAATTAAC
CCGGGACTAGTGCGGCCGCTCGAGACTAGTCTGTGCCTTCTAGTTGCCAGCCATCTGTTGTTTGCCCCTCCCCCGTG
CCTTCCTTGACCCTGGAAGGTGCCACTCCCACTGTCCTTTCCTAATAAAATGAGGAAATTGCATCGCATTGTCTGAG
TAGGTGTCATTCTATTCTGGGGGGTGGGGTGGGGCAGGACAGCAAGGGGGAGGATTGGGAAGACAATAGCAGGCATG
CTGGGGATGCGGTGGGCTCTATGGTACCAGGAACCCCTAGTGATGGAGTTGGCCACTCCCTCTCTGCGCGCTCGCTC
GCTCACTGAGGCCGGGCGACCAAAGGTCGCCCGACGCCCGGGCTTTGCCCGGGCGGCCTCAGTGAGCGAGCGAGCGC
GCAGAGAGGGAGTGGCC (SEQ ID NO:5)
4.6 kb optimized vector genome sequence (ITR-A1MB2-mTTR-HI[noATGs]-hPAH V1-BGHpA-stuffer-ITR)(4561 bp) 
TTGGCCACTCCCTCTCTGCGCGCTCGCTCGCTCACTGAGGCCGCCCGGGCAAAGCCCGGGCGTCGGGCGACCTTTGG
TCGCCCGGCCTCAGTGAGCGAGCGAGCGCGCAGAGAGGGAGTGGCCAACTCCATCACTAGGGGTTCCTTACCGCGTG
GCCCCAGGTTAATTTTTAAAAAGCAGTCAAAGGTCAAAGTGGCCCTTGGCAGCATTTACTCTCTCTATTGACTTTGG
TTAATAATCTCAGGAGCACAAACATTCCTGGAGGCAGGAGAAGAAATCAACATCCTGGACTTATCCTCTGGGCCTCT
CCCCACCTTCGATGGCCCCAGGTTAATTTTTAAAAAGCAGTCAAAGGTCAAAGTGGCCCTTGGCAGCATTTACTCTC
TCTATTGACTTTGGTTAATAATCTCAGGAGCACAAACATTCCTGGAGGCAGGAGAAGAAATCAACATCCTGGACTTA
TCCTCTGGGCCTCTCCCCACCGATATCTACCTGCTGATCGCCCGGCCCCTGTTCAAACATGTCCTAATACTCTGTCG
GGGCAAAGGTCGGCAGTAGTTTTCCATCTTACTCAACATCCTCCCAGTGTACGTAGGATCCTGTCTGTCTGCACATT
TCGTAGAGCGAGTGTTCCGATACTCTAATCTCCCGGGGCAAAGGTCGTATTGACTTAGGTTACTTATTCTCCTTTTG
TTGACTAAGTCAATAATCAGAATCAGCAGGTTTGGAGTCAGCTTGGCAGGGATCAGCAGCCTGGGTTGGAAGGAGGG
GGTATAAAAGCCCCTTCACCAGGAGAAGCCGTCACACAGATCCACAAGCTCCTGCTAGCCAATTGAGTCGCTGCGCG
CTGCCTTCGCCCCGTGCCCCGCTCCGCCGCCGCCTCGCGCCGCCCGCCCCGGCTCTGACTGACCGCGTTACTCCCAC
AGGTGAGCGGGCGGGACGGCCCTTCTCCTCCGGGCTGTAATTAGCGCTTGGTTTATTGACGGCTTGTTTCTTTTCTG
TGGCTGCGTGAAAGCCTTGAGGGGCTCCGGGAAGGCCCTTTGTGCGGGGGGAGCGGCTCGGGGGGTGCGTGCGTGTG
TGTGTGCGTGGGGAGCGCCGCGTGCGGCTCCGCGCTGCCCGGCGGCTGTGAGCGCTGCGGGCGCGGCGCGGGGCTTT
GTGCGCTCCGCAGTGTGCGCGAGGGGAGCGCGGCCGGGGGCGGTGCCCCGCGGTGCGGGGGGGGCTGCGAGGGGAAC
AAAGGCTGCGTGCGGGGTGTGTGCGTGGGGGGGTGAGCAGGGGGTGTGGGCGCGTCGGTCGGGCTGCAACCCCCCCT
GCACCCCCCTCCCCGAGTTGCTGAGCACGGCCCGGCTTCGGGTGCGGGGCTCCGTACGGGGCGTGGCGCGGGGCTCG
CCGTGCCGGGCGGGGGGTGGCGGCAGGTGGGGGTGCCGGGCGGGGCGGGGCCGCCTCGGGCCGGGGAGGGCTCGGGG
GAGGGGCGCGGCGGCCCCCGGAGCGCCGGCGGCTGTCGAGGCGCGGCGAGCCGCAGCCATTGCCTTTTTTGGTAATC
GTGCGAGAGGGCGCAGGGACTTCCTTTGTCCCAAATCTGTGCGGAGCCGAAATCTGGGAGGCGCCGCCGCACCCCCT
CTAGCGGGCGCGGGGCGAAGCGGTGCGGCGCCGGCAGGAAGGAATTGGGCGGGGAGGGCCTTCGTGCGTCGCCGCGC
CGCCGTCCCCTTCTCCCTCTCCAGCCTCGGGGCTGTCCGCGGGGGGACGGCTGCCTTCGGGGGGGACGGGGCAGGGC
GGGGTTCGGCTTCTGGCGTGTGACCGGCGGCTCTAGAGCCTCTGCTAACCTTGTTCTTGCCTTCTTCTTTTTCCTAC
AGCTCCTGGGCAACGTGCTGGTTATTGTGCTGTCTCATCATTTTGGCAAAGAATTCATTTCGAAGCCGCCACCATGA
GCACAGCCGTGCTGGAAAACCCCGGCCTGGGCAGAAAGCTGAGCGACTTCGGCCAGGAAACCAGCTACATCGAGGAC
AACTGCAACCAGAACGGCGCCATCAGCCTGATCTTCAGCCTGAAAGAAGAAGTGGGCGCCCTGGCCAAGGTGCTGCG
GCTGTTCGAGGAGAACGACGTGAACCTGACCCACATCGAGAGCCGGCCCAGCAGACTGAAGAAGGACGAGTACGAGT
TCTTCACCCACCTGGACAAGCGGAGCCTGCCCGCCCTGACCAACATCATCAAGATCCTGCGGCACGACATCGGCGCC
ACCGTGCACGAGCTGAGCCGGGACAAGAAAAAGGACACCGTGCCCTGGTTCCCCAGAACCATCCAGGAACTGGACAG
ATTCGCCAACCAGATCCTGTCCTACGGCGCCGAGCTGGATGCCGACCACCCTGGCTTCAAGGACCCCGTGTACCGGG
CCAGACGGAAGCAGTTCGCCGATATCGCCTACAACTACCGGCACGGCCAGCCCATCCCCAGAGTCGAGTACACCGAA
GAGGGCAAGAAAACCTGGGGCACCGTGTTCAAGACCCTGAAGTCCCTGTACCCCACCCACGCCTGCTACGAGTACAA
CCACATCTTCCCACTGCTCGAAAAGTACTGCGGCTTCCACGAGGACAATATCCCTCAGCTGGAGGACGTGTCCCAGT
TTCTGCAGACCTGCACCGGCTTCAGACTCAGGCCTGTGGCCGGCCTGCTGCCCAGCAGAGATTTTCTGGCCGGACTG
GCCTTCCGGGTGTTCCACTGCACCCAGTACATCAGACACGGCAGCAAGCCCATGTACACCCCTGAGCCCGACATCTG
CCACGAGCTGCTGGGACATGTGCCCCTGTTCAGCGACAGAAGCTTCGCCCAGTTCAGCCAGGAAATCGGCCTGGCCT
CTCTGGGCGCTCCCGACGAGTATATCGAGAAGCTGGCCACCATCTACTGGTTCACCGTGGAATTCGGCCTGTGCAAG
CAGGGCGACAGCATCAAGGCCTATGGCGCCGGACTCCTGTCCAGCTTCGGCGAGCTGCAGTACTGTCTGAGCGAGAA
GCCCAAGCTGCTGCCCCTGGAACTGGAAAAGACCGCCATCCAGAACTACACCGTGACCGAGTTCCAGCCCCTGTACT
ACGTGGCCGAGAGCTTCAACGACGCCAAAGAAAAAGTGCGGAACTTCGCCGCCACCATCCCTCGGCCCTTCAGCGTC
AGATACGACCCCTACACCCAGCGGATCGAGGTGCTGGACAACACACAGCAGCTGAAAATTCTGGCCGACTCCATCAA
CAGCGAGATCGGCATCCTGTGCAGCGCCCTGCAGAAAATCAAGTGAACTAGTCTGTGCCTTCTAGTTGCCAGCCATC
TGTTGTTTGCCCCTCCCCCGTGCCTTCCTTGACCCTGGAAGGTGCCACTCCCACTGTCCTTTCCTAATAAAATGAGG
AAATTGCATCGCATTGTCTGAGTAGGTGTCATTCTATTCTGGGGGGTGGGGTGGGGCAGGACAGCAAGGGGGAGGAT
TGGGAAGACAATAGCAGGCATGCTGGGGATGCGGTGGGCTCTATACCGGTCCAGGGGTGAGTGAAGGTTTGGAAGAG
TGTAGCAGAATAAGAAACCATGAGTCCCCTCCCTGAGAAGCCCTGAGCCCCCTTGACGACACACATCCCTCGAGGCT
CAGCTTCATCATCTGTAAAAGGTGCTGAAACTGACCATCCAAGCTGCCGAAAAAGATTGTGTGGGGATAATTCAAAA
CTAGAGGAAGATGCAGAATTTCTACATCGTGGCGATGTCAGGCTAAGAGTTGCCATCGTGGCTGTCCATCGATTTTA
TTGGAATCATATGTTTATTTGAGGGTGTCTTGGATATTACAAATAAATTGTTGGAGCATCAGGCATATTTGGTAATT
CTGTCTAAGGCTCCCTGCCCCTTGTTAATTGGCAGCTCAGTTATTCATCCAGGGCAAACATTCTGCTTACTATTCCT
GAGAGCTTTCCTCATCCTCTAGATTGGCAGGGGAATTGCAGTTGCCTGAGCAGCCTCCCCTCTGCCATACCAACAGA
GCTTCACCATCGAGGCTTGCAGAGTGGACAGGGGCCTCAGGGACCCCTGATCCCAGCTTTCTCATTGGACAGAAGGA
 
GGAGACTGGGGCTGGAGAGGGACCTGGGCCCCCACTAAGGCCACAGCAGAGCCAGGACTTTAGCTGTGCTGACTGCA
GCCTGGCTTGCCTCCACTGCCCTCCTTTGCCTCAAGAGCAAGGGAGCCTCAGAGTGGAGGAAGCAGCCCCTGGCCTT
GCCTCCCACCTCCCCTCCCCTTTGCTGTTTTCCTGGGACAGTGGGAGCTGGCTTAGATTGCCCTGGGGCCCCCAGGA
CCCTGGCATTTTAACCCCTCAGGGGCAGGAAGGCAGCCTGAGATACAGAAGAGTCCATCACCTGCTGTATGCCACAC
ACCATCCCCACAGTCGACATTTAAATTAGGAACCCCTAGTGATGGAGTTGGCCACTCCCTCTCTGCGCGCTCGCTCG
CTCACTGAGGCCGCCCGGGCAAAGCCCGGGCGTCGGGCGACCTTTGGTCGCCCGGCCTCAGTGAGCGAGCGAGCGCG
CAGAGAGGGAGTGGCCAA (SEQ ID NO:6)
5' and 3' sequences used in liver expression cassettes
   Modified PrT2 enhancer sequence 
GCGAGAACTTGTGCCTCCCCGTGTTCCTGACCTTTGACCCTCTGTCCTACTTAGACTAATATTGACTTTGGGTACTG
CAAACAGGAAATGGGGGAGGGATTCGATGCGAGAACTTGTGCCTCCCCGTGTTCCTGACCTTTGACCCTCTGTCCTA
CTTAGACTAATATTGACTTTGGGTACTGCAAACAGGAAATGGGGGAGGGA (SEQ ID NO:7)
 Underlined, hepatic nuclear factor binding sites; bold, modifications introduced to generated higher affinity binding sites, italics, repeat sequence
Modified A1MB2 enhancer 
GGCCCCAGGTTAATTTTTAAAAAGCAGTCAAAGGTCAAAGTGGCCCTTGGCAGCATTTACTCTCTCTATTGACTTTG
GTTAATAATCTCAGGAGCACAAACATTCCTGGAGGCAGGAGAAGAAATCAACATCCTGGACTTATCCTCTGGGCCTC
TCCCCACCTTCGATGGCCCCAGGTTAATTTTTAAAAAGCAGTCAAAGGTCAAAGTGGCCCTTGGCAGCATTTACTCT
CTCTATTGACTTTGGTTAATAATCTCAGGAGCACAAACATTCCTGGAGGCAGGAGAAGAAATCAACATCCTGGACTT
ATCCTCTGGGCCTCTCCCCACC (SEQ ID NO:8)
 Underlined, hepatic nuclear factor binding sites; bold, modifications introduced to generated higher affinity binding sites, italics, repeat sequence
Modified Ealb sequence 
GTTCCTAGATTACATTACACATTCTGCAAGCATAGCACAGGTCAAAGTTCAACTTTAATTACTTTCATTTTCTTGTA
TCCTCACAGCCTAGAAAATAACCTGCGTTACAGCATCCACTCAGTATCCCTTGAGCATGAGGTGACACTACTTAACA
TAGGGACGAGATGGTACTTTGTGTCTCCTGCTCTGTCAGCAGGGCACTGTACTTGCTGATACCAGGGAATATTGATT
TGTAAATACCATCATTCCGAACGTGTTTGCCTTGGCCAGTTTTCCATGTACATGCAGAAAGAAGTTTGGGACTGATC
AATACAGTCCTCTGCCTTTAAAGCAATAGGAAAAGGCCAACTTGTCTACGTTTAGTATGTGGCTGTAGA (SEQ ID
NO:9)
 Underlined, hepatic nuclear factor binding sites; bold, modifications introduced to generated higher affinity binding sites, italics, repeat sequence
HEII enhancer 
CCATCAGATCCTGCCCAAGGTCTTACATAAGAGGACTCTTGGACTCCCAGCAATGTCAACGACCGACCTTGAGGCCT
ACTTCAAAGACTGTGTGTTTAAGGACTGGGAGGAGCTGGGGGAGGAGATTAGGTTAAAGGTCTTTGTATTAGGAGGC
TG (SEQ ID NO:10)
CRM8 enhancer
   GGGGAGGCTGCTGGTGAATATTAACCAAGGTCACCCCAGTTATCGGAGGAGCAAACAGGGGCTAAGTCCAC (SEQ ID NO:11)
3'Alb stability element 
CTCAATTGGATGACACTAGTCATCACATTTAAAAGCATCTCAGGTAACTATATTTTGAATTTTTTAAAAAAGTAACT
ATAATAGTTATTATTAAAATAGCAAAGATTGACCATTTCCAAGAGCCATATAGACCAGCACCGACCACTATTCTAAA
CTATTTATGTATGTAAATATTAGCTTTTAAAATTCTCAAAATAGTTGCTGAGTTGGGAACCACTATTATTTCTATCG
ATTCAGCAGCCGTAAGTCTAGGACAGGCTTAAATTGTTTTCACTGGTGTAAATTGCAGAAAGATGATCTAAGTAATT
TGGCATTTATTTTAATAGGTTTGAAAAACACATGCCATTTTACAAATAAGACTTATATTTGTCCTTTTGTTTTTCAG
CCTACCATGAGAATAAGAGAAAGAAAATGAAGATCAAAAGCTTATTCATCTGTTTTTCTTTTTCGTTGGTGTAAAGC
CAACACCCTGTCTAAAAAACATAAATTTCTTTAATCATTTTGCCTCTTTTCTCTGTGCTTCAATTAATAAAAAATGG
AAAGAATCTAATAGAGTGGTACAGCACTGTTATTTTTCAAAGATGTGTTGCTATCCTGAAAATTCTGTAGGTTCTGT
GGAAGTTCCAGTGTTCTCTCTTATTCCACTTCGGTAGAGGATTTCTAGTTTCTTGTGGGCTAATTAAATAAATCATT
AATACTCTTCTAAGTTATGGATTATAAACATTCAAAATAATATTTTGACATTATGATAATTCTGAATAAAAGAACAA
AAACCATGGTATAGGTAAGGAATATAAAACATGGCTTTTACCTTAGAAAAAACAATTCTAAAATTCATATGGAATCA
AAAAAGAGCCTGCAGGTACCCT (SEQ ID NO:12)
3'alb and SMAR stability element 
CTCAATTGGATGACACTAGTCATCACATTTAAAAGCATCTCAGGTAACTATATTTTGAATTTTTTAAAAAAGTAACT
ATAATAGTTATTATTAAAATAGCAAAGATTGACCATTTCCAAGAGCCATATAGACCAGCACCGACCACTATTCTAAA
CTATTTATGTATGTAAATATTAGCTTTTAAAATTCTCAAAATAGTTGCTGAGTTGGGAACCACTATTATTTCTATCT
ACTGTTTTAATTAAAATTATCTCTAAGGCATGTGAACTGGCTGTCTTGGTTTTCATCTGTACTTCATCTGCTACCTC
TGTGACCTGAAACATATTTATAATTCCATTAAGCTGTGCATATGATAGATTTATCATATGTATTTTCCTTAAAGGAT
TTTTGTAAGAACTAATTGAATTGATACCTGTAAAGTCTTTATCACACTACCCAATAAATAATAAATCTCTTTGTTCA
GCTCTCTGTTTCTATAAATATGTACCAGTTTTATTGTTTTTAGTGGTAGTGATTTTATTCTCTTTCTATATATATAC
ACACACATGTGTGCATTCATAAATATATACAATTTTTATGAATAAAAAATTATTAGCAATCAATATTGAAAACCACT
GATTTTTGTTTATGTGAGCAAACAGCAGATTAAAAGGAATTCCTGCAGATTCAGCAGCCGTAAGTCTAGGACAGGCT
TAAATTGTTTTCACTGGTGTAAATTGCAGAAAGATGATCTAAGTAATTTGGCATTTATTTTAATAGGTTTGAAAAAC
ACATGCCATTTTACAAATAAGACTTATATTTGTCCTTTTGTTTTTCAGCCTACCATGAGAATAAGAGAAAGAAAATG
AAGATCAAAAGCTTATTCATCTGTTTTTCTTTTTCGTTGGTGTAAAGCCAACACCCTGTCTAAAAAACATAAATTTC
TTTAATCATTTTGCCTCTTTTCTCTGTGCTTCAATTAATAAAAAATGGAAAGAATCTAATAGAGTGGTACAGCACTG
TTATTTTTCAAAGATGTGTTGCTATCCTGAAAATTCTGTAGGTTCTGTGGAAGTTCCAGTGTTCTCTCTTATTCCAC
TTCGGTAGAGGATTTCTAGTTTCTTGTGGGCTAATTAAATAAATCATTAATACTCTTCTAAGTTATGGATTATAAAC
ATTCAAAATAATATTTTGACATTATGATAATTCTGAATAAAAGAACAAAAACCATGGTATAGGTAAGGAATATAAAA
CATGGCTTTTACCTTAGAAAAAACAATTCTAAAATTCATATGGAATCAAAAAAGAGCCTGCAGGTACCCT(SEQ ID
NO:13)
Codon-optimized human PAH cDNA 
ATGAGCACAGCCGTGCTGGAAAACCCCGGCCTGGGCAGAAAGCTGAGCGACTTCGGCCAGGAAACCAGCTACATCGA
GGACAACTGCAACCAGAACGGCGCCATCAGCCTGATCTTCAGCCTGAAAGAAGAAGTGGGCGCCCTGGCCAAGGTGC
TGCGGCTGTTCGAAGAGAACGACGTGAACCTGACCCACATCGAGAGCCGGCCCAGCAGACTGAAGAAGGACGAGTAC
GAGTTCTTCACCCACCTGGACAAGCGGAGCCTGCCCGCCCTGACCAACATCATCAAGATCCTGCGGCACGACATCGG
CGCCACCGTGCACGAGCTGAGCCGGGACAAGAAAAAGGACACCGTGCCCTGGTTCCCCAGAACCATCCAGGAACTGG
ACAGATTCGCCAACCAGATCCTGTCCTACGGCGCCGAGCTGGATGCCGACCACCCTGGCTTCAAGGACCCCGTGTAC
CGGGCCAGACGGAAGCAGTTCGCCGATATCGCCTACAACTACCGGCACGGCCAGCCCATCCCCAGAGTCGAGTACAT
GGAAGAGGGCAAGAAAACCTGGGGCACCGTGTTCAAGACCCTGAAGTCCCTGTACAAGACCCACGCCTGCTACGAGT
ACAACCACATCTTCCCACTGCTCGAGAAGTACTGCGGCTTCCACGAGGACAATATCCCTCAGCTCGAGGACGTGTCC
CAGTTTCTGCAGACCTGCACCGGCTTCAGACTCAGGCCTGTGGCCGGCCTGCTGAGCAGCAGAGATTTTCTGGGCGG
ACTGGCCTTCCGGGTGTTCCACTGCACCCAGTACATCAGACACGGCAGCAAGCCCATGTACACCCCTGAGCCCGACA
TCTGCCACGAGCTGCTGGGACATGTGCCCCTGTTCAGCGACAGAAGCTTCGCCCAGTTCAGCCAGGAAATCGGCCTG
GCCTCTCTGGGCGCTCCCGACGAGTATATCGAGAAGCTGGCCACCATCTACTGGTTCACCGTGGAATTCGGCCTGTG
 
CAAGCAGGGCGACAGCATCAAGGCCTATGGCGCCGGACTCCTGTCCAGCTTCGGCGAGCTGCAGTACTGTCTGAGCG
AGAAGCCCAAGCTGCTGCCCCTGGAACTGGAAAAGACCGCCATCCAGAACTACACCGTGACCGAGTTCCAGCCCCTG
TACTACGTGGCCGAGAGCTTCAACGACGCCAAAGAAAAAGTGCGGAACTTCGCCGCCACCATCCCTCGGCCCTTCAG
CGTCAGATACGACCCCTACACCCAGCGGATCGAGGTGCTGGACAACACACAGCAGCTGAAAATTCTGGCCGACTCCA
TCAACAGCGAGATCGGCATCCTGTGCAGCGCCCTGCAGAAAATCAAGTGA (SEQ ID NO:14)
Modified a chicken β-actin (CBA)/rabbit β-globin hybrid promoter/intron 
AGTCGCTGCGCGCTGCCTTCGCCCCGTGCCCCGCTCCGCCGCCGCCTCGCGCCGCCCGCCCCGGCTCTGACTGACCG
CGTTACTCCCACAGGTGAGCGGGCGGGACGGCCCTTCTCCTCCGGGCTGTAATTAGCGCTTGGTTTATTGACGGCTT
GTTTCTTTTCTGTGGCTGCGTGAAAGCCTTGAGGGGCTCCGGGAAGGCCCTTTGTGCGGGGGGAGCGGCTCGGGGGG
TGCGTGCGTGTGTGTGTGCGTGGGGAGCGCCGCGTGCGGCTCCGCGCTGCCCGGCGGCTGTGAGCGCTGCGGGCGCG
GCGCGGGGCTTTGTGCGCTCCGCAGTGTGCGCGAGGGGAGCGCGGCCGGGGGCGGTGCCCCGCGGTGCGGGGGGGGC
TGCGAGGGGAACAAAGGCTGCGTGCGGGGTGTGTGCGTGGGGGGGTGAGCAGGGGGTGTGGGCGCGTCGGTCGGGCT
GCAACCCCCCCTGCACCCCCCTCCCCGAGTTGCTGAGCACGGCCCGGCTTCGGGTGCGGGGCTCCGTACGGGGCGTG
GCGCGGGGCTCGCCGTGCCGGGCGGGGGGTGGCGGCAGGTGGGGGTGCCGGGCGGGGCGGGGCCGCCTCGGGCCGGG
GAGGGCTCGGGGGAGGGGCGCGGCGGCCCCCGGAGCGCCGGCGGCTGTCGAGGCGCGGCGAGCCGCAGCCATTGCCT
TTTTTGGTAATCGTGCGAGAGGGCGCAGGGACTTCCTTTGTCCCAAATCTGTGCGGAGCCGAAATCTGGGAGGCGCC
GCCGCACCCCCTCTAGCGGGCGCGGGGCGAAGCGGTGCGGCGCCGGCAGGAAGGAATTGGGCGGGGAGGGCCTTCGT
GCGTCGCCGCGCCGCCGTCCCCTTCTCCCTCTCCAGCCTCGGGGCTGTCCGCGGGGGGACGGCTGCCTTCGGGGGGG
ACGGGGCAGGGCGGGGTTCGGCTTCTGGCGTGTGACCGGCGGCTCTAGAGCCTCTGCTAACCTTGTTCTTGCCTTCT
TCTTTTTCCTACAGCTCCTGGGCAACGTGCTGGTTATTGTGCTGTCTCATCATTTTGGCAAAGAATTC (SEQ ID
NO:15)
Bold = G, A, A, A, A changed to T to eliminate ATG (5 changes)
0.9 kb A1AT intron stuffer sequence 
CCAGGGGTGAGTGAAGGTTTGGAAGAGTGTAGCAGAATAAGAAACCATGAGTCCCCTCCCTGAGAAGCCCTGAGCCC
CCTTGACGACACACATCCCTCGAGGCTCAGCTTCATCATCTGTAAAAGGTGCTGAAACTGACCATCCAAGCTGCCGA
AAAAGATTGTGTGGGGATAATTCAAAACTAGAGGAAGATGCAGAATTTCTACATCGTGGCGATGTCAGGCTAAGAGT
TGCCATCGTGGCTGTCCATCGATTTTATTGGAATCATATGTTTATTTGAGGGTGTCTTGGATATTACAAATAAATTG
TTGGAGCATCAGGCATATTTGGTAATTCTGTCTAAGGCTCCCTGCCCCTTGTTAATTGGCAGCTCAGTTATTCATCC
AGGGCAAACATTCTGCTTACTATTCCTGAGAGCTTTCCTCATCCTCTAGATTGGCAGGGGAATTGCAGTTGCCTGAG
CAGCCTCCCCTCTGCCATACCAACAGAGCTTCACCATCGAGGCTTGCAGAGTGGACAGGGGCCTCAGGGACCCCTGA
TCCCAGCTTTCTCATTGGACAGAAGGAGGAGACTGGGGCTGGAGAGGGACCTGGGCCCCCACTAAGGCCACAGCAGA
GCCAGGACTTTAGCTGTGCTGACTGCAGCCTGGCTTGCCTCCACTGCCCTCCTTTGCCTCAAGAGCAAGGGAGCCTC
AGAGTGGAGGAAGCAGCCCCTGGCCTTGCCTCCCACCTCCCCTCCCCTTTGCTGTTTTCCTGGGACAGTGGGAGCTG
GCTTAGATTGCCCTGGGGCCCCCAGGACCCTGGCATTTTAACCCCTCAGGGGCAGGAAGGCAGCCTGAGATACAGAA
GAGTCCATCACCTGCTGTATGCCACACACCATCCCCACAGTCGACATTTAAATT (SEQ ID NO:16)
Bold= 7 bases changed to eliminate ATGs; all A to T changes.

Further embodiments are set out in the following numbered items:
1. A variant phenylalanine hydroxylase (PAH) polypeptide comprising two amino acid substitutions, wherein the amino acid substitutions are at sites selected from M180, K199, S250, and G256 of a wild-type human PAH polypeptide.
2. A variant phenylalanine hydroxylase (PAH) polypeptide comprising three amino acid substitutions, wherein the amino acid substitutions are at sites selected from M180, K199, S250, and G256 of a wild-type human PAH polypeptide.
3. A variant phenylalanine hydroxylase (PAH) polypeptide comprising four amino acid substitutions at sites M180, K199, S250, and G256 of a wild-type human PAH polypeptide.
4. The variant PAH polypeptide of any one of items 1-3, wherein the amino acid substitution comprises one of more of M180T, K199P, S250P, and G256A.
5. The variant PAH polypeptide of any one of items 1-4, wherein the amino acid substitution comprises K199P, S250P, and G256A; M180T, S250P, and G256A; M180T, K199P, and G256A; or M180T, K199P, and S250P.
6. The variant PAH polypeptide of any one of items 1-5, wherein the amino acid substitution comprises M180T, K199P, S250P, and G256A.
7. The variant PAH polypeptide of any one of items 1-6, wherein the variant PAH polypeptide further comprises H264P, G272A, G272P, P275L, P279Q, G272P and P275L, or T323R and F327T amino acid substitutions.
8. The variant PAH polypeptide of any one of items 1-7, wherein the wild-type human PAH polypeptide comprises the amino acid sequence of SEQ ID NO: 1.
9. The variant PAH polypeptide of any one of items 1-8, wherein the variant PAH polypeptide is a human PAH polypeptide.
10. The variant PAH polypeptide of any one of items 1-9, wherein the variant PAH polypeptide comprises an amino acid sequences that is at least about 80% identical to the amino acid sequence of SEQ ID NO:3.
11. The variant PAH polypeptide of any one of items 1-6, wherein the variant PAH polypeptide comprises the amino acid sequence of SEQ ID NO:3.
12. The variant PAH of any one of items 1-11, wherein the variant PAH polypeptide further comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide.
13. The variant PAH of any one of items 1-12, wherein the variant PAH polypeptide further comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide.
14. The variant PAH of any one of items 1-13, wherein the variant PAH polypeptide further comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide.
15. A variant PAH polypeptide, wherein the variant PAH polypeptide comprises one or more amino acid substitutions selected from G33A, G46A, G46P, G103A, G139A, G139P, G148A, G188A, G218A, G239A, G247A, G257A, G272A, G289A, G307A, G312A, G332A, G337A, G344A, G352A, and G442A of a wild-type human PAH polypeptide.
16. A variant PAH polypeptide, wherein the variant PAH polypeptide comprises one or more amino acid substitutions selected from P9G, G10V, G12S, K184R, K192R, S196A, Y206H, H220R, Q336E, E360D, I374C, N376E, N401T, I421V, I441V, S446H, and the addition of S at position 453 of a wild-type human PAH polypeptide.
17. A variant PAH polypeptide, wherein the variant PAH polypeptide comprises one or more amino acid substitutions selected from F240W, A246P, G247A, Y268W, C284F, T323R, F327Y, E319P, I306(Y,F), K113P, G188A, F191Y, T193R, Y206H, G337P, and N376P of a wild-type human PAH polypeptide.
18. The variant PAH polypeptide of any one of items 1-17, wherein the variant PAH polypeptide comprises an N-terminal truncation.
19. The variant PAH polypeptide of item 8, wherein the N-terminal truncation comprises a truncation of the N-terminal regulatory domain.
20. The variant PAH polypeptide of items 18 or 19, wherein the N-terminal truncation comprises a truncation of amino acid residues 1-102 of the wild-type PAH polypeptide.
21. The variant PAH polypeptide of any one of items 1-20, wherein the variant PAH polypeptide comprises a C-terminal truncation.
22. The variant PAH polypeptide of item 21, wherein the C-terminal truncation comprises a truncation of the tetramerization domain.
23. The variant PAH polypeptide of items 21 or 22, wherein the C-terminal truncation comprises a truncation of amino acid residues 429-452 of the wild-type PAH polypeptide.
24. The variant PAH polypeptide of any one of items 1-23, wherein the variant PAH polypeptide comprises an amino acid sequence corresponding to amino acid residues 103-428 of the wild-type PAH polypeptide.
25. The variant PAH polypeptide of any one of items 1-24, wherein the variant PAH polypeptide comprises one or more amino acid substitutions to eliminate potential protease cleavage sites.
26. The variant PAH polypeptide of item 25, wherein the one or more amino acid substitutions to eliminate potential protease cleavage sites is located at positions 270-295 and/or 380-405 of the wild type PAH polypeptide.
27. The variant PAH polypeptide of any one of items 1-26, wherein the variant PAH polypeptide is fused to a liver targeting polypeptide.
28. The variant PAH polypeptide of item 27, wherein the liver targeting polypeptide is a HGF or fragments thereof or glycoproteins that bind to hepatocyte asialoglycoprotein receptor.
29. The variant PAH polypeptide of any one of items 1-25, wherein the variant PAH polypeptide is PEGylated and/or nitrosylated.
30. The variant PAH polypeptide of item 26, wherein the variant PAH polypeptide comprises a I374C amino acid substitution, wherein the cys residue at position 374 is nitrosylated.
31. A composition comprising the variant PAH polypeptide of any one of items 1-30.
32. The composition of item 31, wherein the composition further comprises a pharmaceutically acceptable carrier.
33. An isolated nucleic acid encoding the variant PAH polypeptide of any one of items 1-30.
34. The isolated nucleic acid of item 33, wherein the nucleic acid encoding the variant PAH polypeptide is operably linked to a promoter.
35. The isolated nucleic acid of item 34, wherein the promoter is selected from a cytomegalovirus (CMV) immediate early promoter, an RSV LTR, a MoMLV LTR, a phosphoglycerate kinase- 1 (PGK) promoter, a simian virus 40 (SV40) promoter, a CK6 promoter, a transthyretin promoter (TTR), a mTTR482 promoter, a mA1MB2-mTTR482 promoter, a TK promoter, a tetracycline responsive promoter (TRE), an HBV promoter, an hAAT promoter, a LSP promoter, an LP1 promoter, a chimeric liver-specific promoter (LSP), an E2F promoter, a telomerase (hTERT) promoter; a cytomegalovirus enhancer/chicken beta-actin/Rabbit β-globin promoter (CAG) promoter, an elongation factor 1-alpha promoter (EFl-alpha) promoter, a human β-glucuronidase promoter, a chicken β-actin (CBA) promoter, a modified chicken β-actin (CBA) promoter or SEQ ID NO: 17, a retroviral Rous sarcoma virus (RSV) LTR promoter, a dihydrofolate reductase promoter, and a 13-actin promoter.
36. The isolated nucleic acid of item 34 or 35, wherein the promoter is an LP1 promoter or an mA1MB2-mTTR482 promoter.
37. The isolated nucleic acid of any one of items 33-36, wherein the nucleic acid further comprises a polyadenylation signal.
38. The isolated nucleic acid of item 37, wherein the polyadenylation signal is a bovine growth hormone polyadenylation signal, an SV40 polyadenylation signal, or a HSV TK pA.
39. The isolated nucleic acid of any one of items 33-38, wherein the nucleic acid further comprises an intron.
40. The isolated nucleic acid of item 39, wherein the intron is a chicken β-actin (CBA)/rabbit β-globin hybrid intron.
41. The isolated nucleic acid of item 39, wherein the intron is a modified chicken β-actin (CBA)/rabbit β-globin hybrid intron of SEQ ID NO: 15.
42. The isolated nucleic acid of any one of items 33-41, wherein the nucleic acid further comprises one or more ITRs.
43. The isolated nucleic acid of any one of items 33-42, wherein the nucleic acid further comprises a stuffer nucleic acid.
44. The isolated nucleic acid of item 43, wherein the stuffer nucleic acid is optimized to remove ATG sequences.
45. The isolated nucleic acid of item 44, wherein the stuffer nucleic acid is an A1AT intron stuffer sequence of SEQ ID NO: 16.
46. An isolated nucleic acid encoding a human PAH polypeptide, wherein the nucleic acid is codon-optimized.
47. The isolated nucleic acid of item 46, wherein the nucleic acid sequence is at least 80% identical to the nucleic acid sequence of SEQ ID NO: 14.
48. The isolated nucleic acid of item 46, wherein the nucleic acid comprises the nucleic acid sequence of SEQ ID NO: 14.
49. The isolated nucleic acid of item 33, wherein the nucleic acid is an mRNA.
50. A composition comprising the nucleic acid of any one of items 33-49.
51. The composition of item 50, wherein the composition further comprises a pharmaceutically acceptable carrier.
52. A vector comprising the nucleic acid of any one of items 33-49.
53. The vector of item 52, wherein the vector is a recombinant adeno-associated virus (rAAV) vector.
54. An rAAV vector comprising the nucleic acid of any one of items 33-41 or 43-49 flanked by one or more AAV inverted terminal repeat (ITR) sequences.
55. The rAAV vector of item 54, wherein the nucleic acid of any one of items 33-48 is flanked by two AAV ITRs.
56. The rAAV vector of item 54 or 55, wherein the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs.
57. The rAAV vector of any one of items 54-56, wherein the AAV ITRs are AAV2 ITRs.
58. The rAAV vector of item 57, wherein the rAAV vector comprises 5' to 3' an AAV2 ITR, a promoter, an intron, nucleic acid encoding a PAH polypeptide, a stuffer nucleic acid, a polyadenylation signal, and an AAV2 ITR.
59. The rAAV vector of item 58, wherein the promoter is a m1A1MB2-mTTR482 promoter or an LP1 promoter.
60. The rAAV vector of item 58 or 59, wherein the intron is a chicken β-actin (CBA)/rabbit β-globin hybrid intron.
61. The rAAV vector of any one of items 58-59, wherein the PAH polypeptide is the variant PAH polypeptide of any one of items 1-30.
62. The rAAV vector of any one of items 58-60, wherein the nucleic acid encoding the PAH polypeptide is the codon optimized nucleic acid of any one of items 46-48.
63. The rAAV vector of any one of items 58-62, wherein the stuffer nucleic acid comprises nucleic acid from an intron of the human alpha 1 antitrypsin gene.
64. The rAAV vector of item 63, wherein the intron of the human alpha 1 antitrypsin gene has been mutated to remove ATG sequences.
65. The rAAV vector of any one of items 58-64, wherein the polyadenylation signal is a bovine growth hormone polyadenylation signal.
66. The rAAV vector of any one of items 53-65, wherein the vector is a self-complimenting vector.
67. The rAAV vector of item 66, wherein the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length.
68. The rAAV vector of item 57, wherein the first nucleic acid sequence and the second nucleic acid sequence are linked by a mutated AAV ITR, wherein the mutated AAV ITR comprises a deletion of the D region and comprises a mutation of the terminal resolution sequence.
69. A rAAV particle comprising the rAAV vector of any one of items 53-68.
70. The rAAV particle of item 69, wherein the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid.
71. The rAAV particle of item 69, wherein the AAV viral particle comprises an engineered AAV capsid.
72. The rAAV particle of item 71, wherein the engineered AAV capsid is a DJ capsid or an LK03 capsid.
73. The rAAV particle of item 69 or 70, wherein the ITR and the capsid of the rAAV viral particle are derived from the same AAV serotype.
74. The rAAV particle of item 69 or 70, wherein the ITR and the capsid of the rAAV viral particles are derived from different AAV serotypes.
75. The rAAV particle of any one of items 69-70 or 73-74, wherein the rAAV viral particle comprises an AAV8 capsid.
76. The rAAV particle of item 74, wherein the rAAV viral particle comprises an AAV8 capsid, and wherein the vector comprises AAV2 ITRs.
77. A composition comprising the rAAV particle of any one of items 69-76.
78. The composition of item 77, wherein the composition further comprises a pharmaceutically acceptable carrier.
79. A cell comprising the nucleic acid of any one of items 33-49 or the vector of items 52 or 53 or the rAAV vector of any one of items 54-68.
80. A method of producing a variant PAH polypeptide, the method comprising culturing the cell of item 79 under conditions to produce the variant PAH polypeptide.
81. The methods of item 80, further comprising the step of purifying the variant PAH polypeptide.
82. A method for treating phenylketonuria in an individual in need thereof, comprising administering to the individual the variant PAH polypeptide of any one of items 1-30 or the composition of item 31 or 32.
83. A method for treating phenylketonuria in an individual in need thereof, comprising administering to the individual a nucleic acid encoding the variant PAH polypeptide of any one of items 1-30 or the nucleic acid of item 33, 34 or 49.
84. A method for treating phenylketonuria in an individual in need thereof, comprising administering to the individual the rAAV vector of any one of items 53-68.
85. A method for treating phenylketonuria in an individual in need thereof, comprising administering to the individual the rAAV particle of any one of items 69-76.
86. A method for treating phenylketonuria in an individual in need thereof, comprising administering to the individual the composition of item 31, 32, 50, 51, 77 or 78.
87. A method for treating phenylketonuria in an individual in need thereof, comprising administering to the individual the cell of item 79.
88. The method of any one of items 82-87, wherein the individual lacks PAH activity.
89. A method for reducing the level of phenylalanine in the blood of in an individual in need thereof, comprising administering to the individual the variant PAH polypeptide of any one of items 1-30 or the composition of item 31 or 32.
90. A method for reducing the level of phenylalanine in the blood of in an individual in need thereof, comprising administering to the individual nucleic acid encoding the variant PAH polypeptide of any one of items 1-30 or the nucleic acid of item 33, 34 or 49.
91. A method for reducing the level of phenylalanine in the blood of in an individual in need thereof, comprising administering to the individual the rAAV vector of any one of items 53-68.
92. A method for reducing the level of phenylalanine in the blood of in an individual in need thereof, comprising administering to the individual the rAAV particle of any one of items 69-76.
93. A method for reducing the level of phenylalanine in the blood of an individual in need thereof, comprising administering to the individual the composition of item 31, 32, 50, 51, 77 or 78.
94. A method for reducing the level of phenylalanine in the blood of an individual in need thereof, comprising administering to the individual the cell of item 79.
95. The method of any one of items 89-94, wherein the level of phenylalanine in the blood of the individual prior to treatment is elevated compared to the level of phenylalanine in the blood of peer-matched control individuals.
96. The method of any one of items 82-95, wherein the variant PAH polypeptide, the nucleic acid, rAAV vector, rAAV particle, composition or cell is administered intravenously, intraarterially, intrahepatically, intraportally, intraperitoneally, or subcutaneously.
97. The method of any one of items 82-96, wherein the administration is in combination with another therapy.
98. The method of item 97, wherein the another therapy is treatment with tetrahydribiopterin, treatment with phenylalanine ammonia lyase (PAL) or pegylated PAL, or a phenylalanine-restricted diet.
99. A method for making a PAH polypeptide comprising culturing the cell of item 79 under conditions produce the PAH polypeptide.
100. The method or item 99, further comprising purifying the PAH polypeptide.
101. A kit comprising the variant PAH polypeptide of any one of items 1-24.
102. A kit comprising the nucleic acid of any one of items 33-46, the rAAV vector of any one of items 53-68, the rAAV particle of any one of items 69-76, or the composition of item 77 or 78.
103. The kit of item 101 or 102, wherein the kit further comprises instructions for use; buffers and/or pharmaceutically acceptable excipients; and/or bottles, vials and/or syringes.
104. An expression cassette for expressing a transgene in a liver cell, wherein the expression cassette comprises a transgene operably linked to a promoter and enhancer, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer.
105. The expression cassette of item 104, wherein the mTTR promoter is a mTTR482 promoter.
106. The expression cassette of item 104 or 105, wherein the enhancer is 5' to the mTTR promoter.
107. An expression cassette for expressing a transgene in a liver cell, wherein the expression cassette comprises a transgene operably linked to a promoter and a 3' element, wherein the promoter comprises a mouse transthyretin (mTTR) promoter and the 3' element is an albumin 3' element (3'Alb) or an albumin 3' element linked to a human alpha 1 antitrypsin scaffold/matrix attachment region (SMAR) (3'AlbSMAR).
108. The expression cassette of item 107, wherein the mTTR promoter is a mTTR482 promoter.
109. The expression cassette of item 107 or 108, wherein the 3' element is located 3' to the transgene.
110. An expression cassette for expressing a transgene in a liver cell, wherein the expression cassette comprises a transgene operably linked to a promoter and enhancer and a 3' element, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer; and wherein the 3' element is an albumin 3' element (3'Alb) or an albumin 3' element linked to a human alpha 1 antitrypsin scaffold/matrix attachment region (SMAR) (3'AlbSMAR).
111. The expression cassette of item 110, wherein the mTTR promoter is a mTTR482 promoter.
112. The expression cassette of item 110 or 111, wherein the enhancer is 5' to the mTTR promoter.
113. The expression cassette of any one of items 110-112, wherein the 3' element is located 3' to the transgene.
114. The expression cassette of any one of items 104-113, wherein the expression cassette further comprises an intron.
115. The expression cassette of item 114, wherein the intron is a chicken β-actin/rabbit β-globin hybrid intron.
116. The expression cassette of any one of items 104-115, wherein the expression cassette further comprises a polyadenylation signal.
117. The expression cassette of item 116, wherein the polyadenylation signal is a bovine growth hormone polyadenylation signal.
118. The expression cassette of any one of items 104-117, wherein the transgene encodes a PAH polypeptide or a variant PAH polypeptide.
119. The expression cassette of item 118, wherein the variant PAH polypeptide is the variant PAH polypeptide of any one of items 1-30.
120. A vector comprising the expression cassette of any one of items 104-119.
121. The vector of item 120, wherein the vector is a recombinant adeno-associated virus (rAAV) vector.
122. An rAAV vector comprising the expression cassette of any one of items 104-119 is flanked by one or more AAV inverted terminal repeat (ITR) sequences.
123. The rAAV vector of item 122, wherein the expression cassette of any one of items 104-119 is flanked by two AAV ITRs.
124. The rAAV vector of item 122 or 123, wherein the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs.
125. The rAAV vector of any one of items 122-124, wherein the AAV ITRs are AAV2 ITRs.
126. The rAAV vector of any one of items 122-125, wherein the vector is a self-complimenting vector.
127. The rAAV vector of item 126, wherein the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length.
128. The rAAV vector of item 127, wherein the first nucleic acid sequence and the second nucleic acid sequence are linked by a mutated AAV ITR, wherein the mutated AAV ITR comprises a deletion of the D region and comprises a mutation of the terminal resolution sequence.
129. A rAAV particle comprising the rAAV vector of any one of items 122-127.
130. The rAAV particle of item 128, wherein the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid.
131. The rAAV particle of item 130, wherein the AAV viral particle comprises an engineered AAV capsid.
132. The rAAV particle of item 131, wherein the engineered AAV capsid is a DJ capsid or an LK03 capsid.
133. The rAAV particle of item 131 or 132, wherein the ITR and the capsid of the rAAV viral particle are derived from the same AAV serotype.
134. The rAAV particle of item 131 or 132, wherein the ITR and the capsid of the rAAV viral particles are derived from different AAV serotypes.
135. A composition comprising the rAAV particle of any one of items 129-134.
136. The composition of item 135, wherein the composition further comprises a pharmaceutically acceptable carrier.

## Claims

1. An expression cassette for expressing a transgene in a liver cell, wherein the expression cassette comprises a transgene operably linked to a promoter and enhancer, wherein the promoter comprises a mouse transthyretin (mTTR) promoter and the enhancer comprises one or two modified prothrombin enhancers (pPrT2), one or two modified alpha1-microbikunin enhancers (mA1MB2), a modified mouse albumin enhancer (mEalb), a hepatitis B virus enhancer II (HE11) or a CRM8 enhancer.

2. The expression cassette of claim 1, wherein the mTTR promoter is a mTTR482 promoter.

3. The expression cassette of claim 1 or 2, wherein the enhancer is 5' to the mTTR promoter.

4. The expression cassette of any one of claims 1-3, wherein the expression cassette further comprises an intron, optionally wherein the intron is a chicken β-actin/rabbit β-globin hybrid intron.

5. The expression cassette of any one of claims 1-4, wherein the expression cassette further comprises a polyadenylation signal, optionally wherein the polyadenylation signal is a bovine growth hormone polyadenylation signal.

6. The expression cassette of any one of claims 1-5, wherein the transgene encodes a PAH polypeptide.

7. A vector comprising the expression cassette of any one of claims 1-6, optionally wherein the vector is a recombinant adeno-associated virus (rAAV) vector.

8. An rAAV vector comprising the expression cassette of any one of claims 1-7 flanked by one or more AAV inverted terminal repeat (ITR) sequences, optionally wherein the expression cassette is flanked by two AAV ITRs.

9. The rAAV vector of claim 8, wherein the AAV ITRs are AAV ITRs are AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV DJ, a goat AAV, bovine AAV, or mouse AAV serotype ITRs, preferably wherein the AAV ITRs are AAV2 ITRs.

10. The rAAV vector of claim 8 or 9, wherein the vector is a self-complimenting vector.

11. The rAAV vector of claim 10, wherein the vector comprises first nucleic acid sequence encoding the PAH polypeptide and a second nucleic acid sequence encoding a complement of the PAH polypeptide, wherein the first nucleic acid sequence can form intrastrand base pairs with the second nucleic acid sequence along most or all of its length, optionally wherein the first nucleic acid sequence and the second nucleic acid sequence are linked by a mutated AAV ITR, wherein the mutated AAV ITR comprises a deletion of the D region and comprises a mutation of the terminal resolution sequence.

12. A rAAV particle comprising the rAAV vector of any one of claims 8-11, optionally wherein the AAV viral particle comprises an AAV1, AAV2, AAV3, AAV4, AAV5, AAV6, AAV7, AAV8, AAVrh8, AAVrh8R, AAV9, AAV10, AAVrh10, AAV11, AAV12, AAV2R471A, AAV2/2-7m8, AAV DJ, AAV2 N587A, AAV2 E548A, AAV2 N708A, AAV2 V708K, goat AAV, AAV1/AAV2 chimeric, bovine AAV, mouse AAV, or rAAV2/HBoV1 serotype capsid.

13. The rAAV particle of claim 12, wherein the AAV viral particle comprises an engineered AAV capsid, optionally wherein the engineered AAV capsid is a DJ capsid or an LK03 capsid.

14. The rAAV particle of claim 12 or 13, wherein the ITR and the capsid of the rAAV viral particle are derived from the same AAV serotype or from different AAV serotypes.

15. A composition comprising the rAAV particle of any one of claims 12-14, optionally wherein the composition further comprises a pharmaceutically acceptable carrier.
